(19) Europäisches Patentamt / European Patent Office / Office européen des brevets

(11) **EP 4 428 232 A1**

(12) **EUROPEAN PATENT APPLICATION**
published in accordance with Art. 153(4) EPC

(43) Date of publication:
**11.09.2024 Bulletin 2024/37**

(21) Application number: **22889405.1**

(22) Date of filing: **04.11.2022**

(51) International Patent Classification (IPC):
***C12N 9/22*** *(2006.01)*

(52) Cooperative Patent Classification (CPC):
**C12N 5/10; C12N 9/14; C12N 9/22; C12N 15/113; C12N 15/85**

(86) International application number:
**PCT/CN2022/129825**

(87) International publication number:
**WO 2023/078384 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.11.2021 CN 202111306149**
**13.05.2022 CN 202210518826**

(71) Applicants:
• **Guangzhou Reforgene Medicine Co., Ltd.**
**Guangzhou, Guangdong 510700 (CN)**
• **Zheijiang Synsorbio Technology Co., Ltd.**
**Shaoxing City, Zhejiang 312300 (CN)**

(72) Inventors:
• **LIANG, Junbin**
**Guangzhou, Guangdong 510700 (CN)**
• **LIANG, Xingxiang**
**Guangzhou, Guangdong 510700 (CN)**

• **SUN, Yang**
**Shaoxing, Zhejiang 312300 (CN)**
• **XU, Hui**
**Guangzhou, Guangdong 510700 (CN)**
• **PENG, Zhiqin**
**Guangzhou, Guangdong 510700 (CN)**
• **SI, Kaiwei**
**Guangzhou, Guangdong 510700 (CN)**
• **HUANGFU, Desheng**
**Guangzhou, Guangdong 510700 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

(54) **ISOLATED CAS13 PROTEIN AND USE THEREOF**

(57) The present disclosure relates to an isolated Cas13 protein and use thereof. The amino acid sequence of the isolated Cas13 protein comprises a sequence having $\geq 50\%$ sequence identity with the sequence as shown in any one of SEQ ID NO: 1-SEQ ID NO: 7, and SEQ ID NO: 60. The Cas13 protein is a Cas13 enzyme with an endonuclease activity, which can be used in a CRISPR/Cas system to achieve targeting and modification of a target nucleic acid, enriching the enzymes and systems available in a CRISPR-Cas editing system.

Downregulation Amplitude (%)

Fig. 7

**(Cont. next page)**

Fig. 8

## Description

This application is a continuation application of PCT/CN2022/129825, filed on November 4, 2022, which claims priorities from Chinese patent application CN2021113061499, filed on November 5, 2021 and Chinese patent application CN2022105188261, filed on May 13, 2022, the entire contents of which are hereby incorporated by reference.

## TECHNICAL FIELD

The present invention relates to the technical field of gene editing, and in particular to an isolated Cas13 protein and use thereof.

## BACKGROUND

CRISPR-Cas13 is an RNA targeting and editing system based on a bacterial immune system, which can protect the bacterial from virus attack. This system is basically similar to a CRISPR-Cas9 system, but different from a DNA-targeting CRISPR-Cas9 system in that the Cas13 protein can cleaves an RNA in a targeting manner.

The CRISPR-Cas13 is of type VI in a second major type of a CRISPR-Cas system. It contains a single effector protein Cas13. When assembled with a CRISPR RNA (e.g. crRNA), it can form an RNA-targeting effector complex guided by the crRNA. Many Cas13 proteins have two different types of ribonuclease activities. One type is pretreatment of a pre-crRNA to form a type VI mature interference complex, which an RNase is responsible for; and the other type of RNase activity is provided by two higher eukaryotes and prokaryotes nucleotide-binding (HEPN) domains. The HEPN domains can help to cleave an RNA, such as an ssRNA, and when there is a folding structure in the target RNA, Cas13 generally prefers cleavage in a non-base-paired ssRNA region.

Currently, CRISPR-Cas13 can be divided into several subtypes (A, B, C and D) according to phylogeny.

The art has been always devoted to finding a novel Cas13 protein. Up to now, thousands of Cas13 proteins have been found, but not many of them are active. For example, most of the Cas13 proteins have not been reported as having an RNA targeting or modifying activity. It has been pointed out in the literature that once activated by target recognition, Cas13 will cleave the RNA indiscriminately and induce dormancy or death of a cell.

It is still a difficult problem to develop a Cas13 protein having an RNA targeting/modification activity.

## CONTENT OF THE PRESENT INVENTION

In view of the above, it is necessary to provide an isolated Cas13 protein having activities of binding to, targeting and/or modifying a target RNA, aiming at the aforementioned problems.

The present invention discloses an isolated Cas13 protein, wherein the amino acid sequence of the Cas13 protein comprises a sequence having ≥ 50% sequence identity with the sequence as shown in any one of SEQ ID NO: 1-SEQ ID NO: 7, and SEQ ID NO: 60.

The aforementioned Cas13 protein is obtained by the inventor after repeated screening and attempts among many proteins, which has activities such as capable of forming a complex with a gRNA, forming a complex with a gRNA and binding to a target nucleic acid, being guided to a target nucleic acid by a gRNA, and/or targeting or modifying a target nucleic acid.

It can be understood that the amino acid sequence of the aforementioned Cas13 protein can also include a sequence having ≥ 50%, >_ 60%, >_ 70%, >_ 75%, >_ 80%, >_ 85%, >_ 90%, >_ 92%, >_ 95%, ≥ 96%, >_ 97%, >_ 98%, >_ 99%, >_ 99.5% or 100% sequence identity with the sequence as shown in any one of SEQ ID NO: 1-SEQ ID NO: 7 and SEQ ID NO: 60. That is, the amino acid sequence shown in the aforementioned sequence is only a part of the sequence of the Cas13 protein, and the Cas13 protein may further include other functional or non-functional domains. The amino acid sequence of the aforementioned Cas13 protein can also be a sequence having ≥ 50%, >_ 60%, >_ 70%, ≥ 75%, ≥ 80%, ≥ 85%, ≥ 90%, ≥ 92%, ≥ 95%, ≥ 96%, ≥ 97%, ≥ 98%, ≥ 99%, ≥ 99.5% or 100% sequence identity with the sequence as shown in any one of SEQ ID NO: 1-SEQ ID NO: 7 and SEQ ID NO: 60. That is, the protein composed of the amino acid sequence shown in the aforementioned sequence is the Cas13 protein.

In some embodiments, the Cas13 protein can form a complex with a gRNA.

In some embodiments, the Cas13 protein can be guided to a target nucleic acid by a gRNA. It can be understood that, after the Cas13 protein is guided to the target nucleic acid by the gRNA, optionally, the target nucleic acid may be or may not be targeted or modified. For example, in some cases, after the Cas13 protein is guided to the target nucleic acid by the gRNA, the target nucleic acid may not be targeted and modified (e.g., the target nucleic acid is not cleaved), and those skilled in the art can only use its ability of recognizing the target nucleic acid, for example to enable the target nucleic acid to be bound but not cleaved. In some cases, after the Cas13 protein is guided to the target nucleic acid by the gRNA, the target nucleic acid can be targeted or modified (e.g., the target nucleic acid is cleaved). For example, a target mRNA is cleaved and thus a translation level is reduced.

In some embodiments, the Cas13 protein can be guided to the target nucleic acid by the gRNA, and target or modify the target nucleic acid.

It can be understood that, when the C as 13 protein targets the target nucleic acid, one or more of the following activities can be produced: cleaving one or more target nucleic acids, visualizing or detecting one or more target nucleic acids, labeling one or more target nucleic acids, transporting one or more target nucleic acids, masking one or more target nucleic acids, binding to one or more target nucleic acids, increasing a transcription and/or translation level of a gene corresponding to a target nucleic acid, and reducing the transcription and/or translation level of the gene corresponding to the target nucleic acid.

In some embodiments, the targeting the target nucleic acid is cleaving the target nucleic acid or binding to the target nucleic acid.

In some embodiments, the targeting the target nucleic acid is binding to the target nucleic acid. The binding can be binding caused by base complementary pairing between a gRNA guide sequence and the target sequence.

In some embodiments, the targeting the target nucleic acid is cleaving the target nucleic acid.

In some embodiments, the target nucleic acid is an RNA. In some embodiments, the RNA is optionally selected from an mRNA, a miRNA, an rRNA, a tRNA, a snRNA and a structural RNA. The Cas13 protein can be guided to the target nucleic acid by the gRNA, and then the target nucleic acid can optionally be cleaved or not.

In some embodiments, the target nucleic acid is an mRNA. In some cases, when the target nucleic acid is an mRNA, the Cas13 protein can be guided to the target nucleic acid by the gRNA, and then the target nucleic acid can optionally be cleaved or not.

In some embodiments, the target nucleic acid is a PTBP1 (polypyrimidine tract binding protein 1) mRNA, an AQp1 (aquaporin 1) mRNA, a VEGFA (vascular endothelial growth factor A) mRNA, a VEGFR1 (vascular endothelial growth factor receptor 1) mRNA, or a VEGFR2 (vascular endothelial growth factor receptor-2) mRNA.

In some embodiments, the target nucleic acid is the PTBP1 (polypyrimidine tract binding protein 1) mRNA or the AQp1 (aquaporin 1) mRNA. That is, it is also used for knocking down the level of the AQp1 mRNA, thereby reducing the generation of aqueous humor, lowering a intraocular pressure, and treating a disease such as glaucoma; or it can be used for knocking down the level of the PTBP1 mRNA, thereby realizing the transdifferentiation of a brain astrocyte into a neuron, so as to treat a disease such as Parkinson's disease. In some embodiments, the target nucleic acid is the VEGFA mRNA, the VEGFR1 mRNA or the VEGFR2 mRNA, which can be used for treating age-related macular degeneration (AMD) by knocking down the mRNA level.

It should be understood that in the previous development, people have found that many gene/protein regulatory targets are related to human and animal/plant diseases, animal and plant traits, etc. A CRISPR system established based on the present invention is feasible for the binding, targeting or modifying of such targets.

In some embodiments, the Cas13 protein is derived from the same kingdom, phylum, class, order, family, genus or species as a protein including the amino acid sequence as shown in any one of SEQ ID NO: 1-SEQ ID NO: 7 and SEQ ID NO: 60.

Herein, the protein including the sequence of SEQ ID NO: 1 is accordingly Cas13m.1, the protein including the sequence of SEQ ID NO: 2 is accordingly Cas13m.2, the protein including the sequence of SEQ ID NO: 3 is accordingly Cas13m.3, the protein including the sequence of SEQ ID NO: 4 is accordingly Cas13m.4, the protein including the sequence of SEQ ID NO: 5 is accordingly Cas13m.5, the protein including the sequence of SEQ ID NO: 6 is accordingly CasRfg.1, the protein including the sequence of SEQ ID NO: 7 is accordingly CasRfg.2, and the protein including the sequence of SEQ ID NO: 60 is accordingly Cas13m.6.

In some embodiments, the Cas13m.1 protein is derived from *Cytophagales bacterium;* the Cas13m.2 protein is derived from a bacterium including a genome numbered CNA0011077 in CNGB database; the Cas13m.3 protein is derived from *Bacteroidetes bacterium;* the Cas13m.4 protein is derived from a bacterium including a genome numbered CNA0007373 in CNGB database; the Cas13m.5 protein is derived from *Bacteroidetes bacterium;* the Cas13m.6 protein is derived from *Prevotellaceae bacterium;* the CasRfg.1 protein is derived from a bacterium including a genome numbered GCA_003940745.1 in a NCBI database; and the CasRfg.2 protein is derived from a bacterium including a genome numbered CNA0009477 in the CNGB database.

In some embodiments, the Cas13 protein is derived from:

1) a sewage metagenome, *Cytophagales bacterium,* or *Bacteroidetes bacterium;*

2) a species with a genome having an ANI value ≥ 95% with a genome numbered GCA_003940745.1, GCA_013298125.1, GCA_902762805.1 or GCA_013298545.1 in NCBI database or a genome numbered CNA0011077, CNA0007373 or CNA0009477 in the CNGB database;

3) a species with a genome having an ANI value ≥ 95% with a genome of a sewage WW isolate, a bin5 .concoct.b 16b 17b 19.071, RUG10805 or bin17.concoct.ball.095 isolate.

Average nucleotide identity (ANI) is an index to evaluate the similarity of all orthologous protein coding genes between two genomes at a nucleic acid level. For bacteria/archaebacteria, a threshold ANI = 95% is generally used as a basis for judging whether they are of the same species (Richter M, Rosselló-Móra R. Shifting the genomic gold standard for the prokaryotic species definition. Proc Natl Acad Sci U S A. 2009 Nov 10;106(45):19126-31). Therefore, the present invention is defined by the aforementioned threshold, and it is considered that each species having an ANI value ≥ 95% with the aforementioned genome is of the same species, in which the Cas13 protein has homology and function similarity with the protein claimed by the present invention, and thus belongs to the scope of the present invention.

In some embodiments, the isolated Cas13 protein is derived from a species with a genome having an ANI value ≥ 95% with a genome numbered GCA_003940745.1, GCA_013298125.1, GCA_902762805.1 or GCA_013298545.1 in NCBI database or a genome numbered CNA0011077, CNA0007373 or CNA0009477 in CNGB database.

In some embodiments, the isolated Cas13 protein is derived from a species with a genome having an ANI value ≥ 95% with a genome numbered GCA_013298125.1, GCA_902762805.1 or GCA_013298545.1 in NCBI database or a genome numbered CNA0011077, CNA0007373 or CNA0009477 in CNGB database.

In some embodiments, the isolated Cas13 protein is derived from a bacterium including a genome numbered GCA_003940745.1, GCA_013298125.1, GCA_902762805.1 or GCA_013298545.1 in NCBI database or a genome numbered CNA0011077, CNA0007373 or CNA0009477 in CNGB database.

In some embodiments, the isolated Cas13 protein is derived from the isolate strain of sewage WW, bin5.concoct.b16b17b19.071, RUG10805 or bin17.concoct.ball.095.

The present invention further discloses an isolated Cas13 protein, including amino acid sequences as shown in the following motifs 1-15:

| | |
|---|---|
| motif 1: | L-x(3)-R-N-x-Y-[ST]-H (SEQ ID NO: 84) |
| motif 2: | R-x(3)-K-x-[VI]-N-G-F-G-R (SEQ ID NO: 85) |
| motif 3: | P-Y-[IV]-T-x(5)-Y-x-[IV]-x(2)-N-x-I-G-L (SEQ ID NO: 86) |
| motif 4: | P-x-L-x(2)-D-x(3)-[NK] |
| motif 5: | P-x-[AC]-x-L-S-x(2)-[ED]-[LF]-P-A-x(2)-F (SEQ ID NO: 87) |
| motif 6: | [LI]-P-x-K-L |
| motif 7: | [KT]-x-[AL]-x(2)-[KVE]-[IL] |
| motif 8: | A-[DRK]-x-L-x(2)-[DS]-[MI]-[MV]-x-[FW]-Q-P (SEQ ID NO: 88) |
| motif 9: | K-L-T-x(2)-N (SEQ ID NO: 89) |
| motif 10: | F-x-[HR]-[AF]-x(5)-[QR] |
| motif 11: | I-x-L-P-x-G-[LM]-F-x(3)-I (SEQ ID NO: 90) |
| motif 12: | [LI]-I-x(2)-[YWF]-F |
| motif 13: | I-x(3)-I |
| motif 14: | [DN]-[TN]-E-x(2)-[IL]-[KR]-[VR]-Y-[KR]-x-Q-D (SEQ ID NO: 91) |
| motif 15: | R-N-[SA]-[FA]-x-H-x(2)-Y (SEQ ID NO: 92) |

wherein A, F, C, U, D, N, E, Q, G, H, L, I, K, O, M, P, R, S, T, V, W, Y are standard amino acid codes, "x" is any amino acid, a numbers in a bracket after x represent multiple consecutive x's, the content in "[ ]" is an optional amino acid code, and "-"is a separator.

In some embodiments, the Cas13 protein includes the motifs 1-15 from the N-terminal to the C-terminal sequentially.

In some embodiments, the motif 1 is selected from the motif 16, the motif 2 is selected from the motif 17, the motif 3 is selected from the motif 18, the motif 4 is selected from the motif 19, the motif 5 is selected from the motif 20, the motif 6 is selected from the motif 21, the motif 7 is selected from the motif 22, the motif 8 is selected from the motif 23, the motif 9 is selected from the motif 24, the motif 10 is selected from the motif 25, the motif 11 is selected from the motif 26, the motif 12 is selected from the motif 27, the motif 13 is selected from the motif 28, the motif 14 is selected from the motif 29, and the motif 15 is selected from the motif 30.

The amino acid sequences as shown in the motifs 16-30 are as follows:

motif 16: L-[RVY]-[EYH]-[LYC]-R-N-[VFM]-Y-[ST]-H (SEQ ID NO: 93)

motif 17: R-[ST]-[IVL]-[SQ]-K-[NAE]-[VI]-N-G-F-G-R (SEQ ID NO: 94)
motif 18: P-Y-[IV]-T-[DN]-[HW]-[HR]-[AT]-[KAT]-Y-[LN]-[IV]-[HS]-[NSA]-N-[RH]-I-G-L (SEQ ID NO: 95)
motif 19: P-[END]-L-[TKD]-[PIT]-D-[GKE]-[AGN]-[RDG]-[NK]
motif 20: P-[TMK]-[AC]-[WYS]-L-S-[IV]-[FY]-[ED]-[LF]-P-A-[LM]-[ALV]-F-[LY]-[LCM]-[HY]-[LI]-[YR] (SEQ ID NO: 96)
motif 21: [SNG]-[QE]-[LI]-P-[RED]-K-L
motif 22: [KT]-[WHK]-[AL]-[AQE]-[SQE]-[KVE]-[IL]
motif 23: A-[DRK]-[FY]-L-[AM]-[HTR]-[DS]-[MI]-[MV]-[FRE]-[FW]-Q-P (SEQ ID NO: 97)
motif 24: [CG]-[NGK]-[ND]-K-L-T-[GS]-[LAQ]-N (SEQ ID NO: 98)
motif 25: F-[ALV]-[HR]-[AF]-[NS]-[QSR]-[NSM]-[KR]-[WY]-[QR]
motif 26: [KA]-[SPV]-I-[ELM]-L-P-[RD]-G-[LM]-F-[ET]-[ST]-[YH]-I (SEQ ID NO: 99)
motif 27: [LI]-I-x(2)-[YWF]-F-x(5)-[DQ]-x(2)-Q-[PT]-F-Y-[DR] (SEQ ID NO: 100)
motif 28: I-[RAL]-[KQ]-[KD]-I
motif 29: [DN]-[TN]-E-[KTR]-[ED]-[IL]-[KR]-[VR]-Y-[KR]-[ILT]-Q-D (SEQ ID NO: 101)
motif 30: R-N-[SA]-[FA]-[AG]-H-[NL]-[SRT]-Y-[PK] (SEQ ID NO: 102)

In some embodiments, the amino acid sequence of the Cas13 protein includes a sequence having ≥ 50%, >_ 60%, >_ 70%, >_ 75%, >_ 80%, >_ 85%, > 90%, >_ 92%, >_ 95%, >_ 96%, >_ 97%, >_ 98%, ≥ 99%, > 99.5% or 100% sequence identity with the sequence as shown in any one of SEQ ID NO: 2, SEQ ID NO: 3, and SEQ ID NO: 60. Further, in some embodiments, any amino acid residue in the amino acid sequence of the Cas13 protein, except the amino acids identified by the motifs 1-15, is subjected to conservative amino acid replacement on the basis of a wild-type sequence, and the wild-type sequence includes the sequence as shown in any one of SEQ ID NO: 2, SEQ ID NO: 3 and SEQ ID NO: 60. In some embodiments, the amino acid sequence of the Cas 13 protein includes a sequence as shown in any one of SEQ ID NO: 2, SEQ ID NO: 3 and SEQ ID NO: 60.

In some embodiments, one or more amino acid residues (e.g., a catalytic residue) in the amino acid sequence of the Cas13 protein can be mutated, so that the Cas13 protein completely or partially loses its nuclease activity under the guidance of a gRNA. For example, an RxxxxH motif of a HEPN (higher eukaryotes and pro-karyotes nucleotide) domain of an RNase is mutated to inactivate the HEPN domain. Although such a changed protein reduces or loses the nuclease activity and does not cleave the target nucleic acid, it can still approach and bind to the target nucleic acid. For example, it can be fused with other domains for single base conversion, translation activation or translation suppression of the target nucleic acid.

In some embodiments, the nuclease activity can be reduced by mutation or modification, such as reduced by at least 10%, at least 20%, at least 30%, at least 40%, at least 50%, at least 60%, at least 70%, at least 80%, at least 90%, at least 95%, at least 97% or 100% compared with that of a wild-type Cas13 protein.

In some embodiments, the Cas13 protein can form a complex with a gRNA.

In some embodiments, the Cas13 protein can be guided to a target nucleic acid by a gRNA.

In some embodiments, the Cas13 protein is derived from the same kingdom, phylum, class, order, family, genus or species as a protein including the sequence as shown in any one of SEQ ID NO: 1-SEQ ID NO: 7 and SEQ ID NO: 60.

In some embodiments, the Cas13 protein is unnatural.

The Cas13 protein of the present invention can be modified, for example, linked to a modifying moiety (e.g. another polypeptide, oligopeptide or other molecules). Generally, the modification of the protein will not adversely affect a desired activity (e.g., an activity of binding to a gRNA, an endonuclease activity, an activity of binding to a specific site of a target nucleic acid under the guidance of a gRNA, and an activity of binding to a specific site of a target nucleic acid under the guidance of a gRNA and cleaving the target nucleic acid) of the protein. Therefore, the present invention is further intended to include such a modified protein. For example, the Cas13 protein of the present invention can be functionally linked (by chemical coupling, covalent linkage, gene fusion, non-covalent linkage or other means) to one or more modifying moieties.

The present invention discloses a conjugate including the aforementioned Cas13 protein and a modifying moiety (i.e. a heterologous functional part) for modifying the Cas13 protein.

In some embodiments, the modifying moiety of the conjugate is selected from another polypeptide, an oligopeptide, a detectable label, a pharmaceutical agent, other molecules, and any combination thereof.

In some embodiments, the modifying moiety is selected from: a localization tag for providing subcellular localization, a tag for facilitating tracking, separation or purification, a translation activation domain, a translation suppression domain, a nuclease domain, a deaminase domain, a methylase domain, a demethylase domain, and a regulatory splicing domain (e.g., for regulating RNA splicing).

In some embodiments, the localization tag for providing subcellular localization is selected from a nuclear localization signal (NLS) and a nuclear export signal (NES) sequence. Non-limiting examples of the NLS include, but are not limited to, NLS sequences derived from the following: a NLS sequence derived from a SV40 virus large T antigen; a NLS sequence derived from a nucleoplasmin; a c-myc NLS sequence; a hRNPA1 M9 NLS sequence; a NLS sequence of a IBB domain of an importin-$\alpha$; a NLS sequence of a myoma T protein; a NLS sequence of human p 53; a NLS sequence of mouse c-abl IV; a NLS sequence of an influenza virus NS1; a NLS sequence of a hepatitis virus delta antigen; a NLS sequence of a mouse Mx1 protein; a NLS sequence of a human poly(ADP-ribose) polymerase; and a NLS sequence of a steroid hormone receptor (human) glucocorticoid.

In some embodiments, the conjugate includes one or more nuclear localization signals (NLSs). In some embodiments, the conjugate includes one or more nuclear export signals (NESs). In some embodiments, the conjugate includes 1, 2, 3, 4, 5, 6, 7, 8, 9, 10 or more nuclear localization signals.

In some embodiments, the nuclear output signal includes at least four hydrophobic residues.

In some embodiments, the tag for facilitating tracking, separation or purification is selected from an epitope tag, a fluorescent protein (e.g., a green fluorescent protein (GFP), YFP, RFP, CFP, mCherry, tdTomato, etc.), a HIS tag (e.g., a 6× His tag), a hemagglutinin (HA) tag, a FLAG tag, a glutathione S-transferase (GST) tag, and a maltose-binding protein (MBP) tag;

In some embodiments, the translation activation domain is selected from domains of eIF4E and other translation initiation factors, a yeast poly(A)-binding protein, and GLD2.

In some embodiments, the translation suppression domain is selected from: a Pumilio protein, a deaminase (e.g., a deaminase CAF1), and an Argonaute protein.

In some embodiments, the nuclease domain is selected from: FOK I, a PIN endonuclease domain, a NYN domain, a SMR domain from SOT1, and an RNase domain from staphylococcal nuclease.

In some embodiments, the deaminase domain is derived from cytidine deaminase or adenosine deaminase.

In some embodiments, the deaminase domain is selected from: a PPR (pentatricopeptide repeat) protein, an ADAR family protein, and an APOBEC family protein.

In some embodiments, the methylase domain is derived from an m6A methyltransferase.

In some embodiments, the demethylase domain is derived from an RNA demethylase ALKBH5.

In some embodiments, the regulatory splicing domain is selected from: SRSF1, hnRNP A1 and RBM4.

In some embodiments, the conjugate includes the aforementioned Cas13 protein, and one or more modifying moieties. In some embodiments, the conjugate consists of the aforementioned Cas13 protein, and one or more modifying moieties. In some embodiments, the conjugate consists of the aforementioned Cast 3 protein, one or more modifying moieties, and a linker for connecting the Cast 3 protein and the modifying moieties. In some cases, the multiple modifying moieties may be the same or different.

In some embodiments, the conjugate includes or does not include the linker for connecting the Cast 3 protein and the modifying moieties.

In some embodiments, the conjugate includes a Cas13 protein, a modifying moiety, and a linker for connecting the Cas13 protein and the modifying moiety.

In some embodiments, the conjugate consists of a Cas13 protein, a modifying moiety, and a linker for connecting the Cas13 protein and the modifying moiety.

In some embodiments, the conjugate does not include the linker for connecting the Cas13 protein and the modifying moiety. In some embodiments, the conjugate is formed by directly connecting the Cas13 protein and the modifying moiety, including directly connecting via a covalent bond.

In some embodiments, the linker may be an amino acid, an amino acid sequence, or other chemical groups. In some embodiments, the linker may be an amino acid, an amino acid derivative, or PEG (polyethylene glycol).

In some embodiments, the linker is a linear polypeptide formed by connecting one or more amino acid residues through peptide bonds, wherein the amino acid residues may be natural or unnatural, and for example may be modified.

Examples of the linker include a linker containing one or more (e.g., 1, 2, 3, 4 or 5) amino acids (e.g., Glu or Ser) or amino acid derivatives (e.g., Ahx, $\beta$-Ala, GABA or Ava), or PEG, etc.

It is also within the scope of the present invention to adopt the same structure as the modifying moiety as the linker. Non-limiting examples, for example, a subcellular localization signal (e.g., NLS or NES), a tag (e.g., a HA tag, a Flag tag), etc. as the linker, are also within the scope of the present invention.

In some embodiments, the conjugate can interact with a gRNA.

In some embodiments, the conjugate can form a complex with a gRNA.

In some embodiments, the conjugate can form a complex with a gRNA, and the complex binds to a target nucleic acid.

In some embodiments, the conjugate can be guided to a target nucleic acid by a gRNA.

In some embodiments, the conjugate can be guided to a target nucleic acid by a gRNA, and target or modify the target nucleic acid. It can be understood that after the conjugate is guided to the target nucleic acid by the gRNA, optionally, the target nucleic acid may be or may not be targeted or modified. For example, in some cases, after the conjugate is guided to the target nucleic acid by the gRNA, the target nucleic acid may not be targeted and modified (e.g., the target nucleic acid is not cleaved), and those skilled in the art can only use its ability of binding to the target nucleic acid. In some cases, after the conjugate is guided to the target nucleic acid by the gRNA, the target nucleic acid can be targeted or modified. For example, a target mRNA is cleaved and thus a translation level is reduced.

In some embodiments, the modifying moiety may be connected to an amino terminal of, the vicinity of the amino terminal of, a carboxyl terminal, and/or the vicinity of the carboxyl terminal of the Cas13 protein. In some embodiments, the modifying moiety is connected to the amino terminal and/or carboxyl terminal of the Cas13 protein. In some embodiments, the modifying moiety is connected to the vicinity of the amino terminal or the vicinity of the carboxyl terminal of the Cas13 protein. In some embodiments, when the modifying moiety is within about 1, 2, 3, 4, 5, 10, 15, 20, 25, 30, 40, 50 or more amino acids from the amino terminal or carboxyl terminal along the polypeptide chain, the modifying moiety is considered to be in the vicinity of the amino terminal or carboxyl terminal.

In some embodiments, the conjugate includes one or more nuclear localization signals and/or nuclear export signals with sufficient intensity to drive the conjugate to accumulate in a detectable amount in and/or outside the nucleus of a eukaryotic cell. Detection of the accumulated amount of the Cas13 protein or conjugate in a specific part of the cell can be performed by any suitable technique.

In some embodiments, the conjugate is unnatural.

The present invention further discloses a gRNA which can form a complex with the aforementioned Cas13 protein or the aforementioned conjugate.

It can be understood that the aforementioned gRNA can guide the aforementioned Cas13 protein or conjugate to a target nucleic acid. In some embodiments, the Cas13 protein or conjugate can be guided to the target nucleic acid by the gRNA, and target or modify the target nucleic acid. In some embodiments, the complex is guided to the target nucleic acid by the gRNA, and then the complex targets or modifies the target nucleic acid. In some embodiments, the targeting the target nucleic acid is cleaving the target nucleic acid or binding to the target nucleic acid.

In some embodiments, the gRNA includes a guide sequence which can be complementary to a target nucleic acid, and a direct repeat sequence which can interact with the Cast3 protein or the conjugate.

In some embodiments, the guide sequence can be complementary (completely or partially complementary) to the target nucleic acid, and the direct repeat sequence can interact with the Cas13 protein or the conjugate.

In some embodiments, when the gRNA is used in combination with the Cas13m protein (Cas13m.1 - Cas13m.6) of the present invention, a protein having ≥ 50% sequence identity with the Cas13m protein, or a conjugate containing the same, the direct repeat sequence of the gRNAis located at the 3' terminal of the guide sequence.

In some embodiments, when the gRNA is used in combination with the CasRfg.1 or CasRfg.2 protein of the present invention, a protein having ≥ 50% sequence identity with the CasRfg.1 or CasRfg.2, or the conjugate containing the same, the direct repeat sequence of the gRNA is located at the 5' terminal of the guide sequence.

In some embodiments, the gRNA includes a guide sequence and a direct repeat sequence, wherein a secondary structure of the direct repeat sequence includes a complementary paired first stem, a non-complementary bulge structure, a complementary paired second stem and a non-complementary loop structure which are connected in sequence.

Further, in some embodiments, the gRNA is characterized in that: a. the first stem consists of 4-7 base pairs; b. one of the sequences of the non-complementary bulge structure is 2-6 nucleotides in length; c. the second stem consists of 4-7 base pairs; and/or d. the non-complementary loop structure (excluding the pair of bases that are complementary paired at the junction between the loop and the stem) has a sequence length of 5-8 nucleotides.

In some embodiments, one of the sequences of the first stem is selected from: GUUG, GUUGU, GUUGUA and GUUGUAA.

In some embodiments, the gRNA incudes a guide sequence and a direct repeat sequence, wherein the direct repeat sequence is selected from a sequence having ≥ 90% sequence identity, or a sequence having ≥ 95% sequence identity with the sequence as shown in any one of SEQ ID NO: 15-SEQ ID NO: 21 and SEQ ID NO: 62.

In some embodiments, the direct repeat sequence is selected from any one of SEQ ID NO: 15-SEQ ID NO: 21 and SEQ ID NO: 62.

In some embodiments, the gRNA includes a guide sequence and a direct repeat sequence, wherein the guide sequence is ≥ 10 nt (10 nucleotides), >_ 11 nt, ≥ 12 nt, ≥ 13 nt, ≥ 14 nt, ≥ 15 nt, ≥ 16 nt, >_ 17 nt, ≥18 nt, >_ 19 nt, >_ 20 nt, >_ 21 nt, >_ 22 nt, >_ 23 nt, >_ 24 nt, >_ 25 nt, >_ 26 nt, >_ 27 nt, >_ 28 nt, >_ 29 nt, >_ 30 nt, >_ 31 nt, >_ 32 nt, >_ 33 nt, >_ 34 nt, >_ 35 nt, >_ 40 nt, >_ 50 nt or ≥ 60 nt in length.

In some embodiments, the gRNA includes a guide sequence and a direct repeat sequence, wherein the guide sequence is ≤10 nt (10 nucleotides), <_ 11 nt, ≤ 12 nt, ≤ 13 nt, ≤ 14 nt, ≤ 15 nt, ≤ 16 nt, <_ 17 nt, <_ 18 nt, <_ 19 nt, <_ 20 nt, <_ 21 nt, <_ 22 nt, <_ 23 nt, <_ 24 nt, <_ 25 nt, <_ 26 nt, <_ 27 nt, <_ 28 nt, <_ 29 nt, <_ 30 nt, <_ 31 nt, <_ 32 nt, <_ 33 nt, <_ 34 nt, <_ 35 nt, <_ 40 nt, <_ 50 nt or ≤ 60 nt in length.

In some embodiments, the gRNA includes a guide sequence and a direct repeat sequence, wherein the guide sequence ranges from 10 nt-60 nt, 10 nt-50 nt, 10 nt-40 nt, 12 nt-35 nt, 15 nt-35 nt, 15 nt-30 nt, 20 nt-35 nt, 20 nt-30 nt, 25 nt-35 nt or 25 nt-30 nt in length.

In some embodiments, the gRNA includes a guide sequence and a direct repeat sequence, wherein the direct repeat sequence is ≥ 10 nt, ≥ 15 nt, ≥ 20 nt, ≥ 25 nt, ≥ 30 nt, ≥ 35 nt, ≥ 40 nt, ≥ 45 nt, >_ 50 nt, >_ 60 nt, >_ 70 nt, >_ 80 nt, >_ 90 nt, >_ 100 nt, >_ 150 nt, >_ 200 nt or ≥ 300 nt in length.

In some embodiments, the gRNA includes a guide sequence and a direct repeat sequence, wherein the direct repeat sequence is ≤ 10 nt, ≤ 15 nt, ≤ 20 nt, ≤ 25 nt, ≤ 30 nt, ≤ 35 nt, ≤ 40 nt, ≤ 45 nt, <_ 50 nt, <_ 60 nt, <_ 70 nt, <_ 80 nt, <_ 90 nt, <_ 100 nt, <_ 150 nt, <_ 200 nt or ≤ 300 nt in length.

In some embodiments, the gRNA includes a guide sequence and a direct repeat sequence, wherein the direct repeat sequence ranges from 10 nt-300 nt, 10 nt-200 nt, 10 nt-100 nt, 15 nt-80 nt, 15 nt-50 nt, 15 nt-40 nt, 15 nt-35 nt, or 20 nt-40 nt in length.

In some embodiments, the direct repeat sequence is located at the 3' terminal of the guide sequence. In some embodiments, the direct repeat sequence is located at the 5' terminal of the guide sequence.

In some embodiments, the target nucleic acid is PTBP1 (polypyrimidine tract binding protein 1) mRNA, AQp1 (aquaporin 1) mRNA, VEGFA mRNA, VEGFR1 mRNAor VEGFR2 mRNA.

In some embodiments, the target nucleic acid is PTBP1 mRNA or AQp1 mRNA. In some embodiments, the target nucleic acid is VEGFA mRNA, VEGFR1 mRNA or VEGFR2 mRNA.

The present invention further discloses a composition including:

1) the aforementioned Cas13 protein, the aforementioned conjugate, a nucleic acid encoding the aforementioned Cas13 protein, or a nucleic acid encoding the aforementioned conjugate;
and
2) the aforementioned gRNA or a nucleic acid encoding the gRNA.

In some embodiments, the gRNA includes a guide sequence which can be complementary to a target nucleic acid, and the target nucleic acid is the PTBP1 mRNA or the AQp1 mRNA.

In some embodiments, the nucleic acid is a DNA. In some embodiments, the nucleic acid is an RNA.

The present invention also discloses a nucleic acid, including:

1) a nucleotide sequence encoding the aforementioned Cas13 protein, or a nucleotide sequence encoding the aforementioned conjugate;
and/or
2) a nucleotide sequence encoding the aforementioned gRNA.

In some embodiments, the nucleotide sequence is used for expression in a prokaryotic or eukaryotic cell.

In some embodiments, the nucleic acid is a DNA. In some embodiments, the nucleic acid is an RNA.

The present invention further discloses a vector, including:

1) a nucleotide sequence encoding the aforementioned Cas13 protein, or a nucleotide sequence encoding the aforementioned conjugate;
and/or
2) a nucleotide sequence encoding the aforementioned gRNA.

In some embodiments, the nucleotide sequence encoding the Cas13 protein is one or more, and the nucleotide sequence encoding the conjugate is one or more.

In some embodiments, the vector includes a regulatory element.

In some embodiments, the regulatory element can regulate the expression of the nucleotide sequence.

In some embodiments, the regulatory element is a promoter and/or enhancer. In some embodiments, the regulatory element is a promoter.

In some embodiments, the vector is selected from a cloning vector and an expression vector. In some embodiments, the vector is a plasmid or viral vector.

In some embodiments, the vector can express the Cas13 protein or conjugate of the present invention in a cell. In some embodiments, the vector can express the Cas13 protein or conjugate of the present invention in a eukaryotic cell. In some embodiments, the vector can express the Cas13 protein or conjugate of the present invention in a human cell.

In some embodiment, the vector is an unnatural vector.

The present invention further discloses a delivery composition, including a delivery vector and at least one selected from: the aforementioned Cas13 protein, conjugate, gRNA, composition, nucleic acid and vector.

In some embodiments, the delivery vector is at least one selected from of a delivery particle, a delivery vesicle and a virus vector.

The present invention further discloses a cell, including at least one of the aforementioned Cas13 protein, conjugate, gRNA, composition, nucleic acid and vector.

In some embodiments, the cell is a eukaryotic cell.

In some embodiments, the target nucleic acid is derived from an animal cell, a plant cell or a microbial cell.

In some embodiments, an animal or plant cannot be produced from the cell.

In some embodiments, an animal or plant cannot be produced from the eukaryotic cell.

In some embodiments, the eukaryotic cell includes a stem cell and a stem cell line. In some embodiments, the stem cell is not an embryonic stem cell, and the stem cell line is not an embryonic stem cell line.

In some embodiments, for the cells including the Cas13 protein, conjugate, gRNA, complex, isolated nucleic acid, vector, composition and delivery composition of the present invention, the target nucleic acid in these cells has been targeted or modified.

The present invention further discloses a method of targeting or modifying a target nucleic acid, including delivering at least one selected from: the aforementioned Cas13 protein, conjugate, gRNA, composition, nucleic acid, vector and cell. In some embodiments, the delivery takes place *ex vivo, in vitro* or *in vivo.* In some embodiments, the method of targeting or modifying the target nucleic acid is used for modifying a cell, a cell line or an organism by changing the target nucleic acid. In some embodiments, the target nucleic acid is derived from an animal cell, a plant cell or a microbial cell.

In some embodiments, the target nucleic acid is PTBP1 (polypyrimidine tract binding protein 1) mRNA, AQp1 (aquaporin 1) mRNA, VEGFA mRNA, VEGFR1 mRNAor VEGFR2 mRNA.

In some embodiments, the target nucleic acid is PTBP1 (polypyrimidine tract binding protein 1) mRNA or AQp1 (aquaporin 1) mRNA. In some embodiments, the target nucleic acid is VEGFA mRNA, VEGFR1 mRNA or VEGFR2 mRNA.

In some embodiments, the method of targeting or modifying the target nucleic acid does not include a method for diagnosing and treating a disease.

In some embodiments, the method of targeting or modifying the target nucleic acid includes a method for diagnosing and treating a disease.

The present invention further discloses use of the aforementioned Cas13 protein, conjugate, gRNA, composition, nucleic acid or vector cell in preparation of a medicament for diagnosing, preventing or treating a disease in a subject. In some embodiments, the subject is a human individual.

The present invention further discloses a method for administrating the Cas13 protein, conjugate, gRNA, composition, nucleic acid or vector cell to a subject in an effective amount to diagnose, prevent or treat a disease.

The present invention further discloses a method for detecting a nucleic acid, including the step of allowing the following a and b to form a complex, and binding the complex to a target nucleic acid to be tested:

a. the aforementioned Cas13 protein or the aforementioned conjugate,
b. the aforementioned gRNA.

In some embodiments, the method includes allowing the aforementioned conjugate to form a complex with the gRNA, and binding the complex with the target nucleic acid, wherein the conjugate contains a detectable label, in which a signal change is caused by the binding, cleavage or modification of the complex to the target nucleic acid,, and the content of the target nucleic acid in a sample to be tested is analyzed by observing the signal change of the detectable label. Further, the detectable label includes a fluorescent group, a color-developing agent, a developer or a radioisotope.

Compared with the prior art, the present invention has the following beneficial effects.

The isolated Cas13 protein of the present invention is a novel Cas13 enzyme, which can be used in a CRISPR/Cas system. Also, as verified by experiments, the Cas13 protein of the present invention can have good editing efficiency for both an exogenous reporter gene and an endogenous gene when exerting its Cas13 nuclease activity.

**BRIEF DESCRIPTION OF THE DRAWINGS**

Fig. 1 is a schematic diagram for comparison of a locus structure between the Cas13 protein of Example 1 and various published subtypes of Cas13.

Fig. 2 shows the position of an RxxxxH motif in an amino acid chain, for the Cas13 protein of Example 1 and other subtypes of Cast 3 protein.

Fig. 3Aand Fig. 3B are schematic cluster analysis diagrams of the Cas13 protein of Example 1 and other subtypes of Cas13 protein, wherein Fig. 3A is a schematic cluster analysis diagram of Cas13m.1-Cas13m.5, and Fig. 3B is a schematic cluster analysis diagram of Cas13m.1-Cas13m.6.

Fig. 4 is a schematic diagram of RNA secondary structure analysis of a corresponding direct repeat sequence of the Cas13 protein of Example 1 by RNAfold.

Fig. 5 shows three-dimensional predicted structures of Cas13m.2, Cas13m.3 and Cas13m.6 proteins in Example 1.

Fig. 6 is a schematic diagram of superposition of the Cas13 protein of Example 1.

Fig. 7 shows a result of GFP fluorescence as detected by a flow cytometer in Example 4.

Fig. 8 is a schematic diagram of mRNA changes of endogenous target genes AQp1 and PTBP1 as detected by qPCR in Example5.

Figs. 9A, 9B and 9C are schematic screenshot diagrams of multi-sequence alignment between a Cas13m protein and PbuCas13b in Example 9.

Fig. 10 is a superposition diagram of Example 9, wherein A-N respectively show the overlapping of motifs 1-15 of Cas13m.6 and corresponding sequences of PbuCas13b after superposition between Cas13m.6 and PbuCas13b.

Fig. 11 shows a result of a collateral cleavage effect assay in Example 11.

## DETAILED DESCRIPTION OF THE PREFERRED EMBODIMENTS

In order to facilitate the understanding of the present invention, the present invention will be more fully described below with reference to relevant drawings. A preferred embodiment of the present invention is shown in the accompanying drawings. However, the present invention can be implemented in many different forms, and is not limited to the examples described herein. On the contrary, these examples are provided for a more thorough and comprehensive understanding of the present invention.

Unless otherwise defined, all technical and scientific terms used herein have the same meanings as those commonly understood by those skilled in the art to which the present invention pertains to. Herein, the terminology used in this specification of the present invention is only for the purpose of describing specific examples, and is not intended to limit the present invention. As used herein, the term "and/or" includes any and all combinations of one or more related listed items.

As used herein, a protein or polypeptide referred to as a "Cas13 protein" or having a "Cas enzyme activity" or "Cas endonuclease activity" refers to a CRISPR associated (Cas) polypeptide or protein encoded by a CRISPR associated (Cas) gene. The Cast3 protein or polypeptide can be guided to a target sequence in a target nucleic acid when being complexed or functionally combined with one or more guide RNAs (gRNAs). Under the guidance of the gRNA, the Cas endonuclease recognizes, targets or modifies a specific target site (a target sequence or a nucleotide sequence near the target sequence) in a target nucleic acid, for example a target site in an RNA (e.g. A coding RNA, e.g. mRNA) molecule.

As used herein, the term "HEPN domain" has the meaning generally recognized in the art. The HEPN domain has been proved to be an RNase domain, and has the ability to bind to and cleave a target RNA molecule. The target RNA can be any suitable form of RNA, including but not limited to a coding RNA and a non-coding RNA. The previously discovered CRISPR, class 2, type VI effector proteins all contain two HEPN domains, including, for example, Cas13a, Cas13b, Cas13c, Cas13d, Cas13e and Cas13f, and each of their HEPN domains has a conserved RxxxxH motif, which is the characteristic of the HEPN domain.

As used herein, the terms "gRNA" and "guide RNA" are used interchangeably, and they have the meanings commonly understood by those skilled in the art. The gRNA generally refers to a RNA molecule (or a collective name of a group of RNA molecules) that can bind to a Cas13 protein and facilitate to guide/target the Cas13 protein to a specific position (target sequence) within a target nucleic acid/target polynucleotide (e.g., a DNA or mRNA molecule). The gRNA contains a guide sequence and a direct repeat (DR) sequence. The gRNA may contain one or more modifications (e.g., base modification, scaffold modification, modification of an internucleoside bond, etc.) to provide the same function as an unmodified gRNA, or to provide a new or enhanced feature (e.g., improved stability) to the gRNA.

As used herein, the terms "guide sequence" and "targeting domain" are used interchangeably, and refer to a continuous nucleotide sequence in a gRNA, which is partially or completely complementary to the target sequence in the target nucleic acid, and can hybridize with the target sequence in the target nucleic acid through base pairing promoted by the Cas13 protein. Complete complementarity between the guide sequence of the present invention and the target sequence is not necessary, as long as there is sufficient complementarity to cause hybridization and promote the formation of a CRISPR/Cas complex.

A suitable direct repeat (DR) sequence can be found from a CRISPR locus structure of a procaryotic organism (e.g., a bacterium and an archaebacterium) through experimental screening. The size of the direct repeat sequence is usually within tens of bp, and partial fragments of it are reverse complementary to each other, which means that a secondary structure, such as a stem-loop structure (often called a hairpin structure), is formed inside the RNA molecule, while other fragments are embodied as unstructured. The direct repeat sequence is a constant part of a gRNA molecule, which contains a strong secondary structure, which facilitates the interaction between the Cas13 protein and the gRNA molecule.

As used herein, a term "target nucleic acid", "target RNA" or "target polynucleotide" refers to a polynucleotide containing a target sequence, and are often used interchangeably herein. The target nucleic acid can include any polynucleotide, such as a DNA (target DNA) or an RNA (target RNA). The "target nucleic acid" refers to a nucleic acid to be targeted or modified by the Cas13 protein as guided by the gRNA. The term "target nucleic acid" can be any polynucleotide endogenous or exogenous to a cell (e.g., a eukaryotic cell). For example, the "target nucleic acid" may be a polynucleotide existed in a eukaryotic cell, or a sequence (or a part thereof) encoding a gene product (e.g., protein) or a non-coding sequence (or a part thereof). In some cases, the "target nucleic acid" can include one or more disease-associated genes and polynucleotides, as well as genes and polynucleotides related to biochemical pathways of signaling. The "disease-associated" gene or polynucleotide refers to any gene or polynucleotide that produces a transcription or translation product at an abnormal level or in an abnormal form in a cell derived from a tissue affected by the disease, as compared with that of a non-disease control tissue or cell. In some cases, the target nucleic acid is a coding RNA. In some cases, the target nucleic acid is a non-coding RNA. In some cases, the target nucleic acid includes an mRNA, a miRNA, an rRNA, a tRNA, a snRNA, and a structural RNA. In some cases, the target nucleic acid is an mRNA. In some cases, the target nucleic acid is the whole mRNA molecule. In some cases, the target nucleic acid is a DNA. In some cases, the target nucleic acid is a whole chromosome DNA molecule. As used herein, the "target RNA", "target nucleic acid" and "target" mean a specific sequence or a reverse complementary sequence thereof that is desired to be bound, targeted or modified by using a CRISPR system.

As used herein, the term "target sequence" refers to a small stretch of sequence in a target nucleic acid molecule, which can be complementary (completely or partially complementary) to the guide sequence of the gRNA molecule. The length of the target sequence is often tens of bp, and for example, it can be about 10 bp, about 20 bp, about 30 bp, about 40 bp, about 50 bp or about 60 bp.

As used herein, the term "targeting" is defined to include one or more of the following: cleaving one or more target nucleic acids, visualizing or detecting one or more target nucleic acids, labeling one or more target nucleic acids, transporting one or more target nucleic acids, masking one or more target nucleic acids, binding to one or more target nucleic acids, increasing a transcription and/or translation level of a gene corresponding to a target sequence, and reducing the transcription and/or translation level of the gene corresponding to the target sequence.

As used herein, the term "modification" is defined to include one or more of the following: base substitution of a nucleic acid, a base deletion of nucleic acid, base insertion of nucleic acid, methylation of nucleic acid, demethylation of nucleic acid, and deamination of nucleic acid.

As used herein, the term "cleavage/cleaving" refers to allowing a covalent bond (e.g., a covalent phosphodiester bond) in a ribosyl phosphodiester backbone of a polynucleotide to be broken, including but not limited to: allowing a single-stranded polynucleotide to be broken, allowing either single strand of a double-stranded polynucleotide containing two complementary single strands to be broken, and allowing both single strands of the double-stranded polynucleotide containing two complementary single strands to be broken.

For example, as can be understood by those skilled in the art that, the Cas13 protein or conjugate of the present invention can be fused or associated with one or more heterologous functional moieties (e.g., by a fusion protein, a linker peptide, etc.). For example, a Cas13 mutant completely or partially losing a nuclease activity is partially fused with a heterologous functional moiety. These functional domains can have various activities, such as a methylase activity, a demethylase activity, a deaminase activity, a translation activation activity, a translation suppression activity, an RNA cleavage activity, a nucleic acid binding activity, a base editing activity, and a switching activity (e.g., light induction). The heterologous functional moiety may include, but is not limited to, a localization signal (e.g., a nuclear localization signal NLS and a nuclear export signal NES), a label or a detection label (e.g., a fluorescent dye such as FITC or DAPI), a targeting moiety, an antigenic determinant tag (e.g., Hismyc, V5, FLAG, HA, VSV-G, Trx, etc.), a deaminase or deamination domain (e.g., ADAR1, ADAR2, APOBEC, AID or TAD), a methylase, a demethylase, a ssRNA cleavage active domain, a dsRNA cleavage active domain, a DNA or RNA ligase, or any combination of the above.

For example, the Cas13 protein of the present invention can be fused with a deaminase, combined with a gRNA, and then used for targeting a target RNA, so as to realize single-base editing of the target RNA molecule.

For example, the heterologous functional moiety may be a detectable label. When a CRISPR-CAS complex contacts or binds to the target nucleic acid, the conjugate containing the Cas13 nuclease cleaves or modifies the target nucleic acid, and the presence of the target nucleic acid in the sample to be tested is analyzed by observing the presence of the detectable label. The detectable label is for example a fluorescent group, a color-developing agent, a developer or a radioisotope.

A method for measuring the binding of the Cas13 protein or conjugate to the target nucleic acid is known in the art, including but not limited to a chromatin immunoprecipitation assay, a gel mobility change assay, a reporter gene assay, and a microplate capture and detection assay. Similarly, a method for measuring cleavage or modification of the target nucleic acid is known in the art, including an *in vitro* or *in vivo* cleavage assay.

As used herein, the terms "complex" and "CRISPR/Cas complex" are used interchangeably. The term "complex" refers to a ribonucleoprotein complex formed by the binding of the gRNA and the Cas13 protein. The ribonucleoprotein complex can recognize (and sometimes further cleave or modify) a target sequence complementary to the guide sequence of the gRNA or a target nucleic acid in which the target sequence is located.

As used herein, the term "unnatural" means "modified", which means that artificial means is involved. When a nucleic acid molecule or polypeptide is referred, the term means that the nucleic acid molecule or the polypeptide is at least substantially free of at least one other component that is naturally associated with them in nature and associated with them when found. Furthermore, this term can indicate that a nucleic acid molecule or polypeptide has a sequence that is not existed in nature.

As used herein, the term "conjugate" refers to a modified Cas13 protein. The conjugate includes a Cas13 protein part and a modifying moiety. The modifying moiety can be a protein or polypeptide (or any functional fragment of them), an oligopeptide and other small molecules (including but not limited to a sugar molecule). The conjugate can be a fusion protein.

As used herein, the term "sequence identity (identity or percent identity)" is used for referring to the matching of sequences between two polypeptides or two nucleic acids. When a certain position in two sequences to be compared is occupied by the same base or amino acid monomer subunit (for example, a certain position of each of two DNA molecules is occupied by adenine, or a certain position of each of two polypeptides is occupied by lysine), then each molecule is identical at the position. Percent sequence identity between two sequences is a function of the number of matching positions shared by the two sequences divided by the number of positions to be compared × 100%. For example, if 6 of 10 positions of two sequences are matched, then the two sequences have a sequence identity of 60%. Generally, two sequences are compared when they are aligned to produce the maximum sequence identity. Such alignment can be made by using published and commercially-available alignment algorithms and programs, such as but not limited to Clustal Ω, MAFFT, Probcons, T-Coffee, Probalign, BLAST, which can be reasonably selected and used by those skilled in the art. Those skilled in the art can determine the appropriate parameters for aligning sequences, including, for example, any algorithm needed to achieve preferred alignment or optimal alignment for the whole length of the compared sequences, and any algorithm needed to achieve preferred alignment or optimal alignment for the local parts of the compared sequences.

The sequence identity is related to sequence similarity. Identity or similarity comparison can be made by visual comparison (naked eyes), and more generally by means of a sequence comparison program. These computer programs can calculate the percentage (%) of identity or similarity among two or more sequences, and can also calculate the sequence identity shared by two or more amino acid or nucleic acid sequences.

The terms "polypeptide", "peptide" and "protein" are used interchangeably herein, and refer to a polymer having amino acids of any length. The polymer may be linear or branched, it may contain modified amino acids, and it may be interrupted by non-amino acids. These terms also cover amino acid polymers that have been modified; and these modifications are, for example, disulfide bond formation, glycosylation, lipidation, acetylation, phosphorylation or any other manipulation, such as conjugation with a labeling component. As used herein, the term "amino acid" includes natural and/or unnatural or synthetic amino acids, including glycine and D and L optical isomers, as well as amino acid analogs and peptide mimics.

As used herein, the term "domain" or "protein domain" refers to a part of a protein sequence that can be existed and function independently of the rest of the protein chain.

As used herein, the term "vector" refers to a nucleic acid carrier into which a polynucleotide can be inserted. When a vector can enable a protein encoded by the inserted polynucleotide to be expressed, the vector is called an expression vector. The vector can enter a host cell by manners of transformation, transduction or transfection, so that a genetic material element carried by the vector can be expressed in the host cell. The vector is well known to those skilled in the art, and includes but is not limited to: a plasmid; a cosmid; a phagemid; an artificial chromosome, such as a yeast artificial chromosome (YAC) or a bacterial artificial chromosome (BAC); a phage such as phage λ, and an animal virus. An animal virus that can be used as a vector includes, but is not limited to, a retrovirus (including a lentivirus), an adenovirus, an adeno-associated virus, a herpes virus (e.g., a herpes simplex virus), a poxvirus, a baculovirus, a papillomavirus, a papovavirus (such as SV40). A vector can contain a variety of elements for controlling expression, including but not limited to: a promoter sequence, a transcription initiation sequence, an enhancer sequence, a selection element, and a reporter gene. Additionally, the vector may also contain a replication origin. The vector includes, but is not limited to, a single-stranded, double-stranded or partially double-stranded nucleic acid molecule; a nucleic acid molecule containing one or more free ends or not containing a free end (e.g., a circular nucleic acid molecule); a nucleic acid molecule containing a DNA, an RNA or both; and other kinds of polynucleotides known in the art. Certain vectors can autonomously replicate in a host cell into which they are introduced. Other vectors are integrated into the genome of the host cell after being introduced into the host cell, and thus replicate along with the host genome. Furthermore, certain vectors can guide the expression of genes to which they are operably linked. Such a vector is referred to herein as an "expression vector". The vector used in a eukaryotic cell and producing expression in the eukaryotic cell can be called a "eukaryotic expression vector" here. A common expression vector used in a recombinant DNA technology is often in a form of plasmid.

A vector can be introduced into a host cell and thus produces a transcript, protein, or peptide, including the protein, conjugate, isolated nucleic acid, complex, composition, and the like as described herein.

The recombinant expression vector can contain the nucleic acid of the present invention in a form suitable for expressing the nucleic acid in a host cell, which means that the recombinant expression vector contains one or more regulatory elements, which can be selected based on the host cell used for expression and operably linked to a nucleic acid sequence to be expressed.

As used herein, the term "operably linked" is intended to mean that a Cas protein coding sequence or gRNA coding sequence in the vector is linked to one or more regulatory elements in a way that allows the nucleotide sequence to be expressed (e.g., to be expressed in an *in vitro* transcription/translation system or in a host cell when the vector is introduced into the host cell). For example, in the vector, a promoter 1 is placed upstream of a coding sequence of the Cas13 protein. When the vector is introduced into a host cell, the transcription of a Cas13 gene can be started under the drive of the promoter 1.

As used herein, the term "regulatory element" is intended to include a promoter, an enhancer, an internal ribosome entry site (IRES), and other expression control elements (e.g., a transcription termination signal, such as a polyadenylation signal and a poly U sequence). The regulatory element includes those that guide nucleotide sequences to be expressed continuously in many types of host cells, and those that guide nucleotide sequences to be expressed only in certain host cells (e.g., a tissue-specific regulatory sequence). A tissue-specific promoter can guide the expression mainly in a desired tissue of interest, such as muscle, a neuron, bone, skin, blood, a specific organ (e.g., liver, pancreas), or a specific cell type (e.g., a lymphocyte). The regulatory element can also guide expression in a time-dependent manner, such as a cell cycle-dependent or developmental stage-dependent manner, which may or may not be tissue-specific or cell-type specific. In some embodiments, the vector includes one or more pol III promoters (e.g., 1, 2, 3, 4, 5, or more pol III promoters), one or more pol II promoters (e.g., 1, 2, 3, 4, 5, or more pol II promoters), one or more pol I promoters (e.g., 1, 2, 3, 4, 5, or more pol I promoters), or a combination thereof. Examples of the pol III promoter include, but are not limited to, U6 and H1 promoters. Examples of the pol II promoter include, but are not limited to, a retrovirus Rous sarcoma virus (RSV) LTR promoter (optionally with an RSV enhancer), a cytomegalovirus (CMV) promoter (optionally with a CMV enhancer), a SV40 promoter, a dihydrofolate reductase promoter, a $\beta$-actin promoter, a phosphoglycerokinase (PGK) promoter, and an EF1$\alpha$ promoter. The term "regulatory element" also encompasses an enhancer element, such as WPRE, a CMV enhancer, a SV40 enhancer, and an intron sequence between exons 2 and 3 of a rabbit beta-globulin. Those skilled in the art will understand that the design of an expression vector may depend on factors such as the selection of a host cell to be transformed and a desired expression level. The vector can be introduced into the host cell to express the Cas13 protein, conjugate or CRISPR complex of the present invention.

As used herein, the term "promoter" has a meaning well-known to those skilled in the art, which refers to a stretch of non-coding nucleotide sequence located upstream of a gene and capable of initiating the expression of a downstream gene. A constitutive promoter is such a nucleotide sequence that, when operably linked to a polynucleotide encoding or defining a gene product, leads to the production of the gene product in a cell under most or all physiological conditions of the cell. An inducible promoter is such a nucleotide sequence that, when operably linked to a polynucleotide encoding or defining a gene product, leads to the production of the gene product in a cell basically only when an inducer corresponding to the promoter is existed in the cell. A tissue-specific promoter is such a nucleotide sequence that, when operably linked to a polynucleotide encoding or defining a gene product, leads to the production of the gene product in a cell basically only when the cell is a cell of the tissue type corresponding to the promoter.

As used herein, the term "host cell" refers to a cell that can be used for introducing a vector, including but not limited to a prokaryotic cell such as *Escherichia coli* or *Bacillus subtilis,* a fungal cell such as a yeast cell or *Aspergillus,* or an animal cell such as a fibroblast, a CHO cell, a COS cell, a NSO cell, a HeLa cell, a BHK cell, a HEK 293 cell or other human cell.

As used herein, the term "expression" or "expressing" refers to a process of transcription from a DNA template into a polynucleotide (such as transcription into an mRNA or other RNA transcripts) and/or a process by which the transcribed mRNA is subsequently translated into a peptide, polypeptide or protein. The transcript and encoded polypeptide can be collectively referred to as a "gene product" or a "gene expression product". As used herein, "expression" of a gene or nucleic acid encompasses not only cell gene expression, but also transcription and translation of one or more nucleic acids in a cloning system or under any other context.

As used herein, the term "linker" refers to a group that connects a protein with a modifying moiety. The group may be an amino acid, an amino acid sequence, or other chemical groups. For example, it may an amino acid (e.g., Glu or Ser), an amino acid derivative, and PEG (polyethylene glycol). In some cases, the "linker" refers to a linear polypeptide formed by connecting one or more amino acid residues through peptide bonds, wherein the amino acid residues may be natural or unnatural, and for example may be modified. The linker of the present invention can be an artificially-synthesized amino acid sequence or a naturally occurring polypeptide sequence, such as a polypeptide with a hinge region function. Such a linker polypeptide is well known in the art. Such a linker may be newly discovered or well known in the art, and examples of it include but are not limited to a linker containing one or more (e.g., 1, 2, 3, 4 or 5) amino acids (e.g., Glu or Ser) or amino acid derivatives (e.g., Ahx, $\beta$-Ala, GABA or Ava), or PEG, etc.

The gRNA of the present invention may contain one or more modifications (e.g., base modification, scaffold modification, etc.) to provide the same function as an unmodified gRNA, or to provide a new or enhanced feature (e.g., improved stability) to the gRNA. Examples of a suitable gRNA containing the modifications include a gRNA containing a modified scaffold or an unnatural internucleoside bond. The gRNA modification includes, for example, a phosphorothioate modification, a 2'-O-methyl modification, a 2'-O-methoxyethyl (MOE) modification, a 2'-deoxy modification, a phosphorothioate internucleotide linkage, a phosphonoacetate (PACE) internucleotide linkage, a phosphorothioate (thioPACE) internucleotide linkage, a locked nucleic acid (LNA) or a cyclohexenyl substituted furanose ring.

The furanose ring of or the furanose ring and internucleotide bond of the gRNA of the present invention can be substituted by a non-furanose group. One such nucleic acid (which has been shown to have excellent hybridization properties) is called a peptide nucleic acid (PNA). In the PNA, a sugar scaffold of the polynucleotide is replaced by an amide-containing scaffold. The furanose ring in the gRNA molecule can also be substituted by a cyclohexenyl ring, and thus is called a cyclohexenyl nucleic acid (CeNA). Another modification includes a locked nucleic acid (LNA), in which a 2'-hydroxyl group is connected to the 4'-carbon atom of a sugar ring to form a 2'-C, 4'-C-oxymethylene bond, thereby forming a bicyclic sugar moiety.

The gRNA of the present invention may also include base modification or substitution. The gRNA of the present invention may contain an unmodified or natural base (e.g., purine bases adenine A and guanine G and pyrimidine bases thymine T, cytosine C and uracil U). The gRNA of the present invention may contain a modified base, including, for example, other synthetic and natural bases such as 5-methylcytosine, 5-hydroxymethylcytosine, xanthine, hypoxanthine, 2-aminoadenine, other derivatives of adenine and guanine, 5-uracil (pseudouracil), 4-thiouracil, other derivatives of cytosine, other derivatives of uracil, and derivatives of thymine.

The modification can be at any position in the molecular structure of the gRNA.

The 5' or 3' terminal of the gRNA may have an additional nucleotide connected to the guide sequence. A non-limiting example is that for example 2 additional guanine nucleotides may be contained at the 5' terminal to improve targeting specificity.

As used herein, the terms "delivering particle", "delivering particle system" and "particle" are used interchangeably. The particle is used for delivering the Cas13 protein, conjugate, gRNA, complex, nucleic acid, composition, etc. of the present invention. It is known that several types of particle delivery systems and/or formulations can be used in different ranges of biomedical applications. Generally speaking, the particle is defined as a small object of which the transport and characteristics are expressed as a whole unit. The particle is further classified according to its diameter. The size of a coarse particle is between 2,500-10,000 nanometers. The size of a fine particle is between 100-2,500 nanometers. The size of an ultrafine particle or a nanoparticle is generally between 1-100 nanometers. Many different conventional techniques can be used for particle characterization (including, for example, characterization of morphology, dimensions, etc.).

The particle delivery system within the scope of the present invention can be provided in any form, including but not limited to: a liposome (including, for example, an immunoliposome), a virion (including, for example, an artificial virion), an extracellular vesicle (including, for example, an exosome, a microvesicle and an apoptotic body), a particle (e.g., a nanoparticle), a microbubble, gene gun, electroporation, sonoporation, calcium phosphate-mediated transfection, cationic transfection, dendritic transfection, heat shock transfection, nuclear transfection, magnetic transfection, lipid transfection, puncture transfection, optical transfection, nucleic acid uptake enhanced by a proprietary agent, and microinjection.

As used herein, the term "exosome" refers to an endogenous nanovesicle that transports certain substances (including but not limited to an RNA and a protein).

As used herein, the term "liposome" is a spherical vesicle structure, which is composed of a single-layer or multi-layer lipid bilayer surrounding an internal aqueous compartment and a relatively impermeable external lipophilic phospholipid bilayer. The liposomes have attracted considerable attention as a drug delivery vectors, because they are biocompatible and non-toxic, can deliver hydrophilic and lipophilic drug molecules, protect their contents from being degraded by plasma enzymes, and transport across a biofilm and a blood-brain barrier. The liposome can be made from several different types of lipids; however, a phospholipid is most commonly used to produce the liposome as the drug carrier. Several other additives can be added into the liposome to modify its structure and characteristics. The liposome can be used for delivery or administration according to the present invention.

The cell of the present invention includes, but is not limited to, a prokaryotic cell such as an *Escherichia coli* cell, and an eukaryotic cell such as a yeast cell, an insect cell, a plant cell and an animal cell (such as a mammalian cell, for example a mouse cell, a human cell, etc., for example a human stem cell, a human stem cell line, such as a human hematopoietic stem cell, a hematopoietic progenitor cell, etc.).

The term eukaryotic cell includes, but is not limited to, a host cell, a cell line and a cell progeny, for example. In some embodiments, the host cell, cell line and cell progeny may be optionally selected from *in vitro, ex vivo* or *in vivo.*

The terms "drug", "medicament", "therapeutic agent" or "agent capable of being used for treatment" are used interchangeably, and refer to a molecule or compound that imparts some beneficial effect when administered to a subject. The beneficial effect includes the realization of diagnostic determination; improving a disease, a symptom, a disorder, or a pathological condition; reducing or preventing the onset of a disease, a symptom, a disorder or a pathological condition; and generally conflicting against a disease, a symptom, a disorder or a pathological condition.

As used herein, the term "subject" includes, but is not limited to, various animals, such as mammals, such as bovine, equine, ovine, porcine, canine, feline, rabbit, rodent (e.g., mouse or rat), non-human primate (e.g., macaque or *cynomolgus macaques)* or human. In certain embodiments, the subject (e.g., human) suffers from a condition (e.g., a condition caused by a disease-associated gene defect).

The term "effective amount" or "therapeutically effective amount" refers to the amount of a medicament sufficient to achieve a beneficial or desired result. The therapeutically effective amount can be changed depending on one or more of the subject and disease condition to be treated, the body weight and age of the subject, the severity of the disease condition, the mode of administration, etc., and can be easily determined by those of ordinary skills in the art. This term is also applicable to providing a dosage for providing an image for detection by any of the imaging methods described here. The specific dosage may vary depending on one or more of the following: the specific medicament as selected, the administration regimen as followed, whether the medicament is administered in combination with other compounds, the dosing time, the tissue to be imaged, and a physical delivery system carrying the medicament.

As used herein, the term a process of "administering..." to an individual can take place *in vitro, ex vivo* or *in vivo.*

As used herein, the term "Conserved Substitution" refers to the substitution (i.e. substitution) between amino acid molecules with similar traits. The traits include, but are not limited to, the ionic property, hydrophobicity and molecular weight of the molecule. Therefore, the substitution can be, for example, (1) substitution among aromatic amino acids (Phe, Trp, Tyr), (2) substitution among nonpolar aliphatic amino acids (Gly, Ala, Val, Leu, Met, Ile, Pro), (3) substitution among uncharged polar amino acids (Ser, Thr, Cys, Asn, Gln), (4) substitution among basic amino acids (Lys, Arg, His), or (5) substitution among acidic amino acids (Asp, Glu).

**Example 1: Screening of Casl3 Protein**

The Cas13 protein of the present invention was obtain by the following method:

1. Annotation for CRISPR and Gene

The (about millions of) proteins of a whole genome was predicted from the microbial genomes of NCBI Gebank and CNGB databases by a software, and then a CRISPR array on the genome was predicted by a CRISPRCasFinder software. The default parameter settings were used for initial screening.

2. Preliminary Screening of Protein

Taking protein sequence similarity of 95% as the standard, we used clustering to remove redundant proteins, remove proteins having a sequence identity of 100% with other proteins and a self-coverage of 100%, and meanwhile filtered out proteins less than 800 aa (amino acids) or greater than 1400 aa, so as to avoid the interference of too long or too short proteins and get hundreds of thousands of proteins.

3. Acquisition of CRISPR-associated protein

The protein sequences within 10 kb upstream and downstream of the CRISPR Array were compared with a known Cas13, and the alignment results with an evalue greater than $1*e^{-5}$ were filtered out.

Then by comparing with the NR library of NCBI and the patent library of EBI, the Cas13 protein having a sequence identity >_ 95% and a self-coverage ≥ 90% was filtered out, and then about 100 candidate proteins were obtained via selection by the inventor.

Upon verified by the experiment, the Cas13 proteins of the present invention Cas 13m.1 (SEQ ID NO: 1), Cas13m.2 (SEQ ID NO: 2), Cas13m.3 (SEQ ID NO: 3), Cas13m.4 (SEQ ID NO: 4), Cas13m.5 (SEQ ID NO: 5), Cas13m.6 (SEQ ID NO: 60), CasRfg.1 (SEQ ID NO: 6), and CasRfg.2 (SEQ ID NO: 7) were obtained finally.

The amino acid sequence of the aforementioned Cas13 protein was as shown in Table 1 below.

Table 1. Amino acid sequence of Cas13 protein

| Name | Amino acid sequence |
|---|---|
| Cas13m.1 (1025 aa) | MNPQARTRKPETPLKPVGNEHFAVFINIARHNAFIAITELSKIYGMTPPNEDELATSRFI AAFDSTTIDVRKLKQRLKRLSSLMPFLKSLQDKTDAEIIGILKDLLSLVNQFRNYYSH YNTSDFLTINSNEFITSDKVSILENIFEKAVLSLTNSEKAYDRFEIISLLKSEKRADKPYF YEFIENNKISEKAIAFFFCLFLDKPNAMKFLKRLKGFKGADTKQFRATLEAYTTYCIQ LPEPKFLSDRPNLAIILDGIEHLKRCPIEVHKTLSFRDKQKFERKVMREDLNGDLQEE NIELLRYDDRFAEFAMRYLDDFDILSDAKKQNTYRFEIQLGKKVVESKKLAEETTEA QDKIPVRFKPVKAFGKLADIPRKKDEAVIDWQQDLEELYAYEPHYKIENHSIGIKKIAT EDTFRLNNPEELPDAYLSEYQLRNIIFLSLESSNKEEFFMACQIRIGKIKSLYKALSAPT HYKESIKQKYNELINNNLLPKPLVKYLTHSLDELPTYKSKAIKKLKFWQDETENLLA EVKRHNEKSEKARKENKFFKSFLKSGQIATWLIKDIQHFLPLQGKLSVLKYNALQAK LAIYNSEELKEMLTDFQVYDTPKGTDRNMPEKGGGHQFIKTVFDKNPQKLPPHWLH FYNDYLNAKKQWIEDKIKFLTAMPDTEAEIMKQQPLFYFLDLGSNYEEGEKIVYFRE NSPAYIAKYCEELLKKPVDLPIALSYDLVANMRTGLEKAKSVTDFIDDKYQSEPPYYH LPRQYDLFKAFGDKPSEKLFATDKKPLHEVYGEYKAKKTDPRTIKKIKGFLDQEQRIR YLKVCDKLLVKILEKYLAKETELKNVQLTDAQGKLILDKVLETEFEMPPYGFKVRLK DYGRYRRFVKDRRLVSMQDYLNQSVFTPDTLITELNLYEKQRSEFLKIVLEFEKRLLS ASDTLNINLADQQTTLGEDKIRDYITHNYLLEVALRNNFISENEARAMLWFRNGALH NQLPDLQKLVGLIDTEEQKTQRYFLKGMEMYKKGIEKIS (SEQ ID NO: 1) |
| Cas13m.2 (1192 aa) | MTILEKYMPLNEMGKIKSLVDELKGIPGAMNATLRQEIFQNIKKTKLQVKFLEEYPET LFTDSGNLIPIIGNNDNSSSGTPSKQEVSDIDANQASRINYLPHETMGEIKSVYGTYIE MAFHNFYLTMHHIYAVVFGEDIMEEAKKEFDKNNTNSTKYFTFDFANERTIWKPMF DRAERAKPEQKEHFEKLVVKHFPFLKAIDALEDRKRKTKIQALCVFSLVLRELRNVY SHYLFYPFKNQVDKYKENIPFVLDMMEILYTGAQREVKGRFGFDDKKMQCAKKYE RNKDHSQRDHQGKIIKAVPKKNFRYNLYKKEDSEAIITPFGLVFLTSLFLEKKYAKILS DKTHCIKYTDQEVLCEIISVYRIRLHIQKLSVTKDTDALALDIINELQRCPKRLFEMLS PDDQQKFRIKPTDSQYADDVLMIRHQDRFAHLLLKYIDDAHLFNCIRFQVSLGRYFFR FYDKSCIDSTGDKRVRSISKNVNGFGRITDIEDYRKEVYGDMIREYEDVHANTSMEK PYITDHHAKYLISNNRIGLYIRKEEDTQCLLPELTPDGARNFAPTCWLSIYELPALAFL LHLYNGDGSRVEEIIQTKVANYQRLFADVRDGKVCPVKDEAELTTILQTYGNIEPSQL PRKLLDYLLKKEICAQDLFNTWAQSKIQRMIAQTDSLLQHLEKDLQAVSDLKQNKFG KKAFVAIKPGHIADFLAHDMMFFQPSMKDCNNKLTGLNFRILQSSMAVYDGNFDELS RIMRSAHIIGNANDACCNPIVMAVCRKHKGFSNIIRFYQAYLKERKAYLQQCANERH YDSLSFLHASQNKWRERTQAYYRSLAAKYLAENYDGVDTTKSIELPRGLFETYIRQE LSEIGSTKSMAGDATKNTSYLIYGYFRQVMSDDAQTFYDTRRCYQLFDVLYRKSPRD NHSYYSTAQIREMLMRSHSKSIRKDIDNYISQTTAAERTKEKERCDALLRKIKDTETE LKVYKIQDILLFLIAKRLLLDRKVENDSAVQMNAINQIRLRNIADGNTLSQKIPISISIK SRKGDPKIIQQDDLKLKNYSQFYSIISDRRLPSLLDLINSRVIKRTDIEDELSNYDKSHP HVLKSVFEFEKHYFDTHPIPSDTAYMALPDTGEMLKESNLTAEKQKEVRKIRNSFAHL SYPSRNITGAASTELPKKAEIISKNLIEHLSNAEIK (SEQ ID NO: 2) |
| Cas13m.3 (1272 aa) | MYHLSDANHGKHIAGTYYEMAMGNFIHTLSHMLVRAGIKVNKLEDNYSIEREIMNL TAPGAYDAQRAALSRLLYRHFPFFGPIMADHTDHILSSKRKKVQSASDDGNLDLGQE LKDEVAGASACQMIRYLATIAGALVYYRNMYSHKNHYDNAQDIAAQQEREQKLAL WLDVVFRGARDILLTRKSHPQPDTDFLTQNGTINYYIEKNGKSAYNPNFYFKPGLKT DNGWVMTDFGKFFFCSLFLRRADAERFAAETDLYVGSPFKITAQERARLQEAENKR AADEQARASSAGFPHIVNPRTIGPSESPQNNIIREMLNMHRARIPRERRIDADMSEGIL AMDIMNELRRCPLSLYNTLSPEAKASFEKTGVTPEGGIVSNLLVRHSDRYPELALRAI DQMELLPTIRFHVRLGSLRFRFYEKKLIDGSHTLRTVQKAVNGFGRWQEVEPRRVEK YTAIQARCQNDKGIDQFLPDSPTTTPYITDWRTTYNIHANRIGLAWNLPQMSDGIYLP NLDTDKGDNLHRKALIDMPAPMCYLSIFDLPALLFYCHIYTHYHGTKYHLPSAESIIQ AKYDALHKFFSFAAAQNHSAEQLREKQLELNLADNEIPDKLRCMMQTKPFFKNGRQ QLSPLGYPIMKNWIGVAEQRKHAAQVLRDVANEAADRLASFEKKHQRVVVGGRDN |

(continued)

| Name | Amino acid sequence |
|------|---------------------|
|  | RYGRRGHADIRHGSLARYLATSMVRWQPALDQPGGDKLTSANHRALAGFLSEYGLH GSNINKLRNVLKEAGLIEGSHPHPFLAHVLESAPANIEALYVAYLKHEQSHATALKNK FTDRNGIVQPSEVPAFVRFNSSRWRNDSATTARRYLQTPPAPGSSDSAEHNAPIMLPD GLFTTHIMTLLNKVLGQNDRVPEEDYLRHDLPRIASIINPNGKTYGAAYIIRAWFDQV ENQDVQPFYDLPRFYREISLLAPRRKPNQELIRDYFSEEQIAQKIQTVPKKQRSEKVG HTIDTEKDIRRYRLQDITLYLTLLDMLTLMLSRNEAERTDRQMKSSTAERVSNMRLVD FDHSFDFDLLGSTSGEAAYSYLHQRSGITISMPALSLRSYGSIFRVLADSRFETLMDAL NRQGVTHVNFGDITSELALYDTLRSHFLLQAHNVEQDAFSAKRGVLENHTSPFFYRS GNLQLDDQGNITNPSTDAIRNHYGELIKILDRYSLKIDKKTKDGKSQDILLRDLMAEL RNAAAHNRYPKADFFFRQFDHFLNTCKPTDSNLTAPNYIRTVLEFLKSIVDNNFTPLL HEESPENESKSE (SEQ ID NO: 3) |
| Cas13m.4 (1183 aa) | MPMSTIIDKYMPLNDWNRIESLIGELRAISGAMNCAVRRAVFENIKKAKLQIKFNDQ YPESIFTDKNGSIPIVGTTEANNPENGGNNECEQSNNYLSIDYLPRVTQEDKKSVYGT YFEMAFHNFFITLHHIYSLIFGEDIMEVAKSEYSQTQTDSFQDDFANKYTVWNPLFTR LRRAKAEQKERFEELAIKHFPFLKALDALKGENRVSKVDALERFSVVVRELRNIYLH YCIIPSDKQKKEYSDNISFIFDLMDLLFTGAKREVKTRFALSDDQMSCADKYEPNSDR SLRDIHGKTLRIVPKKNFRYHLYKVGDDAKIISPFGLVFLASLFLEKKYAKILSDKAHV VRLNDKGVICEMISVYRIRLHINRLSISKSTDTLALDIINELQRCPKKVFELLPPVAQQR FRVKPESSHAPEVLMVRHNDRFVHLLLKYIDDAKLFEHIRFQVSLGRYFFRFYDKICI DTSSEKRVRSICKDVHGFGRISEIEELRREKWKDILREYDEVHANTADEKPYITDHRA NYLIGNNKVGIYLLKEGDEQCIMPELLPNGARNHAPTCWLSTYELPALAFLLHLYNA DGARVEEIIEKQVAGYRRLFADVRDGSVAPVASVEELDELLKGYGDMQACNLPRKM LDYLLMKDVNAHDLFRKWAEAELQQMIEQTDRLSQRIDDDIKAAANMRQNKFGKK SFVAVKPGKIADFLAHDMMLFQPCTEDNSNKLTGLNFRILQSVMAVYNGDFDELSRV LRNAHIIGNATDEMCNPIVMAVCHKSMEFGNIVDFYKAYLRERRIYLERCLRHGDFE SLGFLHASQIRWQERSKEYYRALAARYLVDEYGGTESAKAIELPRGLFEPYIRKELSE MNAMKSLACNSDYNVSYLIYGYFKRVMSDDAQPFYDEKKCYRLFNVLYRKSPHDS PVYRNTAEIRDMLMQNSPNSIRKDIESYLSNTIIADRAKEKERCTALLREMKKCETEL KRYKIQDMLLFLIAKRILSDLPAAHDSAVQMQAISRIHLKDITDGNTLSEKISLSVKVV SKNGYIKKLTQHNLKLKNYSQFYAILSDRRLPSLLDLVRSNYINRNDIEAELDNYDKV HPEVMKAIIGLEKKHFEKHGFDDSGIVPDLSSILAETTMPADKQYEVRKIRNSFAHSH YPGYHVANAGITELPKKAETIFNTLKSSLSDE (SEQ ID NO: 4) |

(continued)

| Name | Amino acid sequence |
|------|---------------------|
| Cas13m.5 (1190 aa) | METTINNRIGKGEYYTEESKEFLAAYFNQAIHNVFIVLNHIAKRFGMDELSSDEELKN WLIGRQREKKRNAIDRQRFLELIDRHFPFLRIANADKKDAKRENDLEDNLALLITLLN DLTER RNKYSH AITHASIESNDRELVWRLYSIYDANINLVKRDYFESNVHTEINEDPY EKQVEHLRRFCMNNDRTKVDEKGKKKPAMPNPRFRTPFLNAETNQLSIYGLVFFVSL FLEKKYAIQMQKNVYGLKDARDTKFKMTNEVFCRSRIIMPRVRLHSDKSTDALVLD MLNELAKAPEVLFDQLTDPFKEKFYIESIDSLEESDIITPVRAIRKQNRFMYFALRYLD ESNAFSKLRFQIDLGNYHYHLYESKINDQTESRHLTRKLFGFGKLIAFEQEFAPEEWK MKSKDLDYYEGATQPFIAKTYPHYHLEENKIGILFNGQAEVQWPHLDVEEHESFPKY KRRANEKADAFLSGNELLAAAFCHHLYASIGKPNTVEKIIRDKYHALRQLFSDLKSG NLQNLLGDNTSNEAISQLLFEKYRLTLSEVPVRLHAFLSGQEQADTKAIARGKLELM AQQNKKRIERFDAMKKAVVKVGKAQYRTLRSGDIGDWLVRDFMRFQPIGYKRNQA GKQEPDLKSKANPKKYQLIQKSLALYEQEKNNLLGLFKSCNLLSSENEHPFLNEVVQ SMPATWQDFYERYLHARSRFLEKCIEKGIKKNSYQACYSFLKIKPQLKDKEKLYQGW DAQMNLPTNMFIDAIHDWFRQTHHESLRTWFTQQEKPHQLIGLIRKYIELAHTDQIQ GFYDMFPLRYDFYKKEFPNGLVLHERINKQKQIWEAQLIQTKKRLEDAASKLKKVK QQVESLPDQELRFRNETEAMVYFTKLFDSSIVKNAILKLQRDQKALRVNTIGEKIIKIY TAKYDAIHQEVRDFKSMLTTEKMIRRVKAEDCVTLFMLTDLMNQSQITIGQEQRTIK LSDIQPMGETQIQGILDAVQVLEQKLDFFSSDEQGKISQVKLGEWTIFSTDTKVKKQG NFKQLLKDRRLNNLAHYILPDIAGGAIRVRRNLLEMELDQYDRNRIPIIKLMYELEYA IYKVDPFSVELKYKKFSQCLREYAQKAVLNVEQVEHLNVLIAI RNAIMH NQYPNRDH LKAIVPFTVSEYAPVQEGLTIARQLLACAQVSVNIILSTISKFE (SEQ ID NO: 5) |
| Cas13m.6 (1188aa) | MKEQKKFSLQGVRHVCGSYFNMALNNYCRTLNGVFIKCKIKFALKEDDFPRSLSSLR KIFSSGPIMPQTEKKVAKMIASVDTAMKLKQQLFKHFPMLGPIMDKTISCMNHHKGS SVADAPLDLCMNAILNFGECLYHC RNFYTH FKPYNSPEDLKLQYDIQHIIALNLGTLF DVSRRIGKKREGLTPEELEFLTGKNRFNQVGKKFLERNDWYLKIEKPSDMKDYDKTI LSDFGMVYLCSIFLAKNYALRLFDESKLFNKETIRNLFSEEQVRFLKEMLVIYRIRTPR GKQLDSHDSKQALAMDMLNELRKCPRPLYDVLSEEYKKRTFYVPVEHENEKTEEY VKMLRSDDRFPYFTLRYIDDMEKFSRIRFQIRLGSYRFKFYDKMNIDGTPRIRSLQKEI NGFGRLSDMENKRKREWKDMFQATEEIDYEDQFGDYQTGVTQFVEDTADTKPYVT NHRAAYNVHSNHIGLIWNDADSIILQDDNKLFFPDLKIDENGKADIYQPSPKASLSVF DFPAMVFYMYLREKTEATKEFPSAEQLIINKYDHLVRFFKDISDGRFGPSENKNAFSK KLKEEYDLKTGEIPEKLLHWLSSESEEDPSEKYAKKLEEEIKLRRERVQRRLEKFNQD LREIRKKDSVPYGKKGHVNIRHSQLAKYLMRSIMEWQPTRNDGKNKLTGQNFNVM TAFLATLGYTSQVKDLRDLFSRANMLEGPNAHPFLKKVLNNNSIKDIQGFYRTYLVE ELNQIEDKQRRIAKAKNVKDTVRQFPFAHFNRMRYQKRDEDYYRNLAKRYLNIGDN EKDKAVILLPDGMFTSYIYDLIMKLPENNEKMRINLASDVAHCNSSFLISRFFENIRND YAQPFYREERTYELFSILNNKKVRNTLQPLFISPHDINIQLTEKEKDGKGRLILQKIDHF CKSITQKGNFNNVEEAKEATSRKLKHLITDCKNNERDIRRYKTQDMVIYLMARDILK DIIPDSEKDKYAKDRKLLLKDVCEEGFLRQAVKMEYEYSIEEKGKRTRTVKITHPNM SLKNYGEFHRLLNDERLKSLLQQLANMDEIDYTDLMGEFADYDQKRSEIFRLAQSIE KHLYEQNEQGLNDEKSDLFYHTRYNGKKIPRRNSFSSLLELIGEEESQMTETDKKQTI SI RNAFGH NTYKVSLAEMNATELPNVAKTILKKMEELRNKL (SEQ ID NO: 60) |

(continued)

| Name | Amino acid sequence |
|---|---|
| CasRfg.1 (1158 aa) | MKEKIKNKSSIIRIIMSNYDDKGLKEFKVLYNKQGGVDTFTCKTDIVDGTIIFLEIEKH LRDFGDDFSWDISSDGKSVEITKLINGKETRKYKVSIKNSSTKDKKNLVELEVEDLKE SAIDRRRTKSSTKRVLLSKDVMERYAEIAFSKKERWEEIDSQKIYKVKRFLDYRSNML IYFQFINDFLTKGIPDELDKNGEIKQLELWKLIDDDETISDKNLNQVSKNLYTYISQEIK DSQTRAENNREKNKEKEHFKEFYAFNDISEESIREDVKKFIYLYANL<u>RHNLMH</u>YNYS FFENLFEGKDLVIEKTKSLLSSTLDLNIFKELSNIVELREENKTNYLDDETTIRVLGKE KKAKTLHKIYSILCSRKNGFNKFINSFFSTDGIEEEFLKSEIKKDFLERLNWVEKSLIEK INNPPSDTKLKYKNDKTIENMTKEKEEKLELISLLNPQVSDYKTENFTPYYWDIHQSP SYKKLYNDRKVLVSELSKLIAIGINSDTKKRITDLNAELLKIKIKMEKITKLNSKIRLQ YKLQMAFGFIYANYSKVYKEKRVLNINGFVQNFDPTKLNKEKELESRLIYLKAPYNI FEDNKSLDFNMKIVENIPVSEKSIFRIKPENNLSKFYILSYLLLPVELRGDFLGYVKHH YYGIKNVDFEEIPDIKEDKPNENSDSFFHNLRLFEKNSKKFELIKYRLVEFGNLKDHLP RIYEKFGIKPDVLEYIENSGNKDSKLFDRNILLPIMKYYQHIFKLLNDIEVHALLRFSE KDSISLDESIKECSKGKFLNFGKLLFLSRYGLEAKKDNKFKDIFNRENGLSITKDDAK TERKKYFEIFET<u>RNKIAH</u>LNYKQLFHDLLFDSNININKELEGIIQETKTIGLNAQTLGY NFLNDFYMRKEMFISNQKKSSMTLINNPLSKDKDTKEIGLLKLYGLSKSQPKDLILAK YKELMNLIEKTEDSILKKKDFLPVKEVSITVKKSTPNKKGIMVELPEILQIKDLNEMD LLAYASNIRGKLHKDSSDLFGIYKKLTIKELKKKLINLFIKGEKRYLNLELVNKTGYM AIYESTGLYPKSYEILNHEISFSEISMKNWYEHDFKPIFQIDGSLPNNTDYKNGVFIYTS PYEFRDKELMKKQRTVHKRDIEKTFYNENDMDYTGIYNQKIKALY (SEQ ID NO: 6) |
| CasRfg.2 (931 aa) | MMGNKKSVAKANGLKSTFVLGENTAYMTSFGRGNAAQPEKHIRDATVTDIQHTFRA KTDGGRTVHIEGRVGASDVLLPDAANQLHAKDAVEQMYFGKAFSDNIHIQIAYNIM DIKKIFGVYANIIVHTVNNLCCDGDKQDDFLGMFKTQNRYQVAAWAHKIVSLHLVK NELRGGGFFMDQEVWRAHVRTDFKSLNLAVNAFMKKYPQKYPYWSVKIVSDFIVQ EMGIKNKVILEKAAESYAEFETVAKRLEKSAYYFSDIFAGKDGKFDEQKAFDLLRVL GMM<u>RQEAFH</u>EKNSSASWLYNLDAEADEDIKAALRTVVDTKVNGINTNFAKQNKVN LLVLQEIYPQKSKADLVREYYDFSVRKAFKNLGFSVKTLRETMCAFDAASVITDKQY DTVRGKLYSLFDFVIYNYCLENEAVCNAFVEELRANLDPENKTALYQTLAEKVWAEI GDIVLQRILPQMHAKKIQERSKETDAETVEMQGYVQAPKDLSLFSKAVYCISMFLDG KEINSFLSALINKFENISSLCAVLAYNGLEPEFVAPTFFADSQAIAEDLRYIKSIARMSK GKKATKDSPVTVKEMQYFDAAAVLGETDTEKVKAAFHLGDKSASTADKAFRNFVV NNVINSNRFVYVVRFINPKNAREIMQNRALIAFVLKDIPQSQLVRYCGTAGIACNADE PNTEAMVNALADMLLQVRFDAFSNVQQKVKADSAEAVQKEKYKAIIGLYLTVLYLL VKTLVKINMNYAIAFGILERDCQIMNQKHGKNPKRDRDAFYMREQQNKQYVYNAR AITELFIENGWLNKRVQKSVENNAALYSDEAFYK<u>YRNLVAH</u>LNVISALPKYAKNITK VKSLFDVYHYILFLSLCEDKYSNLPEAVTKSLCKNGKTMLENAREYQTVCKDFLYGL NTPFAYNAARYINLSNREKFLAGFGK (SEQ ID NO: 7) |

In the aforementioned sequence, two RxxxxH (x represents any amino acid residue) motifs in each Cas13 protein were underlined. In the sequences of some Cas13 proteins (such as Cas13m.1, Cas13m.3), there were many sequences that satisfied the form of RxxxxH. However, by utilizing an online MAFFT v7.487 program (an E-INS-i algorithm, while others being default parameter settings), the amino acid sequences of the 5 proteins Cas13m.1-Cas13m.5 or the 6 proteins Cas13m.1-Cas13m.6 were aligned in multiple sequences, and the positions corresponding to the RxxxxH motifs of other proteins were identified as the RxxxxH motifs of the catalytic activity centers of the Cas13m.1 and Cas13m.3 proteins, which were also underlined in the aforementioned table. The alignment result also showed that the 6 proteins, Cas13m.1-Cas13m.6, contained RNxYxH and RNxxxH motifs from the N-terminal to the C-terminal sequentially, and X was independently selected from naturally occurring amino acid residues.

Additionally, the CasRfg.1 and CasRfg.2 proteins contained a RxxxxH motif and a RNxxxH motif from the N-terminal to the C-terminal sequentially.

The genomic sequence sources of the aforementioned Cas13 proteins were shown in Table 2 below.

Table 2. Source of genome sequence of Cas13 protein

| Protein | Database | Genome No, | The position of corresponding coding sequence in the genome | Annotations on the source of species in the database |
|---|---|---|---|---|
| Cas13m.1 | NCBI Genbank | GCA_01 3298125 .1 | JAAFJP010000015.1:94 576:97653:+ | Species name: Cytophagales bacterium (CFB group bacteria) Isolate: bin5.concoct.b 16b 17b 19.071 |
| Cas 13m.2 | CNGB | CNA001 1077 | LW1-s151260_scaffolds_8411 :1555:5133:+ | Species name: metagenome |
| Cas 13m.3 | NCBI Genbank | GCA_90 2762805 .1 | CACWPQ010000015.1: 53195:57013:+ | Name of species: uncultured *Bacteroidetes bacterium* (CFB group) Isolate: RUG10805 |
| Cas13m.4 | CNGB | CNA000 7373 | Boars10_scaffolds_11462:11 216:14767:+ | Species name: metagenome |
| Cas13m.5 | NCBI Genbank | GCA_01 3298545 .1 | JAAFIA010000070.1:91 3:4485:- | Name of species: *Bacteroidetes bacterium* (CFB group) Isolate: bin17.concoct.ball.095 |
| Cas13m.6 | NCBI Genbank | GCA_90 2779095 .1 | GCA_902779095.1:CA CZAB010000017.1:588 00:62366:+ | Name of species: uncultured Prevotellaceae bacterium (CFB group) |
| CasRfg. 1 | NCBI Genbank | GCA_00 3940745 .1 | RR7U011077219.1: 544 1:8917:+ | Name of species: wastewater metagenome (metagenomes) Isolate: WW |
| CasRfg.2 | CNGB | CNA000 9477 | F4286_scaffolds_12067: 485:3280:+ | Species name: metagenome |

Note: NCBI National Center for Biotechnology Information; and CNGB China National GeneBank.

The natural (wild type) DNA coding sequence of the aforementioned Cas13 protein was as follows:

the wild-type DNA coding sequence of the Cas13 protein Cas13m.1 as shown in SEQ ID NO: 8;
the wild-type DNA coding sequence of the Cas13 protein Cas13m.2 as shown in SEQ ID NO: 9;
the wild-type DNA coding sequence of the Cas13 protein Cas13m.3 as shown in SEQ ID NO: 10;
the wild-type DNA coding sequence of the Cas13 protein Cas13m.4 as shown in SEQ ID NO: 11;
the wild-type DNA coding sequence of the Cas13 protein Cas13m.5 as shown in SEQ ID NO: 12;
the wild-type DNA coding sequence of the Cas13 protein Cas13m.6 as shown in SEQ ID NO: 61;
the wild-type DNA coding sequence of the Cas 13 protein CasRfg.1 as shown in SEQ ID NO: 13;
the wild-type DNA coding sequence of the Cas13 protein CasRfg.2 as shown in SEQ ID NO: 14.

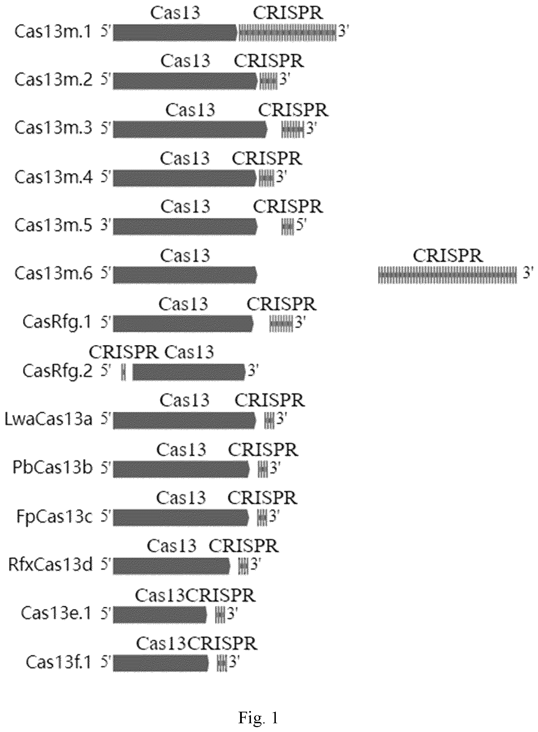

The locus structure of the aforementioned Cast 3 protein was shown in Fig. 1, and in Fig. 1 the locus structure of the Cas13 protein of the present invention was compared with those of various published subtypes of Cas13, wherein CRISPR represented the CRISPR Array (a DNA sequence containing a corresponding DR sequence), and Cas13e.1 and Cas13f.1 were derived from the Chinese patent with the publication number of CN112410377A. It could be seen from the figure that the locus structures of Cas13m.1-Cas13m.5 had basically the same characteristics, and the locus structures of Cas13m.1-Cas13m.6 had basically the same characteristics.

Table 3 below listed the corresponding direct repeat (DR) sequences of the aforementioned Cas13 proteins:

Table 3. The corresponding direct repeat (DR) sequences of the Cas13 proteins

| Cas13 protein | Corresponding direct repeat sequence | Sequence number |
|---|---|---|
| Cas13m.1 | GUUGUUACAGCCCUUAGUUUGUAGGGUAAUGACAAC | SEQ ID NO: 15 |
| Cas13m.2 | GUUGUAGAUGACCUCGUUUUGGAGGGGAAACACAAC | SEQ ID NO: 16 |
| Cas13m.3 | GUUGUAGAAGCCGUUCAUUCGGGACGGUAUGACAAC | SEQ ID NO: 17 |
| Cas13m.4 | GUUGUAAAUACCCACGUUUUGGUGGGCUAAUACAAC | SEQ ID NO: 18 |
| Cas13m.5 | GUUGUGUGUGCCUUUCAAAUUGAAGGCGUUCCCAAC | SEQ ID NO: 19 |
| Cas13m.6 | GUUGUAGAAGCCUAUCGUUAGGAUAGGUAUGACAAC | SEQ ID NO: 62 |
| CasRfg.1 | AUGACUAUACCAGCAAUGGCUGGAUUAAAAC | SEQ ID NO: 20 |
| CasRfg.2 | GGUUUUACACCCGUGUAAAACUACACAGUUCUAAAAC | SEQ ID NO: 21 |

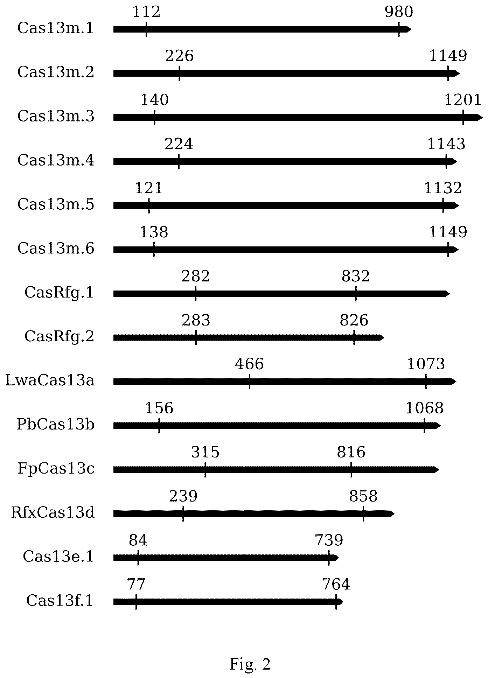

Fig. 2 showed the position of the RxxxxH motif of each Cas13 protein in the amino acid chain in the present invention. Two RxxxxH motifs of Cas13m.1, Cas13m.2, Cas13m.3, Cas13m.4, Cas13m.5 and Cas13m.6 proteins were obviously spaced far apart, which was basically spaced more than 920 aa apart except that of Cas13m.1. Two RxxxxH motifs of Cas13m.2 were spaced 923 aa apart, two RxxxxH motifs of Cas13m.3 were even spaced 1,061 aa apart, two RxxxxH motifs of Cas13m.5 were spaced 1,011 aa apart, and two RxxxxH motifs of Cas13m.6 were spaced 1,011 aa apart.

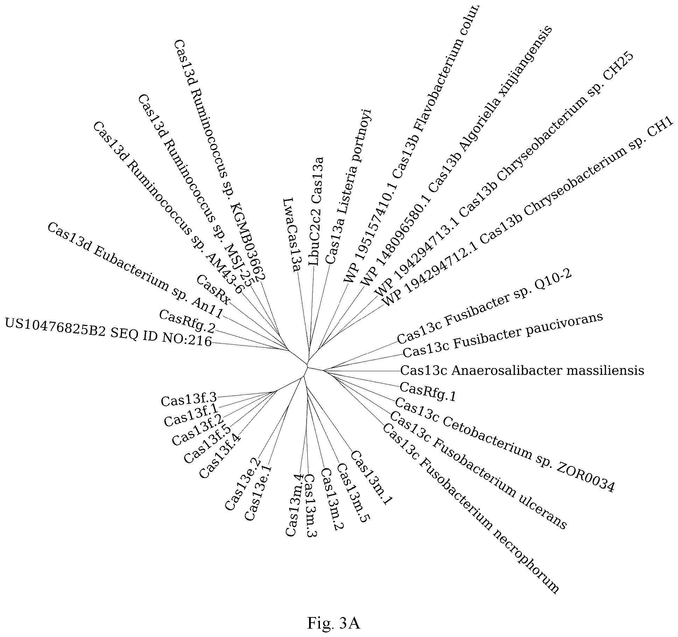

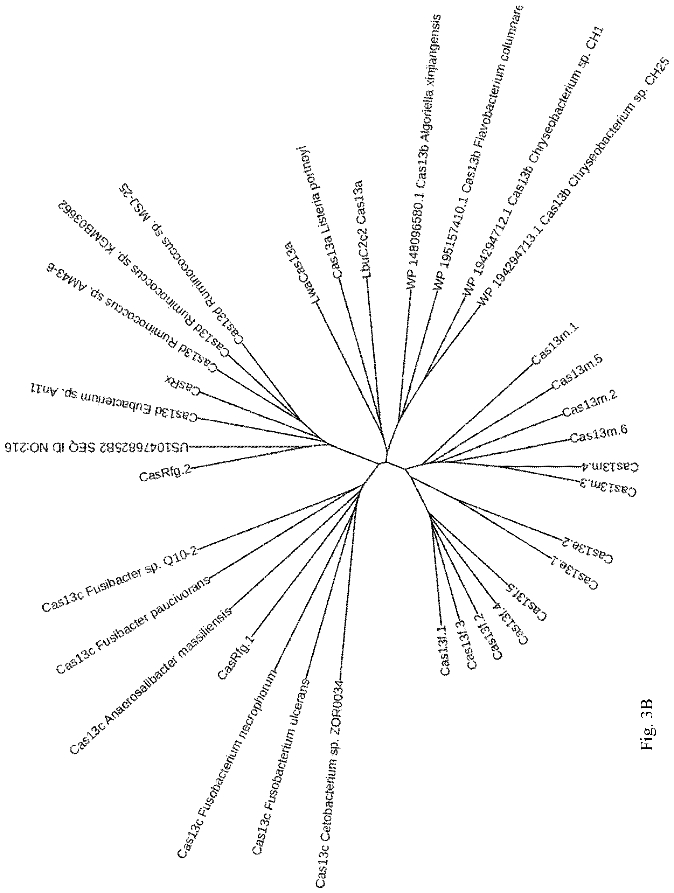

On-line MAFFT version 7(E-INS-i algorithm) was utilized to construct phylogenetic trees for the newly discovered Cas13 protein (Cas13m.1-Cas13m.5 or Cas13m.1-Cas13m.6) of the present invention and the previously discovered various Cas13 subtypes (Cas13a, Cas13b, Cas13c, Cas13d, Cas13e and Cas13f), wherein partial protein sequences were published in NCBI, and Cas13e and Cas13f were available from the patent with the publication number of CN112410377A. The results showed that the Cas13m.1-Cas13m.5 or Cas13m.1-Cas13m.6 proteins of the present invention were clustered into groups on the phylogenetic tree, and other Cas13a/b/c/d/e/f subtypes were also clustered to be distributed in groups individually. The details were as shown in A and B in Figs. 3A and 3B.

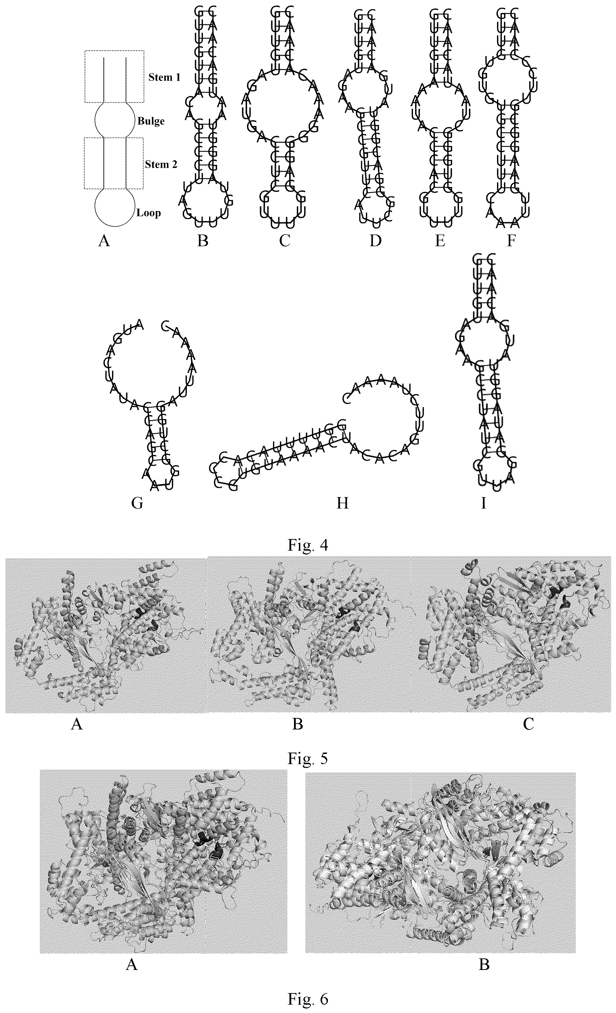

The RNA secondary structures of the corresponding direct repeat sequences of Cas13m.1-Cas13m.6, CasRfg.1 and CasRfg.2 proteins of the present invention were predicted by RNAfold. It was as shown in Fig. 4. It could be seen from the figure that the DR sequences corresponding to Cas13m.1-Cas13m.6 had conserved secondary structures.

We used RNAfold to further analyze the RNA secondary structure of the aforementioned direct repeat sequences. As shown in Fig. 4, the corresponding direct repeat sequences of Cas13m.1, Cas13m.2, Cas13m.3, Cas13m.4 and Cas13m.5 had the following characteristics: obviously, they all had a conserved secondary structure, wherein A was a schematic diagram of the conserved secondary structure, including a complementary paired first stem (stem 1), a non-complementary bulge structure (bulge), a complementary paired second stem (stem 2), and a non-complementary loop structure (loop structure), the stem 1 and the stem 2 respectively contained complementary paired bases; and B-F were the secondary structures of the corresponding direct repeat sequences of Cas13m.1, Cas13m.2, Cas13m.3, Cas13m.4 and Cas13m.5 respectively, wherein the stem 1 contained 4 base pairs (5'-GUUG-3'), 5 base pairs (5'-GUUGU-3'), 6 base pairs (5'-GUUGUA-3') or 7 base pairs (5'-GUUGUUA-3'). The direct repeat sequence of Cas13m.6 also had the common structural characteristics as above. G and H were the secondary structures of the direct repeat sequences corresponding to CasRfg.1 and CasRfg.2, respectively.

The three-dimensional structures of the Cas13m proteins were predicted by using the protein structure database program AlphaFold v2.0, as shown in Fig. 5, wherein A, B and C were Cas13m.2, Cas13m.3 and Cas13m.6, respectively. Although two RxxxxH motifs (dark marks) were spaced far apart in the amino acid chain, they were very close in spatial location.

Then, the proteins were superimposed by PyMOL V2.5.1, and the results were as shown in Fig. 6, wherein A was the superimposition result of Cas13m.2 and Cas13m.3, and B was the superimposition result of Cas13m.3 and Cas13m.6. The results showed that Cas13m.2 and Cas13m.3 had the similar three-dimensional structure (RMSD = 2.402), and Cas13m.3 and Cas13m.6 had the similar three-dimensional structure (RMSD = 2.368).

By alignment of the CasRfg.1 protein with the Cas13 protein included in NCBI by BLASTp, it was found that the evalue value for alignment with Cas13c was the lowest compared with those of other Cas13 subtypes; and as combined with the phylogenetic tree analysis in Figs. 3A-3B, CasRfg.1 was classified as a Cas13c subtype. By alignment of the CasRfg.2 protein with the Cas13 protein included in NCBI by BLASTp, it was found that the evalue value for alignment with Cas13d was the lowest compared with those of other Cas13 subtypes; and as combined with the phylogenetic tree analysis in Figs. 3A-3B, CasRfg.2 was classified as a Cas13d subtype.

**Example 2: Preparation, Separation and Purification of Cas13 Protein**

(1) Construction of vector

1. a pET28a vector plasmid was taken, cleaved by double enzyme digestion via BamHI and XhoI, and subjected to agarose gel electrophoresis. A linearized vector was recovered by cutting the gel, an artificially synthesized DNA fragment containing the coding sequence of a recombinant protein (including the protein sequence and nuclear localization sequence of Example 1) was inserted into the cloning area of the vector pET28a by homologous recombination, and the vector was transformed into Stbl3 competent by a reaction solution, coated onto an LB plate with kanamycin sulfate resistance, and incubated overnight at 37°C, and clones were picked for sequencing and identification.

The constructed recombinant vectors were named Cas13m.1-pET28a, Cas13m.2-pET28a, Cas13m.3-pET28a, Cas13m.4-pET28a, Cas13m.5-pET28a, CasRfg.1-pET28a and CasRfg.2-pET28a, respectively.

The recombinant vectors were respectively used for expressing a Cas13m.1 recombinant protein (with the sequence of SEQ ID NO: 22), a Cas13m.2 recombinant protein (with the sequence of SEQ ID NO: 23), a Cas13m.3 recombinant protein (with the sequence of SEQ ID NO: 24)), a Cas13m.4 recombinant protein (with the sequence of SEQ ID NO: 25), a Cas13m.5 recombinant protein (with the sequence of SEQ ID NO: 26), a CasRfg.1 recombinant protein (with the sequence of SEQ ID NO: 27), and a CasRfg.2 recombinant protein (with the sequence of SEQ ID NO: 28).

The architecture of the recombinant Cas13 series proteins was His tag-NLS-Cas13-SV40 NLS-nucleoplasmin NLS.

2. Positive clones with correct sequences were incubated overnight, subjected to plasmid extraction, then transformed into an expression strain Rosetta (DE3), coated onto an LB plate with kanamycin sulfate resistance, and incubated overnight at 37°C.

(2) Protein Expression

1. Monoclones were picked and plated into an LB culture solution containing 5 ml of kanamycin sulfate resistance, and incubated overnight at 37°C.
2. They were transferred into 500 ml of an LB culture solution with kanamycin sulfate resistance at the ratio of 1:100, cultured at 220 rpm at 37°C until the OD value was 0.6, added with IPTG to a final concentration of 0.2 mM, and induced at 16°C for 24 h.
3. Collection of bacteria by centrifuging: the bacteria were rinsed with 15 ml PBS, then centrifuged for collection, added with a lysis buffer for ultrasonic crushing, and centrifuged at 10,000 g for 30 min to obtain a supernatant containing the recombinant protein, and the supernatant was filtered through a 0.45 $\mu$m filter membrane before purification on a column.

(3) protein purification

The architecture of the recombinant Cas13 series proteins contained a NLS sequence, and the aforementioned recombinant Cas13 series proteins were purified by IMAC (Ni Sepharose 6 Fast Flow, CYTIVA) with 6 His at the N-terminal as a purification tag. Upon SDS-PAGE electrophoresis, it could be seen that a band of various purified recombinant proteins was presented in the interval of 100-250 kDa.

**Example 3: Preparation, separation and purification of Cas13m.6**

A recombinant vector Cas13m.6-pET28a (with the sequence of SEQ ID NO: 83) was constructed by employing the same method as that of the aforementioned Example 2, and transformed into an expression strain BL21-Codon-Plus(DE3)-RIPL. Subsequently, a Cas13m.6 recombinant protein (with an architecture of His tag-NLS-Cas13-SV40 NLS-nucleoplasmin NLS) was expressed and purified by the same method as above. Upon SDS-PAGE electrophoresis, it could be seen that a band of the finally purified recombinant Cas13m.6 protein was presented in the interval of 100-250 kDa.

**Example 4: Editing activity on an exogenous gene in a cell**

1. Synthesizing of an EGFP-targeting vector to be verified

EGFP (enhanced green fluorescent protein) was used as an exogenous reporter gene, and its nucleic acid sequence (720 bp) was as shown in SEQ ID NO: 29.
The sequence of an EGFP-targeting spacer was tgccgttcttctgcttgtcggccatgatat (SEQ ID NO: 30).
The sequence of the exogenous EGFP expression vector was as shown in SEQ ID NO: 31, the sequence of the Cas13m.2 verification vector was as shown in SEQ ID NO: 32, the sequence of the Cas13m.3 verification vector was as shown in SEQ ID NO: 33, the sequence of the Cas13m.5 verification vector was as shown in SEQ ID NO: 34, and the sequence of the CasRfg.2 verification vector was as shown in SEQ ID NO: 35.
The Cas13m.1 verification vector and the Cas13m.4 verification vector both had the same nucleotide backbone sequence as that of the Cas13m.3 verification vector, except that the coding sequence of Cast 3 protein and the coding sequence of DR sequence had been replaced accordingly. The CasRfg.1 verification vector had the same nucleotide backbone sequence as that of the CasRfg.2 verification vector, except that the coding sequence ofCas13 protein and the coding sequence of DR sequence had been replaced accordingly.
The aforementioned verification vector contained a codon-optimized coding sequence of Cas13 protein, which could express a Cas13 protein linked with NLS, and could also express a gRNA which could target EGFP and contained the corresponding DR sequence of Cas13. The guide sequence of the gRNA corresponded to the aforementioned spacer sequence (SEQ ID NO: 30).
All the aforementioned vectors were synthesized by a reagent company by a conventional method.

2. Transfection of a 293T cell with the vector to be verified

A plasmid expressing the exogenous gene EGFP (referred to as EGFP for short) was transfected into a 293T cell in a 24-well plate with the aforementioned various plasmids as verification vectors at a ratio of 1:2 (300 ng:600 ng) respectively.
The transfection method was as follows:
The 293T cells were digested by trypsin (0.25% of Trypsin, EDTA, Thermo, 11058021), counted, and plated into a 24-well plates at $2 \times 10^5$ cells according to 500 μL per well.
For each transfected sample, the complex was prepared according to the following steps:

a. each well of the 24-well plate into which the cells were added, were added with 50 μL of serum-free Opti-MEM I (Thermo, 25200056) reduced serum medium for dilution of the aforementioned plasmid DNA, and mixed gently; and
b. it was gently mixed with Lipofectamine 2000 (Thermo, 11668019) before use, and then 1.8 μL of the Lipofectamine 2000 was diluted in each well, i.e., in 50 μL of the Opti-MEM I medium. It was incubated at room temperature for 5 minutes. Note: it was continued to perform step c within 25 minutes;
c. after incubation for 5 minutes, the diluted DNA was combined with the diluted Lipofectamine 2000. They were gently mixed and incubated at room temperature for 20 minutes (the solution might be cloudy visually). The complex was stabilized at room temperature for 6 hours.

The complex was added into the 293T cells and mixed, and detected by a flow cytometer after 48 h.

3. Detection of the down-regulation effect of Cas13 protein on EGFP expression by flow cytometer

The description of cells and plasmids as used was as shown in Table 4 below:

Table 4. Grouping of transfected cells

| Groups | Transfected with the EGFP vector | Transfected with the EGFP-targeting Cas13 verification vector | Description |
|---|---|---|---|
| 293T | / | / | Cell control |
| EGFP | * | / | Control transfected with EGFP only |
| CasRfg.1 | * | * | Verification vector |
| Cas13m.1 | * | * | Verification vector |
| Cas13m.2 | * | * | Verification vector |
| Cas13m.3 | * | * | Verification vector |
| Cas13m.4 | * | * | Verification vector |
| Cas13m.5 | * | * | Verification vector |
| CasRfg.2 | * | * | Verification vector |

Note: * represented containing related items, and / represented there was no related items.

The 293T cells obtained after 48 h of transfection in the aforementioned step 2 were digested with trypsin (0.25% of Trypsin, EDTA, Thermo, 11058021), and centrifuged at 300 g for 5 min, the supernatant was discarded, and the cells in each well were resuspended with 500 μL of PBS. The EGFP fluorescent expression was detected by a flow cytometer, wherein the cell debris were removed by FCS-A and SSC-A gating, and then detection was conducted by the flow cytometer.

The Mean-FITC-A results of the FITC channel were collected and recorded, and the downregulation amplitude was calculated according to the following calculation formula:

$$\text{downregulation amplitude (\%)} = (a - x) \div a \times 100,$$

wherein the GFP fluorescence of the EGFP group was a, and the GFP fluorescence of other groups was x.

The blank control group did not participate in the comparison.

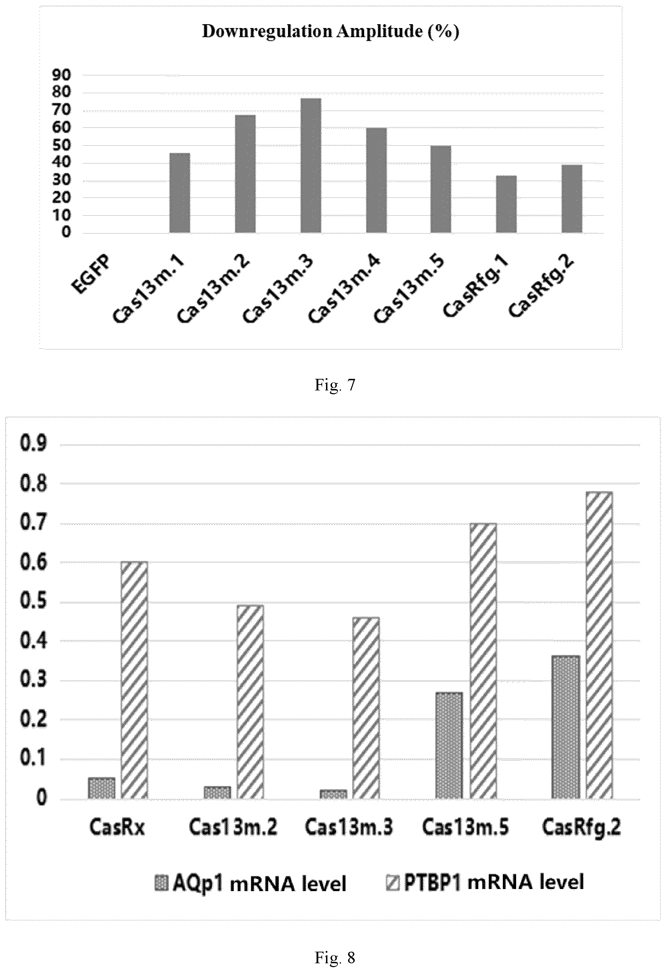

The experiment of this example was conducted in triplicate. The downregulation amplitude results were as shown in Table 5 below and Fig. 7, and the result data was the average of three tests.

Table 5. Results of GFP fluorescence as detected by a flow cytometer

| Groups | Downregulation amplitude (%) |
|--------|------------------------------|
| EGFP | 0.00 |
| Cas13m.1 | 46 |
| Cas13m.2 | 67.31 |
| Cas13m.3 | 76.82 |
| Cas13m.4 | 59.73 |
| Cas13m.5 | 50.08 |
| CasRfg.1 | 33 |
| CasRfg.2 | 39.19 |
| Note: according to the average of three tests, the GFP fluorescence intensity of the 293T group was 1,073.55, and the GFP fluorescence intensity of the EGFP group was 8,052,219.55. | |

It could be seen from the table that the aforementioned Cas13 protein could significantly down-regulate the expression of EGFP, which proved that it could effectively reduce the mRNA level and exert its editing activity in eukaryotic cells under the guidance of the gRNA. Cas13m.2 and Cas13m.3 down-regulated the expression of EGFP at the largest amplitude.

**Example 5: Verification of Endogenous Gene Editing Efficiency**

1. Construction of an editing vector targeting endogenous genes AQp1 and PTBP 1

The codon-optimized Cas13m.2, Cas13m.3, Cas13m.5, CasRfg.2, and CasRx (one of Cas 13 d) expression vectors carrying an universal gRNA scaffold expression cassette were respectively synthesized in a reagent company, which were Cas13m.2-BsaI (with the sequence as shown in SEQ ID NO: 36). Cas13m.3-BsaI (with the sequence as shown in SEQ ID NO: 37), Cas13m.5-Bsa (with the sequence as shown in SEQ ID NO: 38), CasRfg.2-BsaI (with the sequence as shown in SEQ ID NO: 39) and CasRx-BpiI (with the sequence as shown in SEQ ID NO: 40).

The endogenous sites selected in the experiment were AQp1 and PTBP1, wherein AQp1 was verified by using a 293T cell line (293T-AQp1 cells) with high expression of AQp1, and PTBP1 was verified by using a 293T cell line.

A method for constructing the 293T cell line with high expression of AQp1: a vector Lv-AQp1-T2a-GFP with over-expression of the AQP1 gene and the EGFP gene was constructed with the sequence as shown in SEQ ID NO: 41. AQp1 and EGFP were spaced apart by a 2A peptide. The Lv-AQp1-T2a-GFP plasmid was packaged into a lentivirus and transduced into 293T cells to form a cell line stably overexpressing the AQp1 gene.

The guide sequence of an AQp1-targeting gRNA was selected as GAAGACAAAGAGGGUCGUGG (SEQ ID NO: 42)

The guide sequence of an PTBP1-targeting gRNA was selected as: GUGGUUGGAGAACUGGAUGUAGAUGGGCUG (SEQ ID NO: 43)

A target site-targeted fragment was obtained by using a primer annealing manner, and the primers of it were as follows:

PTBP1-targeting group:

Cas13m.2 group:

F: CACCGTGGTTGGAGAACTGGATGTAGATGGGCTG (SEQ ID NO: 44)
R: CAACCAGCCCATCTACATCCAGTTCTCCAACCAC (SEQ ID NO: 45)

Cas13m.3 group:

F: CACCGTGGTTGGAGAACTGGATGTAGATGGGCTG (SEQ ID NO: 44)
R: CAACCAGCCCATCTACATCCAGTTCTCCAACCAC (SEQ ID NO: 45)

Cas13m.5 group:

F: CACCGTGGTTGGAGAACTGGATGTAGATGGGCTG (SEQ ID NO: 44)
R: CAACCAGCCCATCTACATCCAGTTCTCCAACCAC (SEQ ID NO: 45)

CasRfg.2 Group:

F: AAACGTGGTTGGAGAACTGGATGTAGATGGGCTG (SEQ ID NO: 46)
R: AAAACAGCCCATCTACATCCAGTTCTCCAACCAC (SEQ ID NO: 47)

CasRx group:

F: AAACGTGGTTGGAGAACTGGATGTAGATGGGCTG (SEQ ID NO: 46)
R: CTTGCAGCCCATCTACATCCAGTTCTCCAACCAC (SEQ ID NO: 48)

AQp1-targeting group:

Cas13m.2 group:

F: CACCGAAGACAAAGAGGGTCGTGG (SEQ ID NO: 49)
R: CAACCCACGACCCTCTTTGTCTTC (SEQ ID NO: 50)

Cas13m.3 group:

F: CACCGAAGACAAAGAGGGTCGTGG (SEQ ID NO: 49)
R: CAACCCACGACCCTCTTTGTCTTC (SEQ ID NO: 50)

Cas13m.5 group:

F: CACCGAAGACAAAGAGGGTCGTGG (SEQ ID NO: 49)
R: CAACCCACGACCCTCTTTGTCTTC (SEQ ID NO: 50)

CasRfg.2 Group:

F: AAACGAAGACAAAGAGGGTCGTGG (SEQ ID NO: 51)
R: AAAACCACGACCCTCTTTGTCTTC (SEQ ID NO: 52)

CasRx group:

F: AAACGAAGACAAAGAGGGTCGTGG (SEQ ID NO: 51)
R: CTTGCCACGACCCTCTTTGTCTTC (SEQ ID NO: 53)

The primer annealing reaction system was as follows: it was incubated in a PCR instrument at 95°C for 5 minutes, then immediately taken out and incubated on ice for 5 minutes, so that the primers were annealed to each other to form a double-stranded DNA with sticky ends:

| | |
|---|---|
| Oligo-F (10 μM) | 2 μl |
| Oligo-R (10 μM) | 2 μl |
| 2 μl of 10× endonuclease reaction buffer | 2 μl |
| Deionized water | up to 20 μl |

After the synthesized Cas13m-BsaI and CasRfg-BsaI plasmids were digested with a BsaI endonuclease, the annealed products and the backbones purified and recovered after the digestion were subjected to T4 linkage respectively. After the transformation into *Escherichia coli,* the positive clones were selected and the verification vector plasmids targeting the endogenous gene mRNA were extracted for cell experiment verification. After the synthesized CasRx-BpiI plasmid was digested with a BsaI endonuclease, the annealed products and the backbones purified and recovered after the digestion were subjected to T4 linkage. After the transformation into *Escherichia coli,* the positive clones were selected and the verification vector plasmids targeting the endogenous gene mRNA were extracted for cell experiment verification.

2. Transfection of 293T cells and 293T-AQp1 cells with the vector to be verified

293T-AQp1 cells were transfected with the AQp1-targeting plasmids (verification vector plasmids targeting the endogenous gene mRNA) of Cas13m.2, Cas13m.3, Cas13m.5, CasRfg.2 and CasRx obtained in the previous step at 800 ng in a 24-well plate. The negative control group was transfected with the CasRx-BpiI plasmid.

The 293T cells were transfected with the PTBP1-targeting plasmids of Cas13m.2, Cas13m.3, Cas13m.5, Cas-Rfg.2 and CasRx at 800 ng in a 24-well plate. The negative control group was transfected with the CasRx-BpiI plasmid.

The transfection method was as follows:

1) The cells were digested by trypsin (0.25% of Trypsin, EDTA, Thermo, 11058021), counted, and plated into a 24-well plates at $2 \times 10^5$ cells according to 500 μL per well.
2) For each transfected sample, the complex was prepared according to the following steps:

a. each well of the 24-well plate into which the cells were added, were added with 50 μL of serum-free Opti-MEM I (Thermo, 25200056) reduced serum medium for dilution of the aforementioned plasmid DNA, and mixed gently;
b. it was gently mixed with Lipofectamine 2000 (Thermo, 11668019) before use, and then 1.8 μL of the Lipo-fectamine 2000 was diluted in each well, i.e., in 50 μL of the Opti-MEM I medium. It was incubated at room temperature for 5 minutes. Note: it was continued to perform step c within 25 minutes;
c. after incubation for 5 minutes, the diluted DNA was combined with the diluted Lipofectamine 2000. They were gently mixed and incubated at room temperature for 20 minutes (the solution might be cloudy visually). Note: the complex was stabilized at room temperature for 6 hours.

The complex was added into the cells and mixed, and then detected with a QuantStudio™ 5 Real-Time PCR System, 96-well after 72 h.

3. Detection of the mRNA changes of the target gene by qPCR

1) experimental method

At 72 h after transfection, the cells were subjected to RNA extraction with a SteadyPure Universal RNA Extraction Kit AG21017 kit, and the mRNA concentration was detected with an ultramicro spectrophotometer. The mRNA product was reverse transcribed by using an Evo M-MLV Mix Kit with gDNA Clean for qPCR AG11728 reverse transcription kit, and the reverse transcribed product was detected by using a SYBR Green Premix Pro Taq HS qPCR Kit (Low Rox Plus) AG11720 qPCR kit.

Primers used in the qPCR were as follows:

detection of PTBP1: ATTGTCCCAGATATAGCCGTTG (SEQ ID NO: 54)
GCTGTCATTTCCGTTTGCTG (SEQ ID NO: 55)
detection of AQp1: GCTCTTCTGGAGGGCAGTGG (SEQ ID NO: 56)
CAGTGTGACAGCCGGGTTGAG (SEQ ID NO: 57)

detection of internal reference GAPDH: CCATGGGGAAGGTGAAGGTC (SEQ ID NO: 58)
GAAGGGGTCATTGATGGCAAC (SEQ ID NO: 59)

A reaction system was configured according to the instructions of the SYBR Green Premix Pro Taq HS qPCR Kit (Low Rox Plus) AG11720, and detected by using a QuantStudio™ 5 Real-Time PCR System, 96-well.

2) Calculation method

In this experiment, the change of the target RNA was calculated by using a relative quantitative method, namely a 2-ΔΔCt method. The calculation method of it was as follows:

$$\Delta Ct = Ct\,(AQp1) - Ct\,(GAPDH) \text{ or } Ct\,(PTBP1) - Ct\,(GAPDH);$$

$$\Delta\Delta Ct = \Delta Ct \text{ (a sample to be verified, such as the Cas13m.2 group)} - \Delta Ct \text{ (a negative control group)};$$

$$2 - \Delta\Delta Ct = 2^{\wedge}(-\Delta\Delta Ct).$$

The experiment of this example was conducted in triplicate, and the relative mRNA expression levels of AQp1 and PTBP 1 calculated according to the aforementioned calculation manner, were as shown in Table 6 below and Fig. 8, and the result data was the average of three tests:

Table 6. Relative expression levels of target mRNA as calculated by 2-ΔΔCt method

| Groups | AQp1 mRNA level | PTBP1 mRNA level |
|---|---|---|
| Negative Control | 1.00 | 1.00 |
| CasRx | 0.05 | 0.60 |
| Cas13m.2 | 0.03 | 0.49 |
| Cas13m.3 | 0.02 | 0.46 |
| Cas13m.5 | 0.27 | 0.70 |
| CasRfg.2 | 0.36 | 0.78 |

The qPCR results showed that all of Cas13m.2, Cas13m.3, Cas13m.5 and CasRfg.2 had the effect of down-regulating the expression of AQp1 and PTBP1. Cas13m.2 and Cas13m.3 down-regulated the expression of the genes AQp1 and PTBP1 with better effects than that of CasRx, and had good editing activities. Cas13m.5 and CasRfg.2 also had significant editing activities.

**Example 6: Connection order of DR sequence and guide sequence**

The influence of the connection order of the DR sequence and the guide sequence in a gRNA molecule on the editing efficiency was verified.

1. Construction of an editing vector targeting the endogenous gene AQp1

The endogenous site selected in this experiment was AQp1, and AQp1 was verified by using the 293T cell line with high expression of AQp1 of the previous example.
The guide sequence of an AQp1-targeting gRNA was GAAGACAAAGAGGGUCGUGG

(SEQ ID NO: 42)

The verification vectors as used were as follows

| Serial Number | gRNA structure (5'-3') |
|---|---|
| Cas13m.2 | Guide sequence-direct repeat sequence |
| Cas13m.3 | Guide sequence-direct repeat sequence |
| Cas13m.5 | Guide sequence-direct repeat sequence |
| CasRfg.2 | Direct repeat sequence-guide sequence |
| Cas13m.2-r | Direct repeat sequence-guide sequence |
| Cas13m.3-r | Direct repeat sequence-guide sequence |
| Cas13m.5-r | Direct repeat sequence-guide sequence |
| CasRfg.2-r | Guide sequence-direct repeat sequence |

The verification vectors targeting the endogenous gene AQp1 mRNA of Cas13m.2, Cas13m.3, Cas13m.5 and CasRfg.2 had been constructed in the experimental example 5, and the verification vectors targeting the endogenous gene AQp1 mRNA of Cas13m.2-r, Cas13m.3-r, Cas13m.5-r, and CasRfg.2-r with adjusted gRNA structures (the positions of the guide sequence and the direct repeat sequence were reversed) (other sequences except the gRNA coding sequence were the same as those of the verification vectors of Cas13m.2, Cas13m.3, Cas13m.5 and CasRfg.2) were synthesized in a reagent company.

2. Transfection of 293T cells and 293T-AQp1 cells with the vector to be verified

293T-AQp1 cells were transfected with the verification vectors targeting the endogenous gene AQp1 mRNA of Cas13m.2, Cas13m.3, Cas13m.5, CasRfg.2, Cas13m.2-r, Cas13m.3-r, Cas13m.5-r, and CasRfg.2-r, and a control plasmid (which was the same as the verification vector plasmid targeting the endogenous gene AQp1 mRNA of CasRx in the aforementioned Example 5) at 800 ng in a 24-well plate. The negative control group was transfected with the CasRx-BpiI plasmid in the aforementioned Example 5.
The transfection method was as follows:

1. The cells were digested by trypsin (0.25% of Trypsin, EDTA, Thermo, 11058021), counted, and plated into a 24-well plates at $2 \times 10^5$ cells according to 500 μL per well.
2. For each transfected sample, the complex was prepared according to the following steps:

   a. each well of the 24-well plate into which the cells were added, were added with 50 μL of serum-free Opti-MEM I (Thermo, 25200056) reduced serum medium for dilution of the aforementioned plasmid DNA, and mixed gently;
   b. it was gently mixed with Lipofectamine 2000 (Thermo, 11668019) before use, and then 1.8 μL of the Lipofectamine 2000 was diluted in each well, i.e., in 50 μL of the Opti-MEM I medium. It was incubated at room temperature for 5 minutes. Note: it was continued to perform step c within 25 minutes;
   c. after incubation for 5 minutes, the diluted DNA was combined with the diluted Lipofectamine 2000. They were gently mixed and incubated at room temperature for 20 minutes (the solution might be cloudy visually). Note: the complex was stabilized at room temperature for 6 hours.

The complex was added into the cells and mixed, and then detected with a QuantStudio™ 5 Real-Time PCR System, 96-well after 72 h.

3. Detection of the RNA changes of the target gene by qPCR

At 72 h after transfection, the cells were subjected to RNA extraction with a SteadyPure Universal RNA Extraction Kit AG21017 kit, and the RNA concentration was detected with an ultramicro spectrophotometer. The RNA product was reverse transcribed by using an Evo M-MLV Mix Kit with gDNA Clean for qPCR AG11728 reverse transcription kit, and the reverse transcribed product was detected by using a SYBR Green Premix Pro Taq HS qPCR Kit (Low Rox Plus) AG11720 qPCR kit.

The primers used in the qPCR included the primer pair for detecting AQp1 as shown in SEQ ID NOs: 56-57 and the primer pair for detecting the internal reference GAPDH as shown in SEQ ID Nos: 58-59.

A reaction system was configured according to the instructions of the SYBR Green Premix Pro Taq HS qPCR Kit (Low Rox Plus) AG11720, and detected by using a QuantStudio™ 5 Real-Time PCR System, 96-well.

The qPCR results were as follows:

In this experiment, the relative expression level of the target RNA was calculated by using a relative quantitative method, namely a 2-ΔΔCt method. The calculation method of it was as follows:

$$\Delta Ct = Ct\,(AQp1) - Ct\,(GAPDH);$$

$$\Delta\Delta Ct = \Delta Ct \text{ (a sample to be verified, such as the Cas13m.2 group)} - \Delta Ct \text{ (a negative control group);}$$

$$2 - \Delta\Delta Ct = 2^{\wedge}(-\Delta\Delta Ct).$$

The amount of AQp1 mRNA calculated according to the aforementioned calculation manner was as shown in Table 7 below:

Table 7. Relative expression levels of target RNA
as calculated by 2-ΔΔCt method

| Groups | AQp1 mRNA level |
|---|---|
| Negative Control | 1.00 |
| Cas13m.2 | 0.04 |
| Cas13m.3 | 0.04 |
| Cas13m.5 | 0.36 |
| CasRfg.2 | 0.30 |
| Cas13m.2-r | 0.83 |
| Cas13m.3-r | 0.78 |
| Cas13m.5-r | 0.74 |
| CasRfg.2-r | 0.67 |

The qPCR results showed that the editing activities of Cas13m.2-r, Cas13m.3-r, Cas13m.5-r and CasRfg.2-r were decreased obviously after changing the relative positions of the direct repeat sequence and the guide sequence.

**Example 7: The editing activity of Cas13m.6 on an exogenous gene in a cell and the activity comparison between the CasI3m and published proteins**

Unless otherwise specified, the experiment was conducted in this example by using the same method as that of Example 4.

1. Synthesizing of an EGFP-targeting vector to be verified

An EGFP-targeting validation vector Cas13m.6 was obtained by preparation, with the full-length sequence as shown in SEQ ID NO: 105 (7,690 bp).

After looking up in NCBI, it was searched that NCBI disclosed two Cas13 proteins, namely a C13-38 protein (GenBank: MBQ9236733.1) and a C13-40 protein (NCBI Reference Sequence: WP_025000926.1), and corresponding DR sequences thereof.

A comparison of gene editing activity was conducted by the inventor among Cas13m, C13-38 and C13-40.
The sequence of C13-38 was:

MEKHHSQPRKAQFPFSISEKSVMGGYFNIARLNFYKTIVTIFAQVGVKGEYPEDKIDRVLDA

LYKNIAGKDNELSKEQAQWKRLKQLKGEQITKLQRLLFNHFPVLGPIMASEASYKIYKSEL

NAKEAEDAVQNDKEELKKIKKSNVINNEQLMRGVGIDDCLNVLATMAACLTDCRNYYSH

YIPYNSIEDQKKQYKRQAQIARWLDKVIVASRRIDKQRNSLTTNEMEFLTGIDHYFQQDKK

DDTGKLIRDEKGRTLKEFVEYPDYYFRIKGERQLVDIAGKTLNEEQAQNALTDFGIVFFCTL

FLQKTYAKMMQEELKLYENGPYRGDVKGKENDDAKKNTILREMLSIYRIRVPRGKRLDSK

DDATTLSMDMLNELRKCPMPLYDVLGKDGQRFFEDEVQHPNEQTPEKVKRLRATDRFPHL

ALRYIDLHDKTFTRIRFQVQLGNFRFKFYNKKTIDGAEEVRSIQKEINGYGRLQEIEAKRLET

YAPLFQKSELVSTKLEHEDLNLDLVQFTEDHADSKPYITNHRATYNIHNNRIGMYWEASQN

VKEYKVFSSDGMYLPTLNTIDGKAPISMPAPKASLSIYELPAMLFYQYLLDNNNVKKNEYD

APQDILINKHDALVKFFEAVRGGELIPALSKDELSRKLESEYDLKISEVPNKLVDYLIGKEDN

GKRLYDYATHEVLLRLRRSLRRFEHFEEDRKMIGSKDNKYGKKGFVDVRHGRLAQYLAES

IMDWRKPLNGEKDKLTGLNYSKMQAALATFGGKTTFDKLNTLFKEAGLYDNRPGSHPFLQ

STMQKAPQNIEMLYLAYLEAETDKLKKFVVIKNLNNLSEKELKEYKDLVTFTVKEKRTYSD

GRTKMVMVDKVAVNIIGNTNFANLPFIHHQRARFAQRNAEYYKSLAGRYLSVDGKSATIQL

PDGIFTKHILKLLKEKYATHEALQLHLTDDDMNHNAAYLISSFFETVLNDCSQPYYRTFHYE

NNEKKTSKFAHIYDLFNILNNVKEANAYKPYPMTTDDINSRLTKKATNRDGLFVIRKDDNG

EDYLVKQITLDIENHLKKMEDAVEAKIKFKNLYGYNADKARKNGAEEREKMLRKLTHCIS

DVKNNERAIRRYKTQDMVLFLLAKSTLSTILAQQNGVASEELFRLKNVCNNNFLSQTVRFE

FPIKVNEMTIKVVQENMALKNYGEFYRFINDDRLMSLLTQLKDVTEISYADLTGELATYDLR

RSQVFRLMQELEKIAFEQHTKELTNIDNSMFFKDGDMNNVPRRNNFKALINLFDSIDSHQLT

KDDCERLVEIRNAFCHNTYRINIDDLQEKLPTIAIQIVGKIENLLKGADMKK (SEQ ID NO: 63)

The DR sequence corresponding to C13-38 was:

5'-GUUUUCAUACCUAUCCAAACGAUAGGCUUCUAAAAC-3' (SEQ ID NO: 64)

The sequence of C13-40 was:

MEDDKKTTGSISYELKDKHFWAAFLNLARHNVYITINHINKLLEIREIDNDEKVLDIKTLWQ

KGNKDLNQKARLRELMTKHFPFLETAIYTKNKEDKKEVKQEKQAEAQSLESLKDCLFLFL

DKLQEARNYYSHYKYSEFSKEPEFEEGLLEKMYNIFGNNIQLVINDYQHNKDINPDEDFKH

LDRKGQFKYSFADNEGNITESGLLFFVSLFLEKKDAIWMQQKLNGFKDNLENKKKMTHEV

FCRSRILMPKLRLESTQTQDWILLDMLNELIRCPKSLYERLQGDDREKFKVPFDPADEDYNA

EQEPFKNTLIRHQDRFPYFVLRYFDYNEIFKNLRFQIDLGTYHFSIYKKLIGGQKEDRHLTHK

LYGFERIQEFAKQNRPDEWKAIVKDLDTYETSNKRYISETTPHYHLENQKIGIRFRNGNKEI

WPSLKTNDENNEKSKYKLDKQYQAEAFLSVHELLPMMFYYLLLKKEKPNNDEINASIVEG

FIKREIRNIFKLYDAFANGEINNIDDLEKYCADKGIPKRHLPKQMVAILYDEHKDMVKEAKR

KQKEMVKDTKKLLATLEKQTQKEKEDDGRNVKLLKSGEIARWLVNDMMRFQPVQKDNE

GKPLNNSKANSTEYQMLQRSLALYNNEEKPTRYFRQVNLIESNNPHPFLKWTKWEECNNIL

TFYYSYLTKKIEFLNKLKPEDWKKNQYFLKLKEPKTNRETLVQGWKNGFNLPRGIFTEPIRE

WFKRHQNNSKEYEKVEALDRVGLVTKVIPLFFKEEYFKDKEENFKEDTQKEINDCVQPFYN

FPYNVGNIHKPKEKDFLHREERIELWDKKKDKFKGYKEKIKSKKLTEKDKEEFRSYLEFQS

WNKFERELRLVRNQDIVTWLLCKELIDKLKIDELNIEELKKLRLNNIDTDTAKKEKNNILNR

VMPMELPVTVYEIDDSHKIVKDKPLHTIYIKEAETKLLKQGNFKALVKDRRLNGLFSFVKT

NSEAESKRNPISKLRVEYELGEYQEARIEIIQDMLALEEKLINKYKDLPTNKFSEMLNSWLE

GKDEADKARFQNDVDFLIAVRNAFSHNQYPMHNKIEFANIKPFSLYTANNSEEKGLGIANQ

LKDKTKETTDKIKKIEKPIETKE (SEQ ID NO: 65)

The DR sequence corresponding to C13-40 was: 5'-GUUGUUUUUACCUUUCAAACAGAAGGCAGAUACAACA-3' (SEQ ID NO: 66)

C13-38 and C13-40 verification vectors were constructed according to the aforementioned method, and their nucleotide backbone sequences were the same as that of the Cas13m.3 verification vector of Example 4, except that the coding sequence of Cas13 protein and the coding sequence of DR sequence had been replaced accordingly.

The aforementioned Cas13m.6, C13-38 and C13-40 verification vectors all contained the coding sequence of Cas13 protein, which could express a Cas13 protein linked with NLS, and could also express the EGFP-targeting gRNA (the guide sequences of the gRNAs corresponding to C13-38 and C13-40 were both located at the 5' terminal of the DR sequence). The guide sequence of the gRNA corresponded to the aforementioned spacer sequence (SEQ ID NO: 30). All the aforementioned vectors were synthesized by a reagent company by a conventional method.

2. Transfection of a 293T cell with the vector to be verified

The 293T cells were transfected with a plasmid expressing the exogenous gene EGFP and the Cas13 verification vector plasmids (the Cas13m.6 verification vector, the C13-38 verification vector, the C13-40 verification vector, or other Cas13m verification vectors obtained by preparation in Example 4) at the ratio of 300 ng:600 ng.

The description of cells and plasmids as used was as shown in Table 8 below :

Table 8: Experimental Grouping

| Name | Transfected with the EGFP vector | Transfected with the EGFP-targeting Cas13 verification vector | Comments |
|---|---|---|---|
| 293T | | | Cell control |
| EGFP | * | | Control transfected with EGFP only |
| Cas 13m.2 | * | * | Verification vector |
| Cas 13m.3 | * | * | Verification vector |
| Cas 13m.5 | * | * | Verification vector |
| Cas13m.6 | * | * | Verification vector |
| C13-38 | * | * | Verification vector |
| C13-40 | * | * | Verification vector |
| Note: * represented containing related items, and blank represented there was no related items | | | |

3. Detection of the down-regulation the effect of Cas13 protein on EGFP expression by flow cytometer

It was given that the GFP fluorescence of the EGFP group was a, and the GFP fluorescence of other groups was x.

$$\text{downregulation amplitude} \% = (a - x) \div a \times 100\%$$

The experiment of this example was conducted in triplicate. The result data was the average of three tests, and the result was as shown in Table 9 below.

Table 9. Results of GFP fluorescence as detected by a flow cytometer

| Groups | Downregulation amplitude (%) |
|---|---|
| EGFP | 0.00 |
| Cas13m.2 | 61.8 * # |
| Cas 13m.3 | 67.2 * # |
| Cas13m.5 | 42.3 * # |
| Cas13m.6 | 65.5 * # |
| C13-38 | 4.2 |
| C13-40 | 2.9 |
| Note: * indicated a significant difference compared with the C13-38 group (p < 0.01), # indicated a significant difference compared with the C13-40 group (p < 0.01). | |

The results showed that the downregulation amplitude of EGFP of the Cas13m.6 group was 65.5%. It indicated that the Cas13m.6 protein could significantly down-regulate the expression of EGFP, which proved that it could effectively reduce the mRNA level and exert its editing activity in eukaryotic cells under the guidance of the gRNA.
Also, the results showed that the editing activities of Cas13m.2, Cas13m.3, Cas13m.5 and Cas13m.6 were significantly higher than those of C13-38 and C13-40.

**Example 8: Validation of endogenous gene editing efficiency of Cas13m.6**

Unless otherwise specified, this example used the same method as that of Example 5.

1. Construction of an editing vector targeting endogenous genes AQp1 and PTBP 1

An expression vector Cas13m.6-BsaI was constructed, and its sequence was as shown in SEQ ID NO: 77.
The endogenous sites selected in the experiment were AQp1 and PTBP1, wherein AQp1 was verified by using the aforementioned 293T cell line (293T-AQp1 cells) with high expression of AQp1, and PTBP1 was verified by using a 293T cell line.
The guide sequence of the AQp1-targeting gRNA was selected as: [0409] AGGGCAGAACCGATGCTGATGAAGAC (SEQ ID NO: 68)
The guide sequence targeting PTBP1 was selected as: GUGGUUGGAGAACUGGAUGUAGAUGGGCUG (SEQ ID NO: 43)
A target site-targeted fragment was obtained by using a primer annealing manner, and the primers of it were as follows:

PTBP1-targeting:

    Cas13m.6 group: caccGTGGTTGGAGAACTGGATGTAGATGGGCTG (SEQ ID NO: 44)
    caacCAGCCCATCTACATCCAGTTCTCCAACCAC (SEQ ID NO: 45)
    CasRx group: SEQ ID NO: 46 and SEQ ID NO: 48

AQp1-targeting:

Cas13m.6 group: CACCGagggcagaaccgatgctgatgaagac (SEQ ID NO: 69)
CAACgtcttcatcagcatcggttctgccctc (SEQ ID NO: 70)
CasRx group: aaacagggcagaaccgatgctgatgaagac (SEQ ID NO: 71)
CTTGgtcttcatcagcatcggttctgccct (SEQ ID NO: 72)

A *Bsa*I-digested Cas13m.6-BsaI vector was linked with the annealing products by T4. The CasRx-BpiI plasmid was digested with BpiI, and linked with the annealing products by T4. The verification vectors targeting the endogenous genes AQp1 and PTBP1 were obtained.

2. Transfection of 293T cells and 293T-AQp1 cells with the vector to be verified

The verification vector was transfected into the 293T cells and the 293T-AQp1 cells, respectively. The negative control group was transfected with the CasRx-BpiI plasmid.

3. Detection of the mRNA changes of the target gene by qPCR

Detection was conducted by qPCR, and the mRNA levels of AQp1 and PTBP1 were calculated by the 2-ΔΔCt method. The experiment of this example was conducted in triplicate, and the result data was the average of three tests. The result was shown in Table 10 below.

Table 10. Relative expression level of target mRNA

| Groups | AQp1 mRNA level | PTBP1 mRNA level |
|---|---|---|
| Negative Control | 1.00 | 1.00 |
| CasRx | 0.03 | 0.59 |
| Cas13m.6 | 0.01 | 0.51 |

The qPCR results showed that Cas13m.6 could significantly down-regulate the expression of AQp1 and PTBP1, of which the effect was slightly better than that of CasRx.

**Example 9: Identification of key amino acid residues of Cas13m protein**

In an experiment of knocking down the expression of AQp1 and PTBP1, Cas13m.2, Cas13m.3 and Cas13m.6 showed the highest level of knocking down, followed by Cas13m.5. In the knock-down experiment of the exogenous EGFP, Cas13m.2 and Cas13m.3 showed a higher level of knock-down than those of Cas13m.1, Cas13m.4 and Cas 13m.5.

In the literature (Slaymaker, Ian M., et al. "High-resolution structure of Cas13b and biochemical characterization of RNA targeting and cleavage." Cell reports 26.13 (2019): 3741-3751.), the crystal structure of PbuCas13b was reported, and in this paper, the amino acid residues in the PbuCas13b protein interacting with a crRNA and the catalytic residues of the HEPN domain of the PbuCas13b protein were shown.

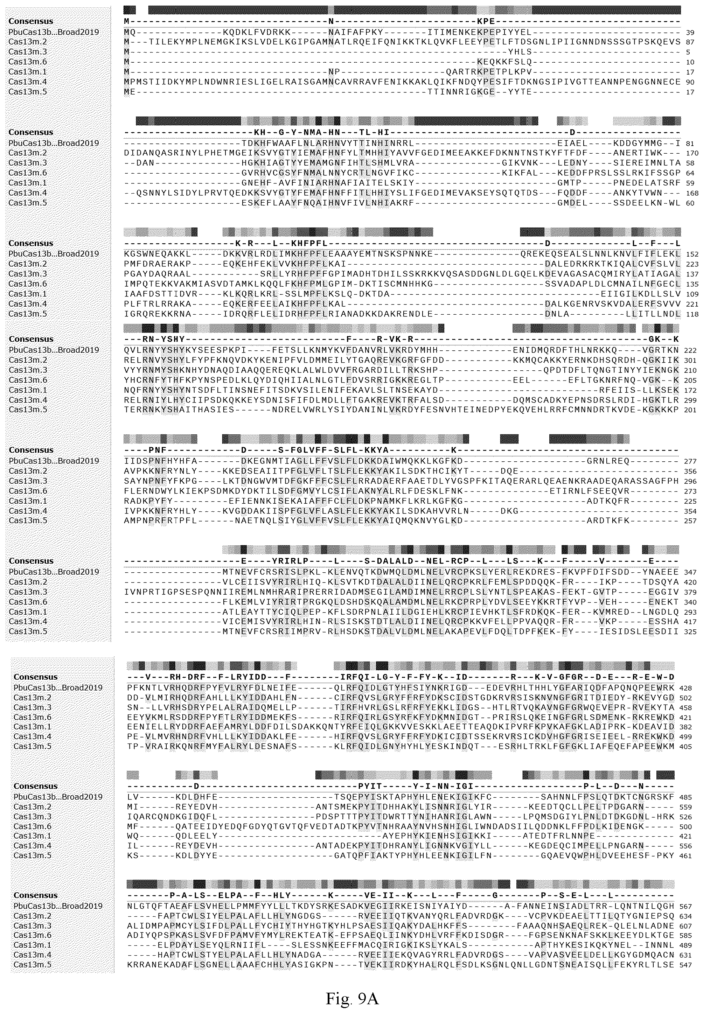

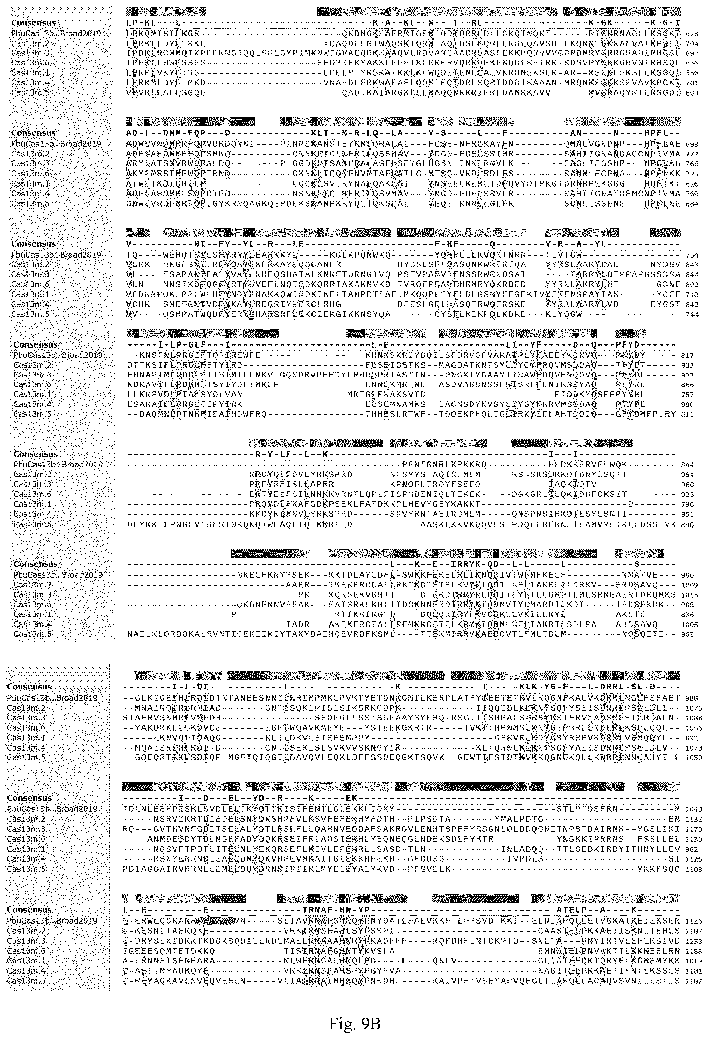

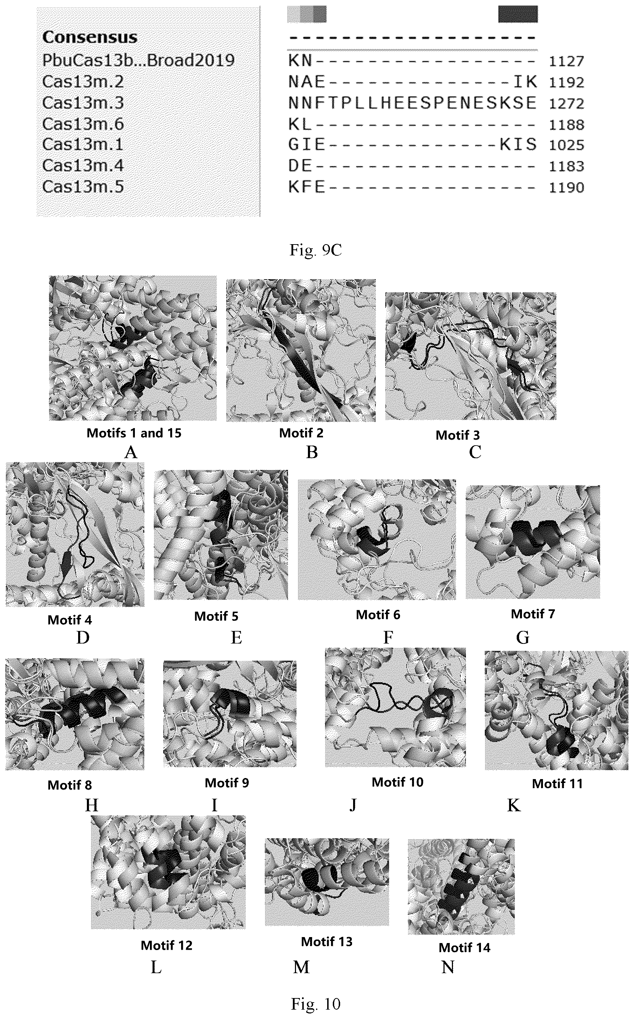

Considering that the Cas13m protein was closer to Cas13b on the evolutionary tree, the inventor had carried out multi-sequence alignment between the Cas13m protein in the file of the present application and PbuCas13b (online MAFFT v7.504, E-INS-i algorithm, and the others were default parameters). The results were as shown in Fig. 9A- Fig. 9C. The conserved motifs (motifs 1-15) of high-activity Cas13m proteins (Cas13m.2, Cas13m.3, Cas13m.6) were identified at positions corresponding to the aforementioned key residues (amino acid residues interacting with the crRNA and the catalytic residues of the HEPN domain) of the PbuCas13b protein, and they were written in a commonly used Prosite form, as shown in Table 11 below. The motifs 1-15 appeared more frequently in Cas13m.2, Cas13m.3 and Cas13m.6, and the motifs 16-30 were further definitions of the motifs 1-15.

Table 11. Results of multi-sequence alignment of conserved motifs

| The amino acid residues corresponding to PbuCas13b | Consensus motif of Cas13m.2, Cas13m.3 and Cas13m.6 | Consensus motif of Cas13m.2, Cas13m.3 and Cas13m.6 (further defined) |
|---|---|---|
| R156, H161 | Motif 1: L-x(3)-R-N-x-Y-[ST]-H | Motif 16: L-[RVY]-[EYH]-[LYC]-R-N-[VFM]-Y-[ST]-H |
| Thr405, His407 | Motif 2: R-x(3)-K-x-[VI]-N-G-F-G-R | Motif 17: R-[ST]-[IVL]-[SQ]-K-[NAE]-[VI]-N-G-F-G-R |
| His452, Asn455, Lys457 | Motif 3: P-Y-[IV]-T-x(5)-Y-x-[IV]-x(2)-N-x-I-G-L | Motif 18: P-Y-[IV]-T-[DN]-[HW]-[HR]-[AT]-[KAT]-Y-[LN]-[IV]-[HS]-[NSA]-N-[RH]-I-G-L |
| Asn480, Lys484, Asn486 | Motif 4: P-x-L-x(2)-D-x(3)-[NK] | Motif 19: P-[END]-L-[TKD]-[PIT]-D-[GKE]-[AGN]-[RDG]-[NK] |
| His500 | Motif 5: P-x-[AC]-x-L-S-x(2)-[ED]-[LF]-PA-x(2)-F | Motif 20: P-[TMK]-[AC]-[WYS]-L-S-[IV]-[FY]-[ED]-[LF]-P-A-[LM]-[ALV]-F-[LY]-[LCM]-[HY]-[LI]-[YR] |
| Gly566, His567 | Motif 6: [LI]-P-x-K-L | Motif 21: [SNG]-[QE]-[LI]-P-[RED]-K-L |
| Lys590 | Motif 7: | Motif 22: [KT]-[WHK]-[AL]-[AQE]- |
| | [KT]-x-[AL]-x(2)-[KVE]-[EL] | [SQE]-[KVE]-[I]L] |
| Arg638 | Motif 8: A-[DRK]-x-L-x(2)-[DS]-[MI]-[MV]-x-[FW]-Q-P | Motif 23: A-[DRK]-[FY]-L-[AM]-[HTR]-[DS]-[MI]-[MV]-[FRE]-[FW]-Q-P |
| Asn652, Asn653, Lys655, Ala656, Ser658 | Motif 9: K-L-T-x(2)-N | Motif 24: [CG]-[NGK]-[ND]-K-L-T-[GS]-[LAQ]-N |
| Lys741 | Motif 10: F-x-[HR]-[AF]-x(5)-[QR] | Motif 25: F-[ALV]-[HR]-[AF]-[NS]-[QSR]-[NSM]-[KR]-[WY]-[QR] |
| Asn756, Ser757, Arg762 | Motif 11: I-x-L-P-x-G-[LM]-F-x(3)-I | Motif 26: [KA]-[SPV]-I-[ELM]-L-P-[RD]-G-[LM]-F-[ET]-[ST]-[YH]-I |
| Arg791, Val795, Ala796 | Motif 12: [LI]-I-x(2)-[YWF]-F | Motif 27: [LI]-I-x(2)-[YWF]-F-x(5)-[DQ]-x(2)-Q-[PT]-F-Y-[DR] |
| Trp842, Lys846 | Motif 13: I-x(3)-I | Motif 28: I-[RAL]-[KQ]-[KD]-I |
| Lys870, Lys871, Glu873, Arg874, Arg877 | Motif 14: [DN]-[TN]-E-x(2)-[IL]-[KR]-[VR]-Y-[KR]-x-Q-D | Motif 29: [DN]-[TN]-E-[KTR]-[ED]-[IL]-[KR]-[VR]-Y-[KR]-[ILT]-Q-D |
| R1068, H1073 | Motif 15: R-N-[SA]-[FA]-x-H-x(2)-Y | Motif 30: R-N-[SA]-[FA]-[AG]-H-[NL]-[SRT]-Y-[PK] |
| Note: A single letter code indicated a highly conserved amino acid residue, x indicated any amino acids, and [] indicated that this position is an optional amino acid code within []. | | |

The three-dimensional structures of the Cas13m protein and PbuCas13b were predicted by the program AlphaFold v2.0, and then the proteins were superimposed by PyMOL V2.5.1.

The result showed that the three-dimensional structure of the PbuCas13b protein predicted by AlphaFold was very similar to that of a protein in the crystal structure of a complex of PbuCas13b and the gRNA as reported in a literature (NDB: 6DTD, https://www.rcsb.org/structure/6dtd) (RMSD = 2.122), which indicated that the conformational difference of PbuCas13b before and after binding to the gRNA was not particularly big, that was, the comparison of the three-dimensional structure between Cas13m and the PbuCas13b protein was meaningful, and it was not necessary to strictly compare the Cas13-gRNA complexes of both of them.

The three-dimensional spatial locations of the motifs 1-15 of the Cas13m protein in the protein were very similar to those of the corresponding sequences of PbuCas13b. Taking Cas13m.6 as an example, it was superimposed with PbuCas13b. A-N in Fig. 10 respectively showed the overlapping of the motifs 1-15 of Cas13m.6 and the corresponding sequences of PbuCas13b.

It could be predicted that motifs 1-13 could enable the Cas13m.2, Cas13m.3 and Cas13m.6 proteins to interact with their respective DR sequences, and the motifs 14 and 15 were catalytic activity centers. It could be understood by those skilled in the art that, the homologous protein or mutant of Cas13m.2, Cas13m.3 or Cas13m.6 was also expected to show the target nucleic acid binding activity or endonuclease activity when it contains the motifs 1-15, especially when amino acid residues other than the motifs 1-15 were subjected to conservative amino acid replacement on the basis of a wild-type sequence. The aforementioned homologous protein or mutant might have a sequence identity $\geq$ 50% with the Cas13m.2, Cas13m.3 or Cas13m.6 protein (e.g., a sequence identity $\geq$ 60%, $\geq$ 70%, $\geq$ 80%, $\geq$ 85%, $\geq$ 90%, $\geq$ 95%, $\geq$ 96%, $\geq$ 97%, $\geq$ 98%, $\geq$ 99% or $\geq$ 99.5%). The source of the aforementioned homologous protein could also be of the same kingdom, phylum, class, order, family, genus or species as the source of the Cas13m.2, Cas13m.3 or Cas13m.6 protein.

Therefore, this paper provided a Cas13 protein having this consensus motif (a conjugate containing it, a nucleic acid encoding these proteins or conjugates, a vector containing these nucleic acids, and a method of using these proteins/nucleic acids).

**Example 10: Off-target Test**

1. Construction of control vector

In this experiment, the PTBP1 gene was selected as the target gene for off-target verification.

The controls shRNA1 and shRNA2 respectively intercepted 21 nt from the head and tail of the target sequence used by Cas13 as the targets, specifically as follows:

ShRNA1 target site: GCCCATCTACATCCAGTTCTC (SEQ ID NO: 73)

ShRNA2 target site: CAGCCCATCTACATCCAGTTC (SEQ ID NO: 74)

Primers used for constructing the control vector were as shown in the table 12 below:

Table 12. List of Primer sequence

| Primer name | Sequence | SEQ ID NO: |
|---|---|---|
| PTBP 1-g3-shRNA-1F | caccGCCCATCTACATCCAGTTCTCCTCGAGGAGAACTGGATGTAGATGGGCTTTTTT | 75 |
| PTBP1-g3-shRNA-1R | ggccAAAAAAGCCCATCTACATCCAGTTCTCCTCGAGGAGAACTGGATGTAGATGGGC | 76 |
| PTBP 1-g3-shRNA-2F | caccCAGCCCATCTACATCCAGTTCCTCGAGGAACTGGATGTAGATGGGCTGTTTTTT | 77 |
| PTBP 1-g3-shRNA-2R | ggccAAAAAACAGCCCATCTACATCCAGTTCCTCGAGGAACTGGATGTAGATGGGCTG | 78 |

The aforementioned primers were respectively annealed according to shRNA1: PTBP1-g3-shRNA-1F/PTBP1-g3-shRNA-1R and shRNA2: PTBP1-g3-shRNA-2F/PTBP1-g3-shRNA-2R, so as to obtain annealed products.

The vector pAAV-CMV-EGFP was subjected to double enzyme digestion via BsaI and NotI to obtain a linearized backbone. The backbone was connected with the annealing products of shRNA1 and shRNA2, respectively, and then transformed into *Escherichia coli* to obtain the control vectors shRNA1 and shRNA2 (which could express shRNA1 and shRNA2 respectively under the drive by an U6 promoter).

The vector CasRx-blank was constructed by a conventional method. The CasRx-blank was obtained by replacing the coding sequence GGGTCTTCGAGAAGACCT (SEQ ID NO: 103) of the guide sequence of the gRNA with GATCAACATTAAATGTGAGCGAGT (SEQ ID NO: 104) (the coded gRNA could target the LacZ of *E. coli.),* on the basis of the aforementioned CasRx-BpiI plasmid. Moreover, the PTBP1-targeting plasmids of Cas13m.2, Cas13m.3, Cas13m.5, CasRfg.2 and CasRx (named Cas13m.2-PTBP1, Cas13m.3-PTBP1, Cas13m.5-PTBP1, CasRfg.2-PTBP1, and CasRx-PTBP1 respectively) constructed in Example 5 were also used.

The sequence of the backbone vector pAAV-CMV-EGFP was as shown in SEQ ID NO: 79.

2. Transfection of a 293T cell with the vector to be verified

The 293T cells were transfected with the plasmid to be verified at 500 ng in a 24-well plate.

The transfection method was as follows:

1). The 293T cells were digested by trypsin (0.25% of Trypsin, EDTA, Thermo, 11058021), counted, and plated into a 24-well plates at $2 \times 10^5$ cells according to 500 $\mu$L per well.

2). For each transfected sample, the complex was prepared according to the following steps:

a. each well of the 24-well plate into which the cells were added, were added with 50 $\mu$L of serum-free Opti-MEM I (Thermo, 25200056) reduced serum medium for dilution of the aforementioned plasmid DNA, and mixed gently;

b. it was gently mixed with Lipofectamine 2000 (Thermo, 11668019) before use, and then 1.8 $\mu$L of the Lipofectamine 2000 was diluted in each well, i.e., in 50 $\mu$L of the Opti-MEM I medium. It was incubated at room temperature for 5 minutes. Note: it was continued to perform step c within 25 minutes;

c. after incubation for 5 minutes, the diluted DNA was combined with the diluted Lipofectamine 2000. They were gently mixed and incubated at room temperature for 20 minutes (the solution might be cloudy visually). Note: the complex was stabilized at room temperature for 6 hours.

At 72 h after transfection, the cells were subjected to RNA extraction with a SteadyPure Universal RNA Extraction Kit AG21017 kit, and the RNA concentration was detected with an ultramicro spectrophotometer. The extracted RNA was sent to a reagent company for RNA sequencing.

3. Off-target analysis

Samples were sequenced by PE150bp RNA-Seq, and several fastq files obtained by sequencing were aligned with the reference genome of the target species by HISAT2 or STAR software, respectively, to obtain several BAM files after the alignment. The expression levels of the obtained transcripts and various genes were detected by kallisto, RSEM or HTSeq.

The variation analysis of expression levels among groups was conducted by using DESeq2, limma-voom and edger, and a gene satisfying p.adj < 0.05, |log2FoldChange| > 0.75 and basemean > 2.5 was taken as the differential expression gene (DEG). The table below listed the number of DEGs in each experimental group compared with that of the CasRx-blank group:

Table 13. Number of differentially expressed genes

| | up (number of up-regulated DEG) | down (number of down-regulated DEG) | up + down |
|---|---|---|---|
| CasRx_vs_CasRx-blank | 74 | 24 | 98 |
| Cas13m.5_vs_CasRx-blank | 8 | 10 | 18 |
| CasRfg.2_vs_CasRx-blank | 23 | 17 | 40 |
| Cas13m.2_vs_CasRx-blank | 13 | 18 | 31 |
| Cas13m.3_vs_CasRx-blank | 4 | 5 | 9 |

(continued)

|  | up (number of up-regulated DEG) | down (number of down-regulated DEG) | up + down |
| --- | --- | --- | --- |
| shRNA1_vs_CasRx-blank | 12 | 37 | 49 |
| shRNA2_vs_CasRx-blank | 22 | 31 | 53 |

It could be seen from the data in the table that, compared with CasRx, shRNA1 and shRNA2, the number of potential off-target genes in Cas13m.2, Cas13m.3 and Cas13m.5 was less, which had less influence on the gene expression profile of the cells. Therefore, this characteristic of Cas 13m.2, Cas13m.3 and Cas13m.5 would make them have better safety and lower toxicity when they were used in disease treatment.

**Example 11: Assay of Collateral Cleavage Effect**

1. Acquisition of gRNA

A PTBP1-targeting gRNA was transcribed by using a T7 *in vitro* transcription kit T7 High Yield RNA Transcription Kit, Vazyme, TR101-01.

The sequence of the gRNA molecular obtained by transcription *in vitro* was as follows:

Cas13m.2-PTBP1:

5'-GUGGUUGGAGAACUGGAUGUAGAUGGGCUGGUUGUAGAUGACCUCGUUU UGGAGGGGAAACACAAC-3' (SEQ ID NO: 80)

Cas13m.3-PTBP1:

5'-GUGGUUGGAGAACUGGAUGUAGAUGGGCUGGUUGUAGAAGCCGUUCAUU

CGGGACGGUAUGACAAC-3' (SEQ ID NO: 81)

Cas13m.6-PTBP1:

5'-GUGGUUGGAGAACUGGAUGUAGAUGGGCUGGUUGUAGAAGCCUAUCGUU AGGAUAGGUAUGACAAC-3' (SEQ ID NO: 82)

2. Detection of Collateral Cleavage Effect

RNaseAlert was a novel RNA substrate, one end of which was labeled with a fluorescent reporter molecule (fluorophor) and the other end was labeled with a quencher. The physical proximity of the quencher would suppress the fluorescence of the phosphor to an extremely low level. However, when the RNase was existed, the RNA substrate was cleaved, and the phosphor and the quencher were separated. When the phosphor was excited by a light at 490 nm, it would emit a green fluorescence signal at 520 nm.

When Cas13 had the collateral cleavage activity (i.e., the non-specific RNA cleaving activity activated by the target RNA), the RNaseAlert substrate would also be cleaved to emit a green fluorescent signal that could be detected.

The main experimental equipment and materials were as follows:

RNaseAlert®-1 Kit, IDT, 11-02-01-02
RNase Inhibitor, Murine, NEB, M0314L
Corning® 96-well all-black polystyrene microplate, Coming, 3915

Microplate reader, BioTek, SLXFA.

The following RNaseAlert collateral cleavage system was formulated:

| | | |
|---|---|---|
| Cas13 protein (Cas13m) | | 45 nM |
| gRNA | 22.5 nM | |
| RNase Inhibitor | | 2 $\mu$L |
| 293T cell RNA | 100 ng | |
| RNaseAlert | 10 $\mu$L | |
| 10X RNaseAlert®-1 Buffer | 10 $\mu$L | |
| RNase-free deionized water | up to 100 $\mu$l | |

Note: the RNA group of the 293T cells were extracted according to the instructions of the SteadyPure Universal RNA Extraction Kit. The Cas13m recombinant protein purified in Example 2 was used. The Cas13 protein and the gRNA were not added in the reaction systems of the RNase group (a positive control group added with the RNase) and the blank group (a blank control group).

The reaction was carried out at 37°C for 1 h, and the fluorescence at 520 nm was detected every 30 min by the microplate reader.

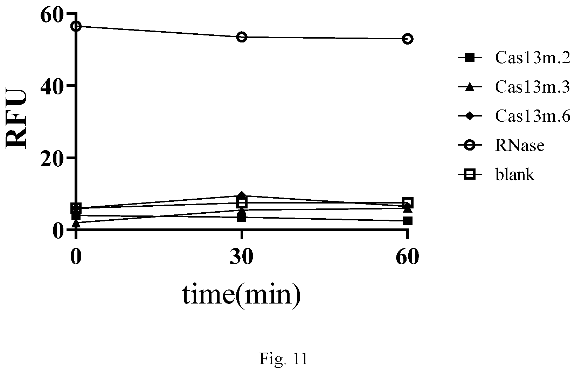

The detected effect was shown in Table 14 below and Fig. 11:

Table 14. Results of collateral cleavage tests

| RFU | 0 min | | | 30 min | | | 60 min | | |
|---|---|---|---|---|---|---|---|---|---|
| | 1 | 2 | mean | 1 | 2 | mean | 1 | 2 | mean |
| Cas 13m.2 | 3 | 5 | 4 | 3 | 4 | 3.5 | 1 | 4 | 2.5 |
| Cas13m.3 | 2 | 2 | 2 | 4 | 7 | 5.5 | 7 | 5 | 6 |
| Cas13m.6 | 7 | 5 | 6 | 8 | 11 | 9.5 | 6 | 7 | 6.5 |
| RNase | 55 | 58 | 56.5 | 52 | 55 | 53.5 | 53 | 53 | 53 |
| blank | 5 | 7 | 6 | 5 | 10 | 7.5 | 8 | 7 | 7.5 |

The relative fluorescence intensity of each of Cas13m.2, Cas13m.3 and Cas13m.6 was lower than 10, and the fluorescence intensity did not increase over time, and no collateral cleavage activity was observed.

The literature (Koonin, Eugene V, and Kira S. Makarova. "Evolutionary plasticity and functional versatility of CRISPR systems." PLoS biology 20.1 (2022): e3001481.) pointed out that Cas13, once activated by target recognition, would cleave the RNA indiscriminately and induce dormancy or death of the cell. The experimental results of this example showed that the characteristic that Cas13m.2, Cas13m.3 and Cas13m.6 had no collateral cleavage activity, would make them have better safety and lower toxicity when they were used in disease treatment.

The technical features of the aforementioned examples can be arbitrarily combined. To simplify the description, we do noy describe all possible combinations of the technical features in the aforementioned examples. However, as long as there is no contradiction in the combination of these technical features, it should be considered as the scope stated in this specification.

The examples described above are merely illustrative of several embodiments of the present invention, the description of them is more specific and detailed, but cannot be construed as limiting the scope of the present invention accordingly. It should be noted that, several variations and modifications can be made by those of ordinary skills in the art, under the premise of not departing from the concept of the present invention, and these variations and modifications all fall within the claimed scope of the present invention. Therefore, the claimed scope of the patent of the present invention shall be determined by the appended claims.

**Claims**

1. An isolated Cas13 protein, wherein the amino acid sequence of the Cas13 protein comprises a sequence having $\geq$ 50% sequence identity with the sequence as shown in any one of SEQ ID NO: 1-SEQ ID NO: 7 and SEQ ID NO: 60.

2. The Cas13 protein according to claim 1, wherein the Cas13 protein can form a complex with a gRNA.

3. The Cast3 protein according to claim 2, wherein the Cast3 protein can be guided to a target nucleic acid by the gRNA.

4. The Cas13 protein according to claim 3, wherein the Cas13 protein can be guided to a target nucleic acid by the gRNA, and targets or modifies the target nucleic acid.

5. The Cas13 protein according to claim 3, wherein the target nucleic acid is an RNA.

6. The Cas13 protein according to claim 5, wherein the target nucleic acid is a PTBP1 mRNA, AQp1 mRNA, VEGFA mRNA, VEGFR1 mRNA or VEGFR2 mRNA.

7. The Cas13 protein according to claim 1, wherein the Cas13 protein is derived from the same kingdom, phylum, class, order, family, genus or species as a protein comprising the amino acid sequence of a sequence as shown in any one of SEQ ID NO: 1-SEQ ID NO: 7 and SEQ ID NO: 60.

8. An isolated Cas13 protein, wherein the amino acid sequence of the Cas13 protein comprises the amino acid sequence as shown in the following motifs 1-15:

   motif 1: L-x(3)-R-N-x-Y-[ST]-H,
   motif 2: R-x(3)-K-x-[VI]-N-G-F-G-R,
   motif 3: P-Y-[IV]-T-x(5)-Y-x-[IV]-x(2)-N-x-I-G-L,
   motif 4: P-x-L-x(2)-D-x(3)-[NK],
   motif 5: P-x-[AC]-x-L-S-x(2)-[ED]-[LF]-P-A-x(2)-F,
   motif 6: [LI]-P-x-K-L,
   motif 7: [KT]-x-[AL]-x(2)-[KVE]-[IL],
   motif 8: A-[DRK]-x-L-x(2)-[DS]-[MI]-[MV]-x-[FW]-Q-P,
   motif 9: K-L-T-x(2)-N,
   motif 10: F-x-[HR]-[AF]-x(5)-[QR],
   motif 11: 1-x-L-P-x-G-[LM]-F-x(3)-I,
   motif 12: [LI]-I-x(2)-[YWF]-F,
   motif 13: I-x(3)-I,
   motif 14: [DN]-[TN]-E-x(2)-[IL]-[KR]-[VR]-Y-[KR]-x-Q-D,
   motif 15: R-N-[SA]-[FA]-x-H-x(2)-Y,
   wherein A, F, C, U, D, N, E, Q, G, H, L, I, K, O, M, P, R, S, T, V, W, Y are standard amino acid codes, "x" is any amino acid, numbers in a bracket after x represent multiple consecutive x's, the content in "[ ]" is an optional amino acid code and "-"is a separator.

9. The Cas13 protein according to claim 8, comprising a sequence as shown in any one of SEQ ID NO: 2, SEQ ID NO: 3 and SEQ ID NO: 60, or a sequence having 50% or more of sequence identity with the sequence as shown in any one of SEQ ID NO: 2, SEQ ID NO: 3, and SEQ ID NO: 60.

10. The Cas13 protein according to claim 8, wherein any amino acid residue in the amino acid sequence of the Cas 13 protein, except the amino acids identified by the motifs 1-15, is subjected to conservative amino acid replacement on the basis of a wild-type sequence, and the wild-type sequence comprises the sequence as shown in SEQ ID NO: 2, SEQ ID NO: 3 or SEQ ID NO: 60.

11. The Cas13 protein according to claim 8, wherein the Cas13 protein can form a complex with gRNA.

12. The Cas13 protein according to claim 8, wherein the Cas13 protein can be guided to a target nucleic acid by the gRNA.

13. The Cas13 protein according to claim 8, wherein the Cas13 protein is derived from the same kingdom, phylum, class, order, family, genus or species as a protein comprising the sequence as shown in any one of SEQ ID NO: 1-SEQ ID NO: 7 and SEQ ID NO: 60.

14. A conjugate comprising the Cas13 protein according to any one of claims 1-13 and a modifying moiety for modifying the Cas13 protein.

15. The conjugate according to claim 14, wherein the modifying moiety is selected from: a localization tag for providing subcellular localization, a tag for facilitating tracking, separation or purification, a translation activation domain, a translation suppression domain, a nuclease domain, a deaminase domain, a methylase domain, a demethylase domain and a regulatory splicing domain.

16. The conjugate according to claim 15, wherein,

the localization tag for providing subcellular localization is selected from: a nuclear localization signal and a nuclear export signal;

the tag for facilitating tracking, separation or purification is selected from: an epitope tag, a fluorescent protein, a HIS tag, a hemagglutinin (HA) tag, a FLAG tag, a Myc tag, a glutathione S-transferase (GST) tag, and a maltose-binding protein (MBP) tag;

the translation activation domain is selected from: domains of eIF4E and other translation initiation factors and a yeast poly(A)-binding protein and GLD2;

the translation suppression domain is selected from: a Pumilio protein, a deadenylase, and an Argonaute protein;

the nuclease domain is selected from: FokI, a PIN endonuclease domain, a NYN domain, a SMR domain from SOT1 and an RNase domain from staphylococcal nuclease;

the deaminase domain is derived from cytidine deaminase or adenosine deaminase;

the methylase domain is derived from a m6A methyltransferase;

the demethylase domain is derived from an RNA demethylase ALKBH5; and

the regulatory splicing domain is selected from: SRSF1, hnRNP A1 and RBM4.

17. The conjugate according to claim 14, wherein the conjugate comprises or does not comprise a linker for connecting the Cas13 protein and the modifying moiety.

18. A gRNA capable of forming a complex with the Cas13 protein according to any one of claims 1-13 or the conjugate according to any one of claims 14-17.

19. The gRNA according to claim 18, comprising a guide sequence which can be complementary to a target nucleic acid, and a direct repeat sequence which can interact with the Cas13 protein or the conjugate.

20. The gRNA according to claim 18, comprising a guide sequence and a direct repeat sequence, wherein a secondary structure of the direct repeat sequence comprises a first stem complementary paired, a non-complementary bulge structure, a second stem complementary paired and a non-complementary loop structure which are connected in sequence.

21. The gRNA according to claim 20, wherein,

a. the first stem consists of 4-7 base pairs,
b. one of the sequences of the non-complementary bulge structure is 2-6 nucleotides in length,
c. the second stem consists of 4-7 base pairs,
and/or
d. a sequence of the non-complementary loop structure is 5-8 nucleotides in length.

22. The gRNA according to claim 18, wherein one of the sequences of the first stem is selected from: GUUG, GUUGU, GUUGUA and GUUGUUA.

23. The gRNA according to claim 19, wherein the direct repeat sequence is selected from any one of SEQ ID NO: 15-SEQ ID NO: 21 and SEQ ID NO: 62, or selected from a sequence having 90% or more of sequence identity with the sequence as shown in any one of SEQ ID NO: 15-SEQ ID NO: 21 and SEQ ID NO: 62.

24. The gRNA according to claim 19, comprising a guide sequence and a direct repeat sequence, wherein the guide sequence is 10 nt-60 nt in length.

25. The gRNA according to claim 19, wherein the target nucleic acid is PTBP1 mRNA, AQp1 mRNA, VEGFA mRNA, VEGFR1 mRNA or VEGFR2 mRNA.

26. A composition comprising:

1) the Cas13 protein according to any one of claims 1-13, the conjugate according to any one of claims 14-17, a nucleic acid encoding the Cas13 protein according to any one of claims 1-13, or a nucleic acid encoding the conjugate according to any one of claims 14-17;
and
2) the gRNA according to any one of claims 18-25 or a nucleic acid encoding the gRNA according to any one of claims 18-25.

27. The composition according to claim 26, wherein the gRNA comprises a guide sequence which can be complementary to a target nucleic acid, and the target nucleic acid is the PTBP1 mRNA or the AQp1 mRNA.

28. A vector comprising:

1) a nucleotide sequence encoding the Cas13 protein according to any one of claims 1-13, or a nucleotide sequence encoding the conjugate according to any one of claims 14-17;
and/or
2) a nucleotide sequence encoding the gRNA according to any one of claims 18-25.

29. The vector according to claim 28, comprising a regulatory element which can regulate the expression of the nucleotide sequence.

30. The vector according to claim 29, wherein the regulatory element is a promoter.

31. A delivery composition, comprising a delivery vector, and at least one selected from following: the Cas13 protein according to any one of claims 1-13, the conjugate according to any one of claims 14-17, the gRNA according to any one of claims 18-25, the composition according to claims 26 or 27, or the vector according to any one of claims 28-30.

32. The delivery composition according to claim 31, wherein the delivery vector is at least one selected from a delivery particle, a delivery vesicle and a virus vector.

33. A cell comprising at least one of the Cas13 protein according to any one of claims 1-13, the conjugate according to any one of claims 14-17, the gRNA according to any one of claims 18-25, the composition according to claims 26 or 27, and the vector according to any one of claims 28-30.

34. The cell according to claim 33, wherein the cell is a eukaryotic cell.

35. A method of targeting or modifying a target nucleic acid, comprising delivering to the target nucleic acid at least one selected from the Cast 3 protein according to any one of claims 1-13, the conjugate according to any one of claims 14-17, the gRNA according to any one of claims 18-25, the composition according to claims 26 or 27, the vector according to any one of claims 28-30, and the cell according to claims 33 or 34.

36. The method according to claim 35, wherein the target nucleic acid is derived from an animal cell, a plant cell or a microbial cell.

37. The method according to claim 35, wherein the target nucleic acid is PTBP1 mRNA, AQp1 mRNA, VEGFA mRNA, VEGFR1 mRNA or VEGFR2 mRNA.

38. Use of the Cas13 protein according to any one of claims 1-13, the conjugate according to any one of claims 14-17, the gRNA according to any one of claims 18-25, the composition according to claims 26 or 27, the vector according to any one of claims 28-30, and the cell according to claims 33 or 34 in preparation of a medicament for diagnosing, preventing or treating a disease in a subj ect.

39. A method for detecting a nucleic acid, comprising the step of allowing the following a and b to form a complex, and binding the complex to a target nucleic acid to be tested:

a. the Cas13 protein according to any one of claims 1-13 or the conjugate according to any one of claims 14-17,
b. the gRNA according to any one of claims 18-25.

**40.** The method according to claim 39, comprising allowing the conjugate according to any one of claims 14-17 to form a complex with the gRNA according to any one of claims 18-25, and binding the complex to the target nucleic acid to be tested;
wherein the conjugate contains a detectable label, in which a signal change is caused by the binding, cleavage or modification of the complex to the target nucleic acid, and the content of the target nucleic acid in a sample to be tested is analyzed by observing the signal change of the detectable label.

**41.** A method for diagnosing, preventing or treating a disease, comprising administering an effective amount of the Cas13 protein according to any one of claims 1-13, the conjugate according to any one of claims 14-17, the gRNA according to any one of claims 18-25, the composition according to claims 26 or 27, the vector according to any one of claims 28-30, and the cell according to claims 33 or 34 to the subject in need thereof.

**42.** A pharmaceutical composition for the use of diagnosing, preventing or treating a disease, comprising the Cas13 protein according to any one of claims 1-13, the conjugate according to any one of claims 14-17, the gRNA according to any one of claims 18-25, the composition according to claims 26 or 27, the vector according to any one of claims 28-30, and the cell according to claims 33 or 34.

Fig. 1

Fig. 2

Fig. 3A

Fig. 3B

Fig. 4

Fig. 5

Fig. 6

Fig. 7

Fig. 8

Fig. 9A

```
Consensus          LP-KL---L--------------------------------K-A--KL--M---T--RL-----------------K-GK------K-G-I
PbuCas13b...Broad2019  LPKQMISILKGR---------------------QKDMGKEAERKIGEMIDDTQRRLDLLCKQTNQKI------RIGKRNAGLLKSGKI 628
Cas13m.2           LPRKLLDYLLKKE------------------ICAQDLFNTWAQSKIQRMIAQTDSLLQHLEKDLQAVSD-LKQNKFGKKAFVAIKPGHI 704
Cas13m.3           IPDKLRCMMQTKPFFKNGRQQLSPLGYPIMKNWIGVAEQRKHAAQVLRDVANEAADRLASFEKKHQRVVVGGRDNRYGRRGHADIRHGSL 697
Cas13m.6           IPEKLLHWLSSES-----------------EEDPSEKYAKKLEEEIKLRRERVQRRLEKFNQDLREIRK-KDSVPYGKKGHVNIRHSQL 656
Cas13m.1           LPKPLVKYLTHS------------------LDELPTYKSKAIKKLKFWQDETENLLAEVKRHNEKSEK-AR--KENKFFKSFLKSGQI 556
Cas13m.4           LPRKMLDYLLMKD------------------VNAHDLFRKWAEAELQQMIEQTDRLSQRIDDDIKAAAN-MRQNKFGKKSFVAVKPGKI 701
Cas13m.5           VPVRLHAFLSGQE------------------QADTKAIARGKLELMAQQNKKRIERFDAMKKAVV------KVGKAQYRTLRSGDI 609

Consensus          AD-L--DMM-FQP---D-------------KLT--N-R-LQ--LA----Y-S-----L---F----------AN-----N---HPFL--
PbuCas13b...Broad2019  ADWLVNDMMRFQPVQKDQNNI----PINNSKANSTEYRMLQRALAL---FGSE-NFRLKAYFN--------QMNLVGNDNP---HPFLAE 699
Cas13m.2           ADFLAHDMMFFQPSMKD----------CNNKLTGLNFRILQSSMAV---YDGN-FDELSRIMR--------SAHIIGNANDACCNPIVMA 772
Cas13m.3           ARYLATSMVRWQPALDQ--------P-GGDKLTSANHRALAGFLSEYGLHGSN-INKLRNVLK--------EAGLIEGSHP---HPFLAH 766
Cas13m.6           AKYLMRSIMEWQPTRND----------GKNKLTGQNFNVMTAFLATLG-YTSQ-VKDLRDLFS--------RANMLEGPNA---HPFLKK 723
Cas13m.1           ATWLIKDIQHFLP-------------LQGKLSVLKYNALQAKLAI---YNSEELKEMLTDFQVYDTPKGTDRNMPEKGGG---HQFIKT 626
Cas13m.4           ADFLAHDMMLFQPCTED----------NSNKLTGLNFRILQSVMAV---YNGD-FDELSRVLR--------NAHIIGNATDEMCNPIVMA 769
Cas13m.5           GDWLVRDFMRFQPIGYKRNQAGKQEPDLKSKANPKKYQLIQKSLAL---YEQE-KNNLLGLFK--------SCNLLSSENE---HPFLNE 684

Consensus          V----------NI--FY--YL--R---LE--------------------F-HF-----Q--------Y-R--A--YL-----------
PbuCas13b...Broad2019  TQ----WEHQTNILSFYRNYLEARKKYL-----KGLKPQNWKQ-------YQHFLILKVQKTNRN---TLVTGW--------------- 754
Cas13m.2           VCRK--HKGFSNIIRFYQAYLKERKAYLQQCANER--------HYDSLSFLHASQNKWRERTQA---YYRSLAAKYLAE-----NYDGV 843
Cas13m.3           VL----ESAPANIEALYVAYLKHEQSHATALKNKFTDRNGIVQ-PSEVPAFVRFNSSRWRNDSAT-------TARRYLQTPPAPGSSDSA 844
Cas13m.6           VLN---NNSIKDIQGFYRTYLVEELNQIEDKQRRIAKAKNVKD-TVRQFPFAHFNRMRYQKRDED---YYRNLAKRYLNI------GDNE 800
Cas13m.1           VFDKNPQKLPPHWLHFYNDYLNAKKQWIEDKIKFLTAMPDTEAEIMKQQPLFYFLDLGSNYEEGEKIVYFRENSPAYIAK------YCEE 710
Cas13m.4           VCHK--SMEFGNIVDFYKAYLRERRIYLERCLRHG--------DFESLSFLHASQIRWQERSKE---YYRALAARYLVD-----EYGGT 840
Cas13m.5           VV----QSMPATWQDFYERYLHARSRFLEKCIEKGIKKNSYQA--------CYSFLKIKPQLKDKE---KLYQGW--------------- 744

Consensus          -----I-LP-GLF---I----------------------L-E-------------------LI--YF-----D--Q---PFYD------
PbuCas13b...Broad2019  --KNSFNLPRGIFTQPIREWFE----------------KHNNSKRIYDQILSFDRVGFVAKAIPLYFAEEYKDNVQ---PFYDY----- 817
Cas13m.2           DTTKSIELPRGLFETYIRQ----------------ELSEIGSTKS--MAGDATKNTSYLIYGYFRQVMSDDAQ---TFYDT----- 903
Cas13m.3           EHNAPIMLPDGLFTTHIMTLLNKVLGQNDRVPEEDYLRHDLPRIASIIN--PNGKTYGAAYIRAWFDQVENQDVQ---PFYDL----- 923
Cas13m.6           KDKAVILLPDGMFTSYIYDLIMKLP-------------ENNEKMRINL--ASDVAHCNSSFLISRFFENIRNDYAQ---PFYRE----- 866
Cas13m.1           LLKKPVDLPIALSYDLVAN---------------MRTGLEKAKSVTD---------------------FIDDKYQSEPPYYHL----- 757
Cas13m.4           ESAKAIELPRGLFEPYIRK----------------ELSEMNAMKS--LACNSDYNVSYLIYGYFKRVMSDDAQ---PFYDE----- 900
Cas13m.5           --DAQMNLPTNMFIDAIHDWFRQ---------------THHESLRTWF--TQQEKPHQLIGLIRKYIELAHTDQIQ---GFYDMFPLRY 811

Consensus          --------------------R-Y-LF--L--K---------------------------------------I---I------------
PbuCas13b...Broad2019  ----------------------------------PFNIGNRLKPKKRQ----------FLDKKERVELWQK-------- 844
Cas13m.2           --------------------RRCYQLFDVLYRKSPRD------NHSYYSTAQIREMLM-----RSHSKSIRKDIDNYISQTT-------- 954
Cas13m.3           --------------------PRFYREISLLAPRR---------KPNQELIRDYFSEEQ---------IAQKIQTV------------ 960
Cas13m.6           --------------------ERTYELFSILNNKKVRNTLQPLFISPHDINIQLTEKEK-----DGKGRLILQKIDHFCKSIT-------- 923
Cas13m.1           --------------------PRQYDLFKAFGDKPSEKLFADDKKPLHEVYGEYKAKKT---------------------D------- 796
Cas13m.4           --------------------KKCYRLFNVLYRKSPHD------SPVYRNTAEIRDMLM-----QNSPNSIRKDIESYLSNTI-------- 951
Cas13m.5           DFYKKEFPNGLVLHERINKQKQIWEAQLIQTKKRLED-----------AASKLKKVKQQVESLPDQELRFRNETEAMVYFTKLFDSSIVK 890

Consensus          ----------------------------------------------L--K--E--IRRYK-QD--L-L-----L-----------S------
PbuCas13b...Broad2019  ----------------NKELFKNYPSEK---KKTDLAYLDFL-SWKKFERELRLIKNQDIVTWLMFKELF--------NMATVE--- 900
Cas13m.2           -------------------AAER---TKEKERCDALLRKIKDTETELKVYKIQDILLFLIAKRLLLDRKV---ENDSAVQ--- 1009
Cas13m.3           --------------------PK---KQRSEKVGHTI----DTEKDIRRYRLQDITLYLTLLDMLTLMLSRNEAERTDRQMKS 1015
Cas13m.6           ----------------QKGNFNNVEEAK---EATSRKLKHLITDCKNNERDIRRYKTQDMVIYLMARDILKDI-----IPDSEKDK-- 985
Cas13m.1           -------------P---------------RTIKKIKGFL----DQEQRIRYLKVCDKLLVKILEKYL--------AKETE--- 836
Cas13m.4           ------------------IADR---AKEKERCTALLREMKKCETELKRYKIQDMLLFLIAKRILSDLPA---AHDSAVQ--- 1006
Cas13m.5           NAILKLQRDQKALRVNTIGEKIIKIYTAKYDAIHQEVRDFKSML----TTEKMIRRVKAEDCVTLFMLTDLM---------NQSQITI-- 965

Consensus          ---------I-L-DI--------------L----------------K----------I-----KLK-YG-F----L-DRRL-SL-D----
PbuCas13b...Broad2019  --GLKIGEIHLRDIDTNTANEESNNILNRIMPMKLPVKTYETDNKGNILKERPLATFYIEETETKVLKQGNFKALVKDRRLNGLFSFAET 988
Cas13m.2           --MNAINQIRLRNIAD-------GNTLSQKIPISISIKSRKGDPK------------IIQQDDLKLKNYSQFYSIISDRRLPSLLDLI-- 1076
Cas13m.3           STAERVSNMRLKFDH-----------SFDFDLLGSTGSGEAAYSYLHQ-RSGITISMPALSLRSYGSIFRVLADSRFETLMDALN- 1088
Cas13m.6           --YAKDRKLLLKDVCE---------EGFLRQAVKMEYE---YSIEEKGKRTR-----TVKITHPNMSLKNYGEFHRLLNDERLKSLLQQL-- 1056
Cas13m.1           -----LKNVQLTDAQG-------KLILDKVLETEFEMPPY-----------------GFKVRLKDYGRYRRFVKDRRLVSMQDYL-- 892
Cas13m.4           --MQAISRIHLKDITD-------GNTLSEKISLSVKVVSKNGYIK-----------KLTQHNLKLKNYSQFYAILSDRRLPSLLDLV-- 1073
Cas13m.5           --GQEQRTIKLSDIQP-MGETQIQGILDAVQVLEQKLDFFSSDEQGKISQVK-LGEWTIFSTDTKVKKQGNFKQLLKDRRLNNLAHYI-L 1050

Consensus          ---------I---D----EL--YD--R----K-----EK-------------------------I----D-----------------M
PbuCas13b...Broad2019  TDLNLEEHPISKLSVDLELIKYQTTRISIFEMTLGLEKKLIDKY------------------------STLPTDSFRN---------M 1043
Cas13m.2           -----NSRVIKRTDIEDELSNYDKSHPHVLKSVFEFEKHYPDTH---PIPSDTA------YMALPDTG-----------------EM 1132
Cas13m.3           RQ---GVTHVNFGDITSELALYDTLRSHFLLQAHNVEQDAFSAKRGVLENHTSPFFYRSGNLQLDDQGNITNPSTDAIRNH--YGELIKI 1173
Cas13m.6           ----ANMDEIDYTDLMGEFADYDQKRSEIFRLAQSIEKHLYEQNEQGLNDEKSDLFYHTR---------YNGKKIPRRNS--FSSLLEL 1130
Cas13m.1           -----NQSVFTPDTLITELNLYEKQRSEFLKIVLEFEKRLLSASD-TLN-----------INLADQQ--TTLGEDKIRDYITHNYLLEV 962
Cas13m.4           -----RSNYINRNDIEAELDNYDKVHPEVMKAIIGLEKKHFEKH--GFDDSG----------IVPDLS-------------------SI 1126
Cas13m.5           PDIAGGAIRVRRNLLEMELDQYDRNRIPIIKLMYELEYAIYKVD--PFSVELK--------------------------YKKFSQC 1108

Consensus          L--E----------E----------------IRNAF-HN-YP----------------------------ATELP--A----K-------
PbuCas13b...Broad2019  L-ERWLQCKANR[Lysine (1142)]VN-----SLIAVRNAFSHNQYPMYDATLFAEVKKFTLFPSVDTKKI--ELNIAPQLLEIVGKAIKEIEKSEN 1125
Cas13m.2           L-KESNLTAEKQKE-------------VRKIRNSFAHLSYPSRNIT----------------------GAASTELPKKAEIISKNLIEHLS 1187
Cas13m.3           L-DRYSLKIDKKTKDGKSQDILLRDLMAELRNAAAHNRYPKADFFF----RQFDHFLNTCKPTD--SNLTA---PNYIRTVLEFLKSIVD 1253
Cas13m.6           IGEEESQMTETDKKQ-----------TISIRNAFGHNTYKVSLA----------------------EMNATELPNVAKTILKKMEELRN 1186
Cas13m.1           A-LRNNFISENEARA-----------MLWFRNGALHNQLPD----L----QKLV-------------GLIDTEEQKTQRYFLKGMEMYKK 1019
Cas13m.4           L-AETTMPADKQYE------------VRKIRNSFAHSHYPGYHVA--------------------NAGITELPKKAETIFNTLKSSLS 1181
Cas13m.5           L-REYAQKAVLNVEQVEHLN-----VLIAIRNAIMHNQYPNRDHL------KAIVPFTVSEYAPVQEGLTIARQLLACAQVSVNIILSTIS 1187
```

Fig. 9B

| Consensus | | |
|---|---|---|
| PbuCas13b...Broad2019 | KN----------------- | 1127 |
| Cas13m.2 | NAE-------------IK | 1192 |
| Cas13m.3 | NNFTPLLHEESPENESKSE | 1272 |
| Cas13m.6 | KL----------------- | 1188 |
| Cas13m.1 | GIE------------KIS | 1025 |
| Cas13m.4 | DE----------------- | 1183 |
| Cas13m.5 | KFE---------------- | 1190 |

Fig. 9C

**Motifs 1 and 15**
A

**Motif 2**
B

**Motif 3**
C

**Motif 4**
D

**Motif 5**
E

**Motif 6**
F

**Motif 7**
G

**Motif 8**
H

**Motif 9**
I

**Motif 10**
J

**Motif 11**
K

**Motif 12**
L

**Motif 13**
M

**Motif 14**
N

Fig. 10

Fig. 11

## INTERNATIONAL SEARCH REPORT

| International application No. |
|---|
| **PCT/CN2022/129825** |

| A. | CLASSIFICATION OF SUBJECT MATTER |
|---|---|

C12N 9/22(2006.01)i

According to International Patent Classification (IPC) or to both national classification and IPC

| B. | FIELDS SEARCHED |
|---|---|

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science, GenBank, 中国专利生物序列检索系统, Chinese Patent Biological Sequence Retrieval System: 核酸酶, 核酶, ribonuclease, CAS, 13, 本申请的SEQ ID NO: 1

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 113348245 A (BROAD INSTITUTE; MASSACHUSETTS INSTITUTE OF TECHNOLOGY; PRESIDENT AND FELLOWS OF HARVARD COLLEGE et al.) 03 September 2021 (2021-09-03) entire document, and in particular, SEQ ID NO: 178, and abstract | 1-7 (in part), 13-17 (in part), 26-42 (in part) |

☐ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 January 2023** | **19 January 2023** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| :--- |
| **PCT/CN2022/129825** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    [1]  The actually submitted sequence listing is an XML file in Standard ST.26.

Form PCT/ISA/210 (continuation of first sheet) (January 2015)

## INTERNATIONAL SEARCH REPORT

International application No.

**PCT/CN2022/129825**

| Box No. III | Observations where unity of invention is lacking (Continuation of item 3 of first sheet) |
|---|---|

This International Searching Authority found multiple inventions in this international application, as follows:

[1]  Invention 1: claims 1-7 (in part), 13-17 (in part), and 26-42 (in part) relate to a Cas13 protein based on SEQ ID NO: 1 and a corresponding invention.

[2]  Invention 2: claims 1-7 (in part), 9-10 (in part), and 13-17 (in part), and 26-42 (in part) relate to a Cas13 protein based on SEQ ID NO: 2 and a corresponding invention.

[3]  Invention 3: claims 1-7 (in part), 9-10 (in part), 13-17 (in part), and 26-42 (in part) relate to a Cas13 protein based on SEQ ID NO: 3 and a corresponding invention.

[4]  Inventions 4-7: claims 1-7 (in part), 13-17 (in part), and 26-42 (in part) respectively relate to a Cas13 protein based on SEQ ID NOs: 4-7 and a corresponding invention.

[5]  Invention 8: claims 1-7 (in part), 9-10 (in part), 13-17 (in part), and 26-42 (in part) relate to a Cas13 protein based on SEQ ID NO: 60 and a corresponding invention.

[6]  Invention 9: claims 8 (in part), 11-12 (in part), 14-17 (in part), and 26-42 (in part) relate to a Cas13 protein based on sequence 1 and a corresponding invention.

[7]  Inventions 10-23: claims 8 (in part), 11-12 (in part), 14-17 (in part), and 26-42 (in part) respectively relate to a Cas13 protein based on sequences 2-15 and a corresponding invention.

[8]  Invention 24: claims 18-25 (in part), and 26-42 (in part) relate to gRNA based on GUUG and a corresponding invention.

[9]  Inventions 25-27: claims 18-25 (in part), and 26-42 (in part) respectively relate to gRNA based on GUUGU, GUUGUA and GUUGUUA and a corresponding invention.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-7 (in part), 13-17 (in part), and 26-42 (in part), based on SEQ ID NO: 1**

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2022/129825**

| Patent document cited in search report | | | Publication date (day/month/year) | Patent family member(s) | | | Publication date (day/month/year) |
|---|---|---|---|---|---|---|---|
| CN | 113348245 | A | 03 September 2021 | CA | 3111432 | A1 | 06 February 2020 |
| | | | | WO | 2020028555 | A2 | 06 February 2020 |
| | | | | IL | 281159 | A | 29 April 2021 |
| | | | | AU | 2019314433 | A1 | 25 March 2021 |
| | | | | EP | 3830256 | A2 | 09 June 2021 |
| | | | | KR | 20210053898 | A | 12 May 2021 |
| | | | | SG | 11202102068 T | A | 30 March 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022129825 W
- CN 2021113061499
- CN 2022105188261
- CN 112410377 A

**Non-patent literature cited in the description**

- *Proc Natl Acad Sci U S A.,* 10 November 2009, vol. 106 (45), 19126-31
- **SLAYMAKER, IAN M et al.** High-resolution structure of Cas13b and biochemical characterization of RNA targeting and cleavage. *Cell reports,* 2019, vol. 26 (13), 3741-3751
- **KOONIN ; EUGENE V ; KIRA S. MAKAROVA.** Evolutionary plasticity and functional versatility of CRISPR systems. *PLoS biology,* 2022, vol. 20 (1), e3001481